(19) Europäisches Patentamt
European Patent Office
Office européen des brevets

(11) EP 2 348 110 B1

(12) **EUROPEAN PATENT SPECIFICATION**

(45) Date of publication and mention of the grant of the patent:
**27.03.2013 Bulletin 2013/13**

(51) Int Cl.:
***C12N 15/11*** *(2006.01)* ***C12Q 1/68*** *(2006.01)*

(21) Application number: **10012191.2**

(22) Date of filing: **01.06.2004**

(54) **PROCESS FOR SCREENING A DRUG RESPONSE IN CANCER PATIENTS**

PROZESS ZUM SCREENING EINER MEDIKAMENTENANSPRACHE IN KREBSPATIENTEN

PROCEDE DE CRIBLAGE D'UNE REPONSE MEDICAMENTEUSE DES PATIENTS ATTEINTS DE CANCER

(84) Designated Contracting States:
**AT BE BG CH CY CZ DE DK EE ES FI FR GB GR HU IE IT LI LU MC NL PL PT RO SE SI SK TR**

(30) Priority: **30.05.2003 GB 0312451**
**26.09.2003 GB 0322636**
**21.11.2003 GB 0327132**

(43) Date of publication of application:
**27.07.2011 Bulletin 2011/30**

(62) Document number(s) of the earlier application(s) in accordance with Art. 76 EPC:
**04735607.6 / 1 633 870**

(73) Proprietor: **OncoTherapy Science, Inc.**
**Kawasaki-shi**
**Kanagawa 213-0012 (JP)**

(72) Inventors:
- **Tsuruo, Takashi**
  **Tokyo**
  **156-0051 (JP)**
- **Nakamura, Yusuke**
  **Yokohama-shi**
  **Kanagawa, 225-0011 (JP)**
- **Sone, Saburo**
  **Tokushima**
  **770-0037 (JP)**
- **Fukuoka, Masahiro**
  **Nara**
  **631-0007 (JP)**

(74) Representative: **Harding, Charles Thomas et al**
**D Young & Co LLP**
**120 Holborn**
**London**
**EC1N 2DY (GB)**

(56) References cited:
- **DATABASE GENEBANK [Online] 5 July 2001 (2001-07-05), XP002304184, retrieved from GENEBANK Database accession no. BC009799**
- **DE BONO JOHANN S ET AL: "The ErbB receptor family: a therapeutic target for cancer.", TRENDS IN MOLECULAR MEDICINE. 2002, vol. 8, no. 4 Suppl, 2002, pages S19-S26, XP002290892, ISSN: 1471-4914**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> ASTRAZENECA UK LIMITED THE UNIVERSITY OF TOKYO

<120> PROCESS

<130> P017248EPA

<150> GB 0312451.8<151> 2003-05-30

<150> GB 0322636.2<151> 2003-09-26

<150> GB 0327132.7<151> 2003-11-21

<160> 95

<170> PatentIn version 3.5

<210> 1
<211> 352
<212> DNA
<213> Homo sapiens

<400> 1

```
atacggcatc catgaaatat atcatgcgat acaacaatta taagaaggat ccttacagta        60
gaggtgaccc ctgtaatacc atctgctgcc gtgaggacct gaactcacct aacccaagtc       120
ctggaggttg ttatgacaca aaggtggcag atatctacct agcatctcag tacacatcct       180
atgccataag tggtcccaca gtacaaggtg gcctccctgt ttttcgctgg gaccgtttca       240
acaaaactct acatcagggc atgccagagg tctacaactt tgattttatt accatgaaac       300
caattttgaa acttgatata aaatgaagga gggagatgac ggactagaag ac               352
```

<210> 2
<211> 748
<212> DNA
<213> Homo sapiens

<400> 2

ctccactcgc tcttccaaca cccgctcgtt ttgcggcagc tcgtgtccca gagaccgagt 60
tgccccagag accgagacgc cgccgctgcg aaggaccaat gagagccccg ctgctaccgc 120
cggcgccggt ggtgctgtcg ctcttgatac tcggctcagg ccattatgct gctggattgg 180
acctcaatga cacctactct gggaagcgtg aaccattttc tggggaccac agtgctgatg 240
gatttgaggt tacctcaaga agtgagatgt cttcagggag tgagatttcc cctgtgagtg 300
aaatgccttc tagtagtgaa ccgtcctcgg gagccgacta tgactactca gaagagtatg 360
ataacgaacc acaaatacct ggctatattg tcgatgattc agtcagagtt gaacaggtag 420
ttaagccccc ccaaaacaag acggaaagtg aaaatacttc agataaaccc aaaagaaaga 480
aaaagggagg caaaaatgga aaaaatagaa gaaacagaaa gaagaaaaat ccatgtaatg 540

cagaatttca aaatttctgc attcacggag aatgcaaata tatagagcac ctggaagcag 600
taacatgcaa atgtcagcaa gaatatttcg gtgaacggtg tggggaaaag tccatgaaaa 660
ctcacagcat gattgacagt agtttatcaa aaattgcatt agcagccata gctgccttta 720
tgtctgctgt gatcctcaca gctgttgc 748

<210> 3
<211> 386
<212> DNA
<213> Homo sapiens

<400> 3

gataggccac attccagtaa gaactcaatt tgtctcccaa atttgcagaa acaaaacgtg 60
atttaaaagc tgagcttttt atcagaaagc ttttttgatg ttttaagtgt tatgtgactt 120
gttgaacttt ttaaaaagtg ctacttttaa aatcccagat actctgaatt ttagaaaaca 180
aactaattct gattgtgtcg tgcccaagta cccttttttt tttaatgagt agggaccaat 240
gccacattgc tttttatatt tctttctttt ttaatgttgc caaaaccaaa agtagctttg 300
ttttcctttg tattttgcta ctttgcagta tttgtgtgtg tggttttttt tccttaattt 360
gaaagggaca gcactgtgta tgttta 386

<210> 4
<211> 565
<212> DNA
<213> Homo sapiens

<400> 4

aggagaccat ttggaagaag aactagatct gttgcttaat ttagatgcac ctataaaaga 60
gggagataac atcttaccag atcagacgtc tcaggacctg aaatccaagg aagatgggga 120
ggtggtccaa gaggaagaag tttgtgcaaa accatctgtg actgaagaaa aaaacatgga 180
acctgagcaa ccaagtacct ccaaaaatgt taccgaggaa gagctggaag actggttgga 240
cagcatgatt tcctaaaaag ggaaaaaaa gtgcctgaag caaatcttgg ttgccttcta 300
acggcaggtg ggcataaggc tgtccttcag gaccagccag tttacaagca tgtctcaagc 360
tagtgtgttc cattatgctc acagcagtaa atgcctacct ctgtgtttga catctgaaag 420
aatacattga agcagcttgt tgcatttgtt tttctggctt agtaatctaa tagatttcct 480
taagggcagg agatagactc tggcccttgt ttctagcctc cttccttgca gtgtttacaa 540
catagccagt gtttacagca tagca 565

<210> 5
<211> 215
<212> DNA
<213> Homo sapiens

<400> 5

ggatccttta ttggtggtag agcaaaaaaa cccaaacacg ataaaccttt caaaagactt 60
tctaaggatg atattggaat gcaccagccc tcacatgtgt atgcacattt gccagaatat 120
aagagttttg ttttaaatac agtcttgtta ggattttacg ttattgttat tatggaaagt 180
gattgtgatg ctatttatct tcagggtcac tctgg 215

<210> 6
<211> 218
<212> DNA
<213> Homo sapiens

<400> 6

gcagtcgttt caaccaggta gttttgggtt gtttttaaag ccctttttgag gtcttacaca 60
ttattaactt taaaataatc aggcagctaa gaataattac tagaaaaatc atctaccact 120
tcaaacatgg tcaactactt caaaactgca cctagagaat caggtacctg aagtagaaca 180
agaagcctgg aggtggactt tgagaggagg gaataccc 218

<210> 7
<211> 525
<212> DNA
<213> Homo sapiens

<400> 7

tacgtgtact tcaccatcgt caccaccgac cacaaggaga tcgacttccg ctgcgcgggc 60
gagagctgct ggaacgcggc catcgcgctg gcgctcatcg atttccagaa ccgccgcgcc 120
ctgcaggact ttcgcagccg ccaggaacgc accgcacccg ccgcacccgc cgaggacgcc 180
gtggctgccg cggccgccgc accctccgag ccctcggagc cctccaggcc atccccgcag 240
cccaaacccc gcacgccatg agcccgccgc gggccatacg ctggacgagt cggaccgagg 300
ctaggacgtg gccggcgctc tccagccctg cagcagaaga acttcccgtg cgcgcggatc 360
ctcgctccgt tgcacgggcg ccttaagtta ttggactatc taatatctat gtatttattt 420
cgctggttct ttgtagtcac atattttata gtcttaatat cttgtttttg catcactgtg 480
cccattgcaa ataaatcact tggccagttt gcttttctac catcc 525

<210> 8
<211> 565
<212> DNA
<213> Homo sapiens

<220>
<221> misc_feature
<222> (69)..(72)
<223> n is a, c, g, or t

<220>
<221> misc_feature
<222> (178)..(181)
<223> n is a, c, g, or t

<220>
<221> misc_feature
<222> (188)..(192)
<223> n is a, c, g, or t

<220>
<221> misc_feature
<222> (525)..(527)
<223> n is a, c, g, or t

<400> 8

```
ctgtgacatg ctcttgagct ttaccctagt tgaacataca tgtgtagatt tacacatact      60
gtttcattnn nnaatttaga aattgttcat taaatcccat ttgaggtata agtcactcag     120
gaagttaaaa tatctctaca cgtatatttt tacattaaaa atacagtgtt agcataannn     180
nccctttnnn nngaagaaca aaaatgtcag tgcatagtta gataaaatgg taaaatgttt     240
tactgaaagc atactttttt ggaaaataga ttcatgaagc ctttaagtgc tgcttctgtc     300
agtcaaacgt taaaaacttt aacattttca aagtgcccag actgtgtaca aagacacatg     360
taatggagat tgtacaggtt gtttttttgt ttgaaccttt gaaagagttt aatcttaacg     420
ttttctaatt ttaaaatttt aaaatcttgt ttaacaaaag cttgtattaa gatactgttt     480
tcatttcatt acagaattgt ttataaaagt tcatttgttg aaaannnagg atccttttta     540
ataccacagc atttgtactg ttcct                                           565
```

<210> 9
<211> 285
<212> DNA
<213> Homo sapiens

<400> 9

```
atatgtgcac acacacactc acacccacac ccataaagat tttgcactcc ttgaaggtac      60
actaactcac catttttatc atacttatcc cagtgtgcca cagttactgg cttatatgcc     120
tgtctctgct atcttatttt atctgtctcc acaacacagc aaactacctg gccttcaata     180
aagggcttat gaattattca tgaatccatt ttgccaggtg cctagccctg tgtctggctt     240
gaagcaggtg ttcccaaggt gtggcatggc tgagtgaata caaat                     285
```

<210> 10
<211> 207
<212> DNA
<213> Homo sapiens

<400> 10

```
caccaatgag cttactaccc aacttcaaaa ctaggactct aacaataact tctgtcatat      60
ctcatcctgt aacgccccca ccttcgctcc ttccgccaag ataattatca ctttaaattg     120
tgtgcgtgtg tattctcatt tcttatgtga tggtaaaaat gcctttattt tgtttggttt     180
taatgcatag aaaggacatc aagctgt                                         207
```

<210> 11
<211> 315
<212> DNA
<213> Homo sapiens

<220>
<221> misc_feature
<222> (173)..(181)

<223> n is a, c, g, or t

<400> 11

ctctaaaagc cattcactcc agattttacc tggggaatat tctacatact gcttactttc 60
tctataaaac tcatcaataa atcatgaaag gcactgagtt ttgtaaatca ggaccctaaa 120
tgtttaattg taaataagtt tcagataatt attatagctt tgcgttgaag ttnnnnnnnn 180
ntttttctca actagttaag tcaactgctt ctgaaataac tctgtattgt agattatgca 240
gatctttaca ggcataaata tttaaactgt aatatgctaa cttgaagaga ttgcaataaa 300
gctgcttcag ctaac 315

<210> 12
<211> 509
<212> DNA
<213> Homo sapiens

<400> 12

ctcactgaag ttgaaatgac tgcccacttc aaaatcttca ttgtgtttac acaccagtgt 60
atttatacaa atcagaggca ttttgtagat gctttgctga cttgttcagc tctgtaaaaa 120
cacagaaatc agacccattt tgtaaagcgg aaaatcatgt tacatggaac atgtcctgta 180
tatatcacat acatggtaat ggagtcttaa tgataagtgc aagataataa tttaatgatg 240
ggattagtct gatcgcttaa tatgcacaat cctggaagtg aattacttgc atcagatata 300
gtgatattta ttattctgta cagagagaaa aatacatata aaacatatgc ttacattaca 360
tgcacgcgga tttcatgctc cataatcttt tctatttttt aatttacctt tctgtaaatg 420
atgtgcatgg aatatgcctt atagaaaaat gctgttcata atttgactac gtggaaaagt 480
gcctatatgg tggtaatgct agtaaggca 509

<210> 13
<211> 1654
<212> DNA
<213> Homo sapiens

<400> 13

tggagcccga ggtccccgcg cggcccgggc ctggcgccct gaggggaaga gcggcccggc 60
ccgagccatg acggacggga tcctagggaa ggcagccaca atggagatcc ctatccacgg 120
gaacggcgaa gccaggcagc ttcctgaaga tgatgggctg gagcaggacc tccagcaggt 180
gatggtgtca ggacccaacc tcaatgaaac cagcattgtg tctggtggct atgggggctc 240

tggtgatgga ctcatcccca cagggtctgg ccgccatcca tctcacagca ccactccttc 300

tggccctgga gatgaggtgg ctcggggcat tgctggagaa aagtttgaca tcgtcaagaa 360

atggggcatc aacacctata agtgcacaaa gcaactgtta tcagaacgat ttggtcgagg 420

ctcacggact gtggacctgg agctagagct gcagattgag ttgctgcgtg agacgaagcg 480

caagtatgag agtgtcctgc agctgggccg ggcactgaca gcccacctct acagcctgct 540

gcagacccag catgcactgg gtgatgcctt tgctgacctc agccagaagt ccccagagct 600

tcaggaggaa tttggctaca atgcagagac acagaaacta ctatgcaaga atggggaaac 660

gctgctagga gccgtgaact tctttgtctc tagcatcaac acattggtca ccaagaccat 720

ggaagacacg ctcatgactg tgaaacagta tgaggctgcc aggctggaat atgatgccta 780

ccgaacagac ttagaggagc tgagtctagg cccccgggat gcagggacac gtggtcgact 840

tgagagtgcc caggccactt tccaggccca tcgggacaag tatgagaagc tgcggggaga 900

tgtggccatc aagctcaagt tcctggaaga aaacaagatc aaggtgatgc acaagcagct 960

gctgctcttc cacaatgctg tgtccgccta ctttgctggg aaccagaaac agctggagca 1020

gaccctgcag cagttcaaca tcaagctgcg gcctccagga gctgagaaac cctcctggct 1080

agaggagcag tgagctgctc ccagcccaac ttggctatca agaaagacat tgggaagggc 1140

agccccaggg tgtgggagat tggacatggt acatcctttg tcacttgccc tctggcttgg 1200

gctccttttt ctggctgggg cctgacacca gttttgccca cattgctatg gtgggaagag 1260

tgcctggagg cccagaagtt gctgccctgt ctatcttcct ggccacaggg cttcattccc 1320

agatcttttc cttccacttc acagccaacg gctatgacaa aaccactccc tggccaatgg 1380

catcactctt caggctgggg tgtgctccct gaccaatgac agagcctgaa aatgccctgt 1440

cagccaatgg cagctcttct cggactcccc tgggccaatg atgttgcgtc taataccctt 1500

tgtctctcct ctatgcgtgc ccattgcaga gaaggggact gggaccaaag gggtggggat 1560

aatggggagc cccattgctg gccttgcatc tgaataggcc taccctcacc cacccaccca 1620

gtttaattgt gcttagagcc caagaagatt ggga 1654

<210> 14
<211> 652
<212> DNA
<213> Homo sapiens

<220>
<221> misc_feature
<222> (188)..(188)
<223> n is a, c, g, or t

<400> 14

tttttttttt tttttttaa agtagtttaa taaactccac aaaataatag cagatgcatt 60

```
gaaatattta cataattcga ttttcaaatc tctcattcaa ataaaaggga taaaaataaa      120
atttctgcct ttacggcagc agaacctctt tcctgaaatg gattggtaaa ataagatact      180
tcactggnag aggaactaat ttatgtttaa gaggtattca tattcagcta agaaaataca      240
accctttttc agctatatag attagggaat ataaaatgat attttctaca tttttttgacc     300
tgtattcaaa gttctaaatt caactttgac ttgaagagag aaggtgattt tggtacccat      360
acagagtaga tcatcacaat tacaatggaa agataattaa cgttttatat gctgtttatt      420
tgcttttgaa agtttgggtc agaaaggctg tgataataat tctggcccaa acaggtatgc      480
ttatacctga cacaaatttc actaaaacct aacacttttg gcttggagtt cttgggattt      540
cgactttctg agtcccttcc atttccaaag catgtttcat tgagagcagg caatgtttgg      600
ggatcaggtg tatgattcaa gactaattaa gatgccaaag ttttccaagc tc              652
```

<210> 15
<211> 1990
<212> DNA
<213> Homo sapiens

<400> 15

```
attgtggcgg tgaggaacag gaagccctga agggtcaaaa gaaatacaaa agcaaaggct      60
attttctttt tttttttctt tctttcattc cttccttcct ctgtttcttt ctttcttcct     120
ttcatttttt tttctttttt aagagcgagc ggctctgcgg tggcggtttg gggtgggcgc     180
cgccgaggtg aggtcgtctc gcctcccgcg cgccggtaga ttggttgttt cattatggat     240
ggaggggatg atggtaacct tattatcaaa aagaggtttg tgtctgaggc agaactagat     300
gaacggcgca aaaggaggca agaagaatgg gagaaagttc gaaaacctga agatccagaa     360
gaatgtccag aggaggttta tgaccctcga tctctatatg aaaggctaca ggaacagaag     420
gacaggaagc agcaggagta cgaggaacag ttcaaattca aaaacatggt aagaggctta     480
gatgaagatg agaccaactt ccttgatgag gtttctcgac agcaggaact aatagaaaag     540
caacgaagag aagaagaact gaaagaactg aaggaataca gaaataacct caagaaggtt     600
ggaatttctc aagagaacaa gaaggaagtg gaaaagaaac tgactgtgaa gcctatagaa     660
accaagaaca agttctccca ggcgaagctg ttggcaggag ctgtgaagca taagagctca     720
gagagtggca acagtgtgaa aagactgaaa ccggaccctg agccagatga caagaatcaa     780
gagccctcat cctgcaagtc tctcggaaac acctccctga gtggcccctc catccactgc     840
ccctctgctg cagtatgtat cggcatcctc ccaggcctgg gtgcctactc tgggagcagc     900
gactccgagt ccagctcaga cagcgaaggc accatcaatg ccaccggaaa gattgtctcc     960
tccatcttcc gaaccaacac cttcctcgag gcccccctagt ttctccgtcc ctacacaggg    1020
agctcctccc caagggtaga tcggaccgtt catgctgcct ataggcatta tgtccctcaa    1080
aaaaaaactc ctttgcctgc atcctgtgta caacatgaca tttttaacca atccaatcta    1140
```

aaaatgtgcc agaatccacc tgtggcccga atcgtgtttg gttcctcttt ctactccact 1200
gcagatgacc aaacctgtcc cgctgccact ttcctcactg atattgggag gagggcaagg 1260
cccagccgaa gttccactaa aaatgcccca ggagaatagg caccggctgg cttgccaaag 1320
ggtttgggtt ttattgcttt ctgtttttc ttttcccgac agcacaaaga agtaagggca 1380
gttattggac aggtgttatt taaacattct attgtaaatg aatgtgttgt ttggttctac 1440
tgcattgtgg agcatgcggg ggaagagaac tgacccaggt aatgaaatgg agcccttccc 1500
tggaactaac cagtccttga tgttgtgtga ctaagtaaag atgataaacc ccatctgctg 1560
ggggtgtcac ttcacactcg gcatgcattg tgaaagcttt ccataccctt ggccattccc 1620
tctctcctct ctctccaacc ccatttatgc aggaggggac tgctaacaag aacgcttcca 1680
tctcaaacct tttctctgcc tgggaaatta ttttatgttt gtttttgaaa taaaggattt 1740
agtttaagat tctaaatttt agagaaacaa acgtaggcct tgtttactaa tagccagaca 1800
tcagaactgc aggtaggtat gttaatgaga tgacttattt ctggcagctc ctggaatcct 1860
aatattgtaa atgagtggga cacacttgca tattgtgacc attctattga ggcccttctc 1920
tgtttaatgc atattatact tgtgctttta actgtggaat ctatttctaa cctaaaaaaa 1980
aaaaaaaaaa 1990

<210> 16
<211> 450
<212> DNA
<213> Homo sapiens

<220>
<221> misc_feature
<222> (355)..(355)
<223> n is a, c, g, or t

<220>
<221> misc_feature
<222> (361)..(361)
<223> n is a, c, g, or t

<220>
<221> misc_feature
<222> (375)..(375)
<223> n is a, c, g, or t

<220>
<221> misc_feature
<222> (380)..(380)
<223> n is a, c, g, or t

<220>
<221> misc_feature
<222> (404)..(404)
<223> n is a, c, g, or t

<220>

<221> misc_feature
<222> (437)..(437)
<223> n is a, c, g, or t

<220>
<221> misc_feature
<222> (445)..(445)
<223> n is a, c, g, or t

<400> 16

```
aaatttctgt attttagttt tgaagtgctt tctatttaaa aataaaaaac atgatttatt      60
ttcatttctg acacagaagt gtttctttta aaaaaaaaag accacatttt aaatttctgc     120
ttaaatgtat ataaagtata catttaagta tactggcact cgcaacaaat gaatccttcc     180
ccagggataa atggattgga aaatttgttt ttcattcaac atttggaaag agaacaaacc     240
tgaaatatgt aatttttaaa attatgtgaa aataatgtga aaaatttcat atagtatttg     300
tgtgaaaatc aggtggaaaa aaacttccat gaagaaccca atttaccaaa attcnccatc     360
nttttaagat ttacnttttn aataccatac tactggtttt aacnggaatt ggggtgtggt     420
atgagggggg ttttgcnggg gaganggga                                       450
```

<210> 17
<211> 508
<212> DNA
<213> Homo sapiens

<400> 17

```
tatttccatg aattcaaagc cttttaatga tgtgaacact tactccccat ttctttttta      60
cattgttaca aaaaatttac atacagtttt ctgaaagtgg cattttgttg gttgttatta     120
tactgatgac acatattaac actttgtatt gaagaagtat cataaaaatc acagggcatt     180
acagattttt gataagaagt agtaatagca ttgtctttta acagctggag gctcccaggc     240
atactctttg gtgagaaatg attaatttta tattttcatt ttgatgagaa tcttttcttg     300
tttttaccag ttataaaaac aaagcttttt ctttgttgtg atactgtgca ctaagactta     360
gtttcttgag ctgatgctaa ataaaatgag atcaatagga atattccggg aggtcgtgag     420
aagtttttag aaaggatggc atctacatat atatggagct ctgaaaactg ttggagagta     480
tgacctggga ctgaaactgt ggagcaca                                        508
```

<210> 18
<211> 475
<212> DNA
<213> Homo sapiens

<220>
<221> misc_feature
<222> (475)..(475)

<223> n is a, c, g, or t

<400> 18

```
tcgacatcta atcctcattc ttatgaactt ggtattatgt gaatccattg ggaaatgaag      60
actcagagag gttaaaccat ttgcccaagg tcacacagct actaagcggt gctaggatta     120
aaccctggca gtttcagtcc ttaaccaaag ggttaagcgc tttacatata tatacctgta     180
tattatcaat tttcaaataa tttgaaatag taaaatgcag ttcctctggt cctgaatatg     240
aggtaatctt cctatggttc agaagacatt tcagcatttc ctaatatatc ataatgagtc     300
cattttggtt gtaccatgat gtagtcattt tgttttatag tatattaaag aatgcagaaa     360
agcatagctc atagttccag tgtcttgctc tgaggttttt ccattcagta gacattttaa     420
gtatatgtac caggcacata aatatccaga taattaaagt ttatatcatt aagcn          475
```

<210> 19
<211> 1061
<212> DNA
<213> Homo sapiens

<400> 19

```
gcgccatcaa tcgccgccgc ctcgtcccgc ttctcggctg aggcgccgcg cggccaggca      60
gcgggtccag gcctcagccg cgcgcccagg ggcctccggg gccctcccgg gtcagcatgc     120
ccggggtgaa actgaccacc caggcctact gcaagatggt gctgcacggc gccaagtacc     180
cgcactgcgc cgtcaacggg ctcctggtgg ccgagaagca gaagccgcgt aaggagcacc     240
tccccctggg cggccccggc gcccaccaca ccctcttcgt ggactgcatc cccctcttcc     300
acggcaccct ggccctcgcc cccatgctgg aggtggctct caccctgatt gattcatggt     360
gcaaagatca tagctacgtg attgctggtt attatcaagc taatgagcga gtaaaggatg     420
ccagtccaaa ccaggttgca gagaaggtgg cctccagaat cgccgagggc ttcagcgaca     480
ctgcgctcat catggtagac aacaccaagt ttacgatgga ctgcgtagcg cctacgatcc     540
acgtgtacga gcaccatgag aacagatggc ggtgcagaga cccacaccat gactactgtg     600
aagactggcc agaggcacag aggatctcag cctcgctcct ggacagccgg tcctacgaga     660
cgctcgtgga tttcgataac cacctggatg acattcggaa tgactggaca aacccagaga     720
tcaataaagc tgtcctacac ttgtgctagg caggcaccgc tgtgactggg ctccgggcct     780
ttcccactac gttgaagaag aaaacctatt tttaaatgta aataaaatat ctggtagcct     840
gtgtggaaag ctgaccgttt taagaagtgg catgtgcctt gaaaggggc agaatgttca      900
gtcggtcgtg tttttaacac agagtctcta gaagaggtgc agacatcccg tctgactgtc     960
cctgtggact ctctcagttg tatgttgcta taatcctcca aatcaaagct ctttctgctt    1020
gtgcaagatt gttcctatta aacagtttta actaaccttt a                        1061
```

<210> 20
<211> 2040
<212> DNA
<213> Homo sapiens

<400> 20

```
gaattccggg cagctcctct tccatctcca gaaatgacct ccaccttcaa cccccgagaa    60
tgtaaactgt ccaagcaaga agggcaaaac tatggcttct tcctgcgaat tgagaaggac   120
accgagggcc acctggtccg ggtggttgag aagtgtagcc cagcagagaa ggctggcctt   180
caagatggag acagagttct taggatcaat ggtgtctttg tggacaaaga agaacatatg   240
caggttgtgg atctggtcag aaagagtggg aattcagtga ctttactagt tctggatggg   300
gattcctatg agaaagcagt gaaaacacgg gtggacttga aagagttggg tcaaagtcag   360
aaggagcaag gtttgagtga taatatactt tcccctgtga tgaatggagg tgtgcaaact   420
tggacccagc cccggctctg ctatctcgtg aaggaaggag gcagctatgg cttctctctg   480
aaaactgtcc aaggtaaaaa gggggtgtac atgactgata ttacacctca aggtgtggct   540
atgagagctg gagttctggc tgatgatcac ttgattgaag tgaatggaga gaatgtagag   600
gatgccagcc atgagaaagt ggttgaaaag gtgaagaagt caggaagccg tgtcatgttc   660
ctgctggtgg acaaagaaac tgacaagcgt catgttgagc agaagataca attcaaaaga   720
gaaacagcca gtttgaaact gttaccccac cagccccgaa ttgtggagat gaagaaagga   780
agcaatggct atggtttcta tctgagggca ggctcagaac agaaaggtca aatcatcaag   840
gacatagatt ctggaagtcc agcagaggag gctggcttga agaacaatga tctggtagtt   900
gctgtcaacg gcgagtctgt ggaaaccctg gatcatgaca gtgtggtaga aatgattaga   960
aagggtggag atcagacttc actgttggtg gtagacaaag agacggacaa catgtacaga  1020
ctggctcatt tttctccatt tctctactat caaagtcaag aactgcccaa tggctctgtc  1080
aaggaggctc cagctcctac tcccacttct ctggaagtct caagtccacc agatactaca  1140
gaggaagtag atcataagcc taaactctgc aggctggcta aaggtgaaaa tggctatggc  1200
tttcacttaa atgcgattcg ggtctgccca ggctcattca tcaaagaggt acagaagggc  1260
ggtcctgctg acttggctgg gctagaggat gaggatgtca tcattgaagt gaatggggtg  1320
aatgtgctag atgaacccta tgagaaggtg gtggatagaa tccagagcag tgggaagaat  1380
gtcacacttc tagtctgtgg aaagaaggcc tatgattatt tccaagctaa gaaaatccct  1440
attgtttcct ccctggctga tccacttgac accccctccag attctaaaga aggaatagtg  1500
gtggagtcaa accatgactc gcacatggca aaagaacggg cccacagtac agcctcacat  1560
tcttcttcca attctgaaga tacagagatg tgatgaaaac aagtaatagc tttggctgtt  1620
tatttgatag ctgtttctgg gtatttaata ggaatccttt ctcaaggaat gagttgtgac  1680
ctgtttactg tctctttaga agaaaaactc cactggaaac cattcaccat gtgtgactgt  1740
cttctgttat catttgtctt acaggcggct attgcagacg gctaatttat gcttaactta  1800
ggaagagata aggcaagagc tagatttttt tcatgtgatc ttttccaagc ttcaacttaa  1860
```

cttaactaca tttctctgta tgatgatgtc tcttacttct acaggttcct tgagcaccaa 1920

agatgattca taactctgta taggtgacag ctgcttataa aagcatctta gcagataagc 1980

ctattaaaat tgtgcttttg taacaatgtt gtggttgcta gaataaatac catgaacccg 2040

<210> 21
<211> 633
<212> DNA
<213> Homo sapiens

<400> 21

tttaaaagcc actaattatc tgttttttat tttgtaagta acaagatata gacatttgaa 60

tgccaatgtc ttattctgga gagacactgg agctgaagtt caacaatgat cacacttatt 120

acctggcaat aaaaacacaa ccatctttcc agtcaggtca aaatatccta ctttttgcct 180

ttctaccaaa tcccaaacat tcacagtttt tcaaggacca ctaataaaat acaggaagct 240

tttaaagaca gtaagagaac acctagtgta agttaggtga attaaagatg gcaaaggaga 300

ttacatcctc aacactgaca gcttccaaga cttagaaaag agattgttcc ttgcttctaa 360

aattgttcta ttttcctctg taggaaaatg aaagtttttt cttacaaata ttaaataatc 420

aaagtactta cgcaaaatta atctgctcct caatgagatg agcactccat ttaaatgatc 480

tttacagatc cctgaagttg ctgtcctgtc actgtattta agtgatggat attcaattga 540

attattctgc ataaataatt ctggtcaacc cagacgtata gtagtatgat gggtcagata 600

cagtcaactg ttcaataaaa atgcagatgt ctg 633

<210> 22
<211> 1878
<212> DNA
<213> Homo sapiens

<400> 22

```
ctgcgtttct cctcaaacct aacgatgccg ccggagcgga ggagacgaat gaaactggac      60
cggagaaccg gagcgaagcc gaagcggaag cccggaatga ggccggactg gaaagccgga     120
gcggggccag gcgggcctcc ccaaaagcct gccccttcat cccagcggaa accgccggcc     180
cggccgagcg cggcggccgc tgcgattgca gtcgcggcgg cggaggaaga gagacggctc     240
cggcagcgga accgcctgag gctggaggag gacaaaccgg ccgtggagcg gtgcttggag     300
gagctggtct tcggcgacgt cgagaacgac gaggatgcgt tgctgcggcg tctgcgaggc     360
ccgagggttc aagaacatga agactcgggt gactcagaag tggagaatga agcaaaaggt     420
aattttccac ctcaaaagaa gccagtttgg gtggatgaag aagatgaaga tgaggaaatg     480
gttgacatga tgaacaatcg gtttcggaag gatatgatga aaaatgctag tgaaagtaaa     540
ctttcgaaag acaaccttaa aaagagactt aaagaagaat tccaacatgc catgggagga     600
gtacctgcct gggcagagac tactaagcgg aaaacatctt cagatgatga aagtgaagag     660

gatgaagatg atttgttgca aaggactggg aatttcatat ccacatcaac ttctcttcca     720
agaggaatct tgaagatgaa gaactgccag catgcgaatg ctgaacgtcc tactgttgct     780
cggatctcat ctgtgcagtt ccatcccggt gcacagattg tgatggttgc tggattagat     840
aatgctgtat cactatttca ggttgatggg aaaacaaatc ctaaaattca gagcatctat     900
ttggaaaggt ttccaatctt taaggcttgt tttagtgcta atggggaaga agttttagcc     960
acgagtaccc acagcaaggt tctttatgtc tatgacatgc tggctggaaa gttaattcct    1020
gtgcatcaag tgagaggttt gaaagagaag atagtgagga gctttgaagt ctccccagat    1080
gggtccttct tgctcataaa tggcattgct ggatatttgc atttgctagc aatgaagacc    1140
aaagaactga ttggaagcat gaaaattaat ggaagggttg cagcatccac attctcttca    1200
gatagtaaga aagtatacgc ctcttcgggg gatggagaag tttatgtttg ggatgtgaac    1260
tcaaggaagt gccttaacag atttgttgat gaaggcagtt tatatggatt aagcattgcc    1320
acatctagga atggacagta tgttgcttgt ggttctaatt gtggagtggt aaatatatac    1380
aatcaagatt cttgtctcca agaaacaaac ccaaagccaa taaaagctat aatgaacttg    1440
gttacaggtg ttacttctct gaccttcaat cctactacag aaatcttggc aattgcttca    1500
gaaaaaaatg aaagaagcag tcagattggt tcatcttcct tcctgtacag tattttcaaa    1560
cttcccagtc attaaaaata agaatatttc tcatgttcat accatggatt tttctccgag    1620
aagtggatac tttgccttgg ggaatgaaaa gggcaaggcc ctgatgtata ggttgcacca    1680
ttactcagac ttctaaagag actatttgaa gtccagttga gtcacaagag aagcctgtct    1740
tgatatatca tctcagaaac tttcctgaat atgtgataat atatggaaaa tgatttatag    1800
atccagctgt gcttaagagc cagtaatgtc ttaataaaca tgtggcagct tttgtttgaa    1860
aaaaaaaaaa aaaaaaaa                                                  1878
```

<210> 23
<211> 3426
<212> DNA
<213> Homo sapiens

<400> 23

```
aattccgggc ggcggcggcc gaggctgaag gaagatggcg gacggcgtgg accacataaa      60
catttacgcg gatgtcggcg aagagttcaa ccaggaagct gaatatggtg ggcatgatca     120
gatagatttg tatgacgatg tcatatctcc atctgcaaat aatggagatg ccccagaaga     180
ccgagattac atggatactc tcccaccaac tgttggtgat gatgtgggta aaggagcagc     240
accaaatgtt gtctatacat atactggaaa gagaattgca ttatatattg gaaatctaac     300
atggtggaca acagatgaag acttaactga agcagttcat tctttgggag taaatgatat     360
tttggagata aaatttttg aaaatcgagc aaatggccag tcaaaggggt ttgcccttgt     420
tggtgttgga tctgaagcat cttcaaaaaa gttaatggat ctgttaccta aaagagaact     480
```

tcatggtcag aatcctgttg taactccatg caataaacag ttcctgagtc aatttgaaat 540
gcagtccagg aaaactacac aatcaggaca aatgtctggg gaaggtaaag ctggtcctcc 600
aggaggcagt tcccgtgcag catttccaca aggtggtaga ggacggggcc gttttccagg 660
ggctgttcct ggtggggaca gatttcctgg gccagcagga ccaggagggc cacccccacc 720
ttttccagct ggacagactc caccacgtcc acccttaggt cctccaggcc cacctggtcc 780
accaggtcct ccacctcctg gtcaggttct gcctcctcct ctagctgggc ctcctaatcg 840
aggagatcgc cctccaccac cagttctttt tcctggacaa ccttttgggc agcctccatt 900
gggtccactt cctcctggcc ctccacctcc agttccaggc tacggccccc ctcctggccc 960
accacctcca caacagggac cacctccacc tccaggcccc tttccacctc gtccacccgg 1020
tccacttggg ccacccctta cactagctcc tcctccgcat cttcctggac cacctccagg 1080
tgccccaccg ccagctccgc atgtgaaccc agctttcttt cctccaccaa ctaacagtgg 1140
catgcctaca tcagatagcc gaggtccacc accaacagat ccatatgggc gacctccacc 1200
atatgatagg ggtgactatg gcccccctgg aagggaaatg gatactgcaa gaacgccatt 1260
gagtgaagct gaatttgaag aaatcatgaa tagaaatagg gcaatctcaa gcagtgctat 1320
ttcgagagct gtgtctgatg ccagtgctgg tgattatggg agtgctattg agacactggt 1380
aactgcaatt tctttaatta aacaatccaa agtatctgct gatgatcgtt gcaaagttct 1440
tattagttct ttgcaagatt gccttcatgg aattgagtcc aagtcttatg gttctggatc 1500
aagacgtgaa cgatcaagag agagggacca tagtagatca cgagaaaaga gtcgacgtca 1560
taaatcccgt agtagagacc gtcatgacga ttattacaga gagagaagca gagaacgaga 1620
gaggcaccgg gatcgtgacc gagaccgtga ccgagagcgt gaccgagagc gcgaatatcg 1680
tcatcgttag aagctgaagg aagaggatca ccttccaaga caaaacagtc ttcatgggcc 1740
aaaaatgacg cttgtccagc agtttgcttc ttgtgattga actgaacctg taaggattca 1800
tggataaaat gaacaggaat agatctgaat aaagcaaatc tgcataaatg gtaaccagta 1860
gctctacttt tattttttat gttgcttaac tgttttattt gaaggaaacc tgtgtgattt 1920
aaaaagttat agcttttgca actttattac tggttatata catttggcca ttatgatgtg 1980
caagcaattg gaaaaaaagt caagtaaatg cttgtttttg tagtagtttg ttcttgttaa 2040
aaatgtttat atgataatgt ctgtaaacag catcactttg attacaatag atgtagtgtt 2100
gtaataaact gtttaatggg gctgatgtgt aaagctgttc aagttatttg atgtttacac 2160
ctcagggaaa gtcttgtgtt cagcaatatc taaagataat gttactatga caacattttt 2220
actgtccttt aaagcattgc aatagcgttt ttggatatgc ctcaatctaa tcttgcgttc 2280
agtgaattaa acatagtaat taagtgtctt ttgcccttga ttttgatatt agaataggtg 2340
attacatgga tatttaatat ttctatattc tgcttttcta gctgttttta cctagttagc 2400

```
ttgtgacttt gctgaatggt atgtaaactt gtaaaaatag agatttgaca gacatagcaa     2460
tctagtcaat gtgtaagggg tcaaaaaaaa cagaggtttt aacacataag taaaaacccg     2520
tacatatttg atgtgtaatg caggttaatt acaacacaga tgtaccgaaa cacttaattg     2580
tgaaccgcta acattgaaga aattttgaca attccgattt gatgctgcaa ttacttgctg     2640
tttttattga tcttatggtt tatttcttaa gccatagtca gtgtaaatac agccctgcag     2700
caggtaaatg tgagtaaaga gagccttata ttttccaatt ggtataaaat ttttgaagga     2760
tgtgatgttc attaacattc ggttgtattc cccagtattt gtaatgggaa attacagata     2820
aaccgtgtct gcacagttta aggaatacta tgtatattca tgcaccgtat tgattcatgc     2880
tatagttact taatcaaaga ttttttttcaa acctgcctta catataggcc cactttaaaa     2940
gcacctgact agcatgtgtt cttgattgca aaattggcag aggcagggtg tcaacttgat     3000
taggtgtttt tatgggaatg taatttgaaa tcactacttc agaaatttga cttaaaattc     3060
ttgagcacgt taatatgttt ttaagatctg attatctttg agagatcttc tgttaataca     3120
cattggttgt taaagagtac ccaaattcta ggacaatgct taaagtgtta aaatacccta     3180
gatactgtgt tatgtgcaac tgtagaaacc ctccagaaat ttccactgct gttcttcact     3240
ttcatcttgt ctgctatcaa accacttctg acaaaattag ctgttttgaa ttacccatat     3300
cactgccagt tttattttaa aatattttgt gtttgaagta tctgtgcatg ggatcgttga     3360
tgtttatcag aactgttcac tttcagaaat gatttttttaa agcattttgt tgaaatgcgg     3420
ttgctt                                                                3426
```

<210> 24
<211> 5401
<212> DNA
<213> Homo sapiens

<400> 24

```
tcactctcgc tgccgctgct ccgccccatc cccttctgtt tttctctctc attctccagt       60
ggcggcggcg gggaaggcgg aggcagaggc agcagcagcc gcgctggctg caatgaatga      120
tccccccagct tgggggggagg actccaggtg agcctctgcc ctcgggaggc ccgggacccc      180
cggccgccca cgaccggcag cccacgctat ggatccctag aggaaggagg agaagacagc      240
tcgccgccca cccccatccc attttcctct tcctttatct cattgttgcc gaagctgttt      300
acggcagcgc tccctctgct ccagcatggg gcgggctccg ggcacggctg ctcggcaggc      360
gctgctcccg cggcgactgg gggattctgc ctaattcacc tcccagccgg tgcagagagg      420
accggagagc ggtggaggcc cggactgcag cagcgttggg gccacctccc agcgtcccca      480
ccctaggagg ctgcatgcgg attgaagagc tgcgcctggg ggctgggccg gccccgctga      540
tcccgaccta gcgagcagga tagcaggacc gcccaggctg cggaggggct cgggggcagg      600
```

```
aaggtcagag cagcaagatg gccagtaaga ccaaggccag cgaggccctc aaggtggtgg      660
cccggtgccg ccccctcagc aggaaggagg aggctgctgg tcacgagcag atcctgacca      720
tggacgtgaa actgggccag gtgaccctgc ggaaccccg cgccgccccg ggggagctgc       780
ccaagacctt cacctttgac gccgtgtatg atgccagctc caagcaggcc gacctgtatg      840
acgaaaccgt gaggcccctg atagactccg tgctccaggg tttcaatggc acggtgtttg      900
cctatggcca gacgggcact ggcaagacct ataccatgca ggggacctgg gtggagcccg      960
agctgcgcgg ggtcatcccg aatgcctttg agcacatctt cacccacatc tcccgctccc     1020
agaaccaaca gtacctggtc cgggcctcct atttggagat ctaccaggaa gagattcgag     1080
acctgctctc caaggagccg ggcaagaggc tagagctgaa agagaacccc gagactggcg     1140
tctacatcaa ggacctctcc tccttcgtca ccaagaatgt caaggagatt gagcatgtga     1200
tgaacctggg gaaccagacc cgggctgtgg gcagcaccca catgaatgag gtcagctccc     1260
gctcccatgc catcttcatc atcactgtgg agtgcagcga acgtggctct gatggccagg     1320
accacatccg agtgggcaag ctcaacctcg tggacctggc tggcagcgag aggcagaaca     1380
aggcaggccc caacacagcg ggaggggcag ccacaccatc ctcgggtggc ggtggtggcg     1440
gtggaggcag tggtggtggt gctggtggag agaggcctaa ggaagcctcc aaaatcaacc     1500
tctcattatc tgccctgggc aacgtgattg ctgccctggc gggcaacagg agcacccaca     1560
ttccctaccg ggactccaag ctgacccggc tgctccagga ctccctgggg gggaatgcca     1620
agaccatcat ggtagccaca ctggggccag cttctcacag ctacgatgag agcctctcca     1680
ccttgcgctt tgccaaccga gccaagaaca tcaagaacaa gccccgggtg aacgaggacc     1740
ccaaggacac actgctgcgg gaattccaag aggagattgc ccgcctgaag gcccagctgg     1800
agaagagggg gatgctgggg aagcggcccc ggaggaagag cagccgcagg aagaaggccg     1860
tgtccgcccc gcctgggtac cctgagggcc cagtgattga ggcctgggtg gcagaagagg     1920
aggatgacaa caacaacaac caccgcccgc cccagcccat cctggagtca gccttggaga     1980
agaacatgga gaattacctg caggaacaga aggagcggct ggaggaggag aaggcagcca     2040
tccaggatga ccgcagcctg gtgagcgagg agaagcagaa gctgctggag gagaaggaga     2100
agatgctgga ggacctgcgg cgggaacagc aggccacaga gctgcttgcg gccaagtaca     2160
aggccatgga gagcaagctc ctcatcgggg gcaggaacat catggatcac accaacgaac     2220
agcagaagat gttggaactg aagaggcagg agattgccga gcagaaacgt cgtgagcggg     2280
agatgcagca ggagatgatg ctccgggacg aggagactat ggagctccgg ggcacctaca     2340
catccctgca gcaggaggtg gaggtcaaaa ccaagaaact caagaagctc tacgccaagc     2400
tgcaggcggt gaaggcggag atccaggacc agcatgatga gtatatccgc gtgcggcagg     2460
acctggagga ggcgcagaac gagcagaccc gcgaactcaa gctcaagtac ctaatcatcg     2520
```

agaacttcat cccgccggag gagaagaaca agatcatgaa ccggcttttc ctggactgtg 2580
aggaggagca gtggaagttc cagccactgg tgccagccgg cgtcagtagc agccagatga 2640
agaagcggcc aacatctgca gtgggctaca agaggcctat cagccagtat gctcgggttg 2700
ccatggcaat ggggtcccac cccaggtaca gggctgaaaa cataatgttt ctggagttgg 2760
atgtgtcccc tccagctgtc tttgagatgg aattctctca cgaccaagaa caagaccctc 2820
gtgcgctaca catggagagg ctcatgcgat tggacagctt tctggaaaga ccttccacgt 2880
ctaaagtccg aaagtccaga tcctggtgcc agagtcctca gcggcctcca ccttccacca 2940
cacatgcctc cctggcctct gcttctctgc gccctgcaac agtggcggac catgagtgac 3000
aaccatcacg tcaggctgcc catccaatag actcctggga tggggcagcc aaccctggct 3060
catctcatct gccgcttggt gcgtgtgcgt gtgcgtgcat gtgcgtgtgc gtgtgtgcag 3120
gggtgagaat ctggcagatg gtgcctctgc ctgctcttct tcgcctcctt tatttaattc 3180
atgttattta ttcgcggagc tctgttcgtg ttggggagat gccctcgcct gagccgtctg 3240
ggcctaccgt ggtcactgcg tagcctcttt ttcttctgac ttgagagctc ccccagtcag 3300
atctcaggct tgtccccctg tcagctgcct ccagaaggga aggtagccag tgcctgagaa 3360
gacagtccct tttctaccca ccgcactcca taacctccat cttctcccac actgatggcg 3420
agcagcccct gagcactttc tgggactggg agactgcttg gtgttccctg aggacaagag 3480
acatcctgac agtgttgggc atctgctccc cgtggacaca gccccactct ccactttctg 3540
agcctcagac aacctcattc agcctcttgg gctccttttc aaggacatta ataacctcac 3600
caacatagct catgcccttc agctttgaca agaactcacg gcttcccaaa ctctgctttc 3660
tgcccacctt ggatgggaac tgtggaccaa gcaattacca tcgccttgga acctgcagga 3720
aatggaacag caattgagac aacttgaaca gtcatcaacg gaagtccctc cactggattc 3780
ctttgtttct gtcccctccg aggagtcatt ttggtcgaca ggctctcaag gcaactcccc 3840
attttcaaga ggctgctcct gcctgcttcg atcatttctc cctgcagctg cctagacccc 3900
gttcacagtg ggaggagtca atgtcattct acccctcgct aaacgaagat attaacatct 3960
attgcttttt cccttcatct gtcacaggaa acagaagccc aggcacaatc ttttccagct 4020
ttgcctgtta cccctgtttc tgaattgcat ctttaaggta ttattttgtt gacaatagat 4080
cctttattca ctagttacgc aaattggttc ctaggggat actccttacc ttcctttgtg 4140
atggcccaaa atgtctctag gtatctcaag tgataagtaa atttctacaa aaaaaaatgg 4200
ttaatgttca ttgactggct ttttaagtgt atattttgga ggacgggtga agaggtcata 4260
acgaaagcaa gcgagtgaat taggatttca aagtgcccta atagtgtgag tctccagttc 4320
ctagaatatg aagagtgctg tcgttggggt gaaaccatga gactgacaga tctgcctgaa 4380
atgggggtg tgggaggtgg tggcgggggt tattctcttt ccttcaggaa atgaaccctt 4440

```
cttacatcat tcaagttctg ctctgaggat caagcttggg tctgatttaa ctcagcgaca      4500
ctgtcatttc tgcttcatta ctggactaga gggttgagcc acccacttgc catttgctcc      4560
tgtccttcca ggaaatcaca attttcatca gagcccaaga gattatttga gactcaggat      4620
tcagatcaga ggttcgactg tggctgggac aggagttgtg tgtagaaatt caccaggtgg      4680
cctgagcgca gggggacctc cagggctgcg ttgagcagcc tctcccactg acctctttct      4740
cgtttgtgga caaagcagca cgtatcacct cattcatcac ttggacacat cgcctttgca      4800
ttgtcttgtc acacctccct cacagtctta tagcacaata tacccaaatc agccccccca      4860
gtccgagggc tgggcccaag gtatggtcgg aggaggagct cctgcctgcg gttttgtgta      4920
tgtgtgtatg tgtgtgcgtg tttgtgtgcg tgtttacctc cacaggggac actctacact      4980
cagtgtaaga tctgctggga acagggccac caggagtggc tggatctcag tctctctgtc      5040
tctctttctc tccttttcct tttggtgtat caaatatttg attgacaaag taagggcctt      5100
gattaggacc aaattctcgt gtgttgctat ggtctttatt taggacaaca attaacaatg      5160
cagtggccca ttcttgtcac tctacacata tgactatacg ggacatatgt aatatataaa      5220
tatatatata aaacattccc ctctgtcccc ttggcttcgg atggaggcct ttctgttgag      5280
ctgaaatgca cctgcagctg ggtgctgcca gcagcttgca ggccccagcc ctgttccaat      5340
caatgcagtt gacaataaag gaatgagtat cgtcacggaa aaaaaaaaaa aaaaaaaaaa      5400
a                                                                      5401
```

<210> 25
<211> 1280
<212> DNA
<213> Homo sapiens

<400> 25

```
taaacaatgg tatcaacgca aagtaagcgg gcagccgcct gcatctgtat ccagcgccag      60
gtcccgccag tcccagtgcg cgcgcccccc agtcccgcac ccgttcggcc aggctaagtt     120
agccctcacc atgcggtcaa aggaggcagc aagtgcatca aatacctgct gttcggattt     180
aacttcatct tctggcttgc cgggattgct gtccttgcca ttggactatg ggtccgattc     240
gactctcaga ccaagagcat cttcgagcaa gaaatttcct aataataata attccagctt     300
ctacacagga gtctatattc tgatcggagc cggcgccctc atgatgctgg tgggcttcct     360
gggctgctgc ggggctgtgc aggagtccca gtgcatgctg ggactgttct tcggcttcct     420
cttggtgata ttcgccattg aaatagctgc ggccatctgg ggatattccc acaaggatga     480
ggtgattaag gaagtccagg agttttacaa ggacacctac aacaagctga aaaccaagga     540
tgagccccag cgggaaacgc tgaaagccat ccactatgcg ttgaactgct gtggtttggc     600
tgggggcgtg gaacagttta tctcagacat ctgccccaag aaggacgtac tcgaaacctt     660
caccgtgaag tcctgtcctg atgccatcaa agaggtcttc gaccaataaa ttccacatca     720
tcggcgcagt gggcatcggc attgccgtgg tcatgatatt tggcatgatc ttcagtatga     780
tcttgtgctg tgctatccgc aggaaccgcg agatggtcta gagtcagctt acatccctga     840
gcaggaaagt ttacccatga agattggtgg gatttttttgt ttgtttgttt tgttttgttt     900
gttgtttgtt gtttgttttt ttgccactaa ttttagtatt cattctgcat tgctagataa     960
aagctgaagt tactttatgt ttgtctttta atgcttcatt caatattgac atttgtagtt    1020
gagcgggggg tttggtttgc tttggtttat attttttcag ttgtttgttt ttgcttgtta    1080
tattaagcag aaatcctgca atgaaaggta ctatatttgc tagactctag acaagatatt    1140
gtacataaaa gaattttttt gtctttaaat agatacaaat gtctatcaac tttaatcaag    1200
ttgtaactta tattgaagac aatttgatac ataataaaaa attatgacaa tggccaaaaa    1260
aaaaaaaaaa aaaaaaaaaa                                                1280
```

<210> 26
<211> 3345
<212> DNA
<213> Homo sapiens

<400> 26

```
gcggccgggg cctggggctg cctgccgggc ggccgggcgc ggcgagccca gggaggcagc    60
gtccatggag caaaaggaat gccaggatcc tgcacaggca gacgcgggcc agcctcagca   120
ccgacagccg acgcgcagat agcagagcca tccttggggt tgaaccatga ttccggtgac   180
agagctccgc tactttgcgg acacgcagcc agcataccgg atcctgaagc cgtggtggga   240
tgtgttcaca gactacatct ctatcgtcat gctgatgatt gccgtcttcg gggggacgct   300
gcaggtcacc caagacaaga tgatctgcct gccttgtaag tgggtcacca aggactcctg   360
caatgattcg ttccggggct gggcagcccc tggcccggag cccacctacc ccaactccac   420
cattctgccg acccctgaca cgggccccac aggcatcaag tatgacctgg accggcacca   480
gtacaactac gtggacgctg tgtgctatga gaaccgactg cactggtttg ccaagtactt   540
cccctacctg gtgcttctgc acacgctcat cttcctggcc tgcagcaact tctggttcaa   600
attcccgcgc accagctcga agctggagca ctttgtgtct atcctgctga agtgcttcga   660
ctcgccctgg accacgaggg ccctgtcgga gacagtggtg gaggagagcg accccaagcc   720
ggccttcagc aagatgaatg ggtccatgga caaaaagtca tcgaccgtca gtgaggacgt   780
ggaggccacc gtgcccatgc tgcagcggac caagtcacgg atcgagcagg gtatcgtgga   840
ccgctcagag acgggcgtgc tggacaagaa ggagggggag caagccaagg cgctgtttga   900
gaaggtgaag aagttccgga cccatgtgga ggagggggac attgtgtacc gcctctacat   960
gcggcagacc atcatcaagg tgatcaagtt catcctcatc atctgctaca ccgtctacta  1020
cgtgcacaac atcaagttcg acgtggactg caccgtggac attgagagcc tgacgggcta  1080
```

ccgcacctac cgctgtgccc accccctggc cacactcttc aagatcctgg cgtccttcta 1140
catcagccta gtcatcttct acggcctcat ctgcatgtat acactgtggt ggatgctacg 1200
gcgctccctc aagaagtact cgtttgagtc gatccgtgag gagagcagct acagcgacat 1260
ccccgacgtc aagaacgact tcgccttcat gctgcacctc attgaccaat acgacccgct 1320
ctactccaag cgcttcgccg tcttcctgtc ggaggtgagt gagaacaagc tgcggcagct 1380
gaacctcaac aacgagtgga cgctggacaa gctccggcag cggctcacca agaacgcgca 1440
ggacaagctg gagctgcacc tgttcatgct cagtggcatc cctgacactg tgtttgacct 1500
ggtggagctg gaggtcctca agctggagct gatccccgac gtgaccatcc cgcccagcat 1560
tgcccagctc acgggcctca aggagctgtg gctctaccac acagcggcca agattgaagc 1620
gcccgcgctg gccttcctgc gcgagaacct gcgggcgctg cacatcaagt tcaccgacat 1680
caaggagatc ccgctgtgga tctatagcct gaagacactg gaggagctgc acctgacggg 1740
caacctgagc gcggagaaca accgctacat cgtcatcgac gggctgcggg agctcaaacg 1800
cctcaaggtg ctgcggctca agagcaacct aagcaagctg ccacaggtgg tcacagatgt 1860
gggcgtgcac ctgcagaagc tgtccatcaa caatgagggc accaagctca tcgtcctcaa 1920
cagcctcaag aagatggcga acctgactga gctggagctg atccgctgtg acctggagcg 1980
catcccccac tccatcttca gcctccacaa cctgcaggag attgacctca aggacaacaa 2040
cctcaagacc atcgaggaga tcatcagctt ccagcacctg caccgcctca cctgccttaa 2100
gctgtggtac aaccacatcg cctacatccc catccagatc ggcaacctca ccaacctgga 2160
gcgcctctac ctgaaccgca acaagatcga gaagatcccc acccagctct tctactgccg 2220
caagctgcgc tacctggacc tcagccacaa caacctgacc ttcctccctg ccgacatcgg 2280
cctcctgcag aacctccaga acctagccat cacggccaac cggatcgaga cgctccctcc 2340
ggagctcttc cagtgccgga agctgcgggc cctgcacctg ggcaacaacg tgctgcagtc 2400
actgccctcc agggtgggcg agctgaccaa cctgacgcag atcgagctgc ggggcaaccg 2460
gctggagtgc ctgcctgtgg agctgggcga gtgcccactg ctcaagcgca gcggcttggt 2520
ggtggaggag gacctgttca acacactgcc acccgaggtg aaggagcggc tgtggagggc 2580
tgacaaggag caggcctgag cgaggccggc ccagcacagc aagcagcagg accgctgccc 2640
agtcctcagg cccggagggg caggcctagc ttctcccaga actcccggac agccaggaca 2700
gcctcgtggc tgggcaggag cctggggccg cttgtgagtc aggccagagc gagaggacag 2760
tatctgtggg gctggcccct tttctccctc tgagactcac gtcccccagg gcaagtgctt 2820
gtggaggaga gcaagtctca agagcgcagt atttggataa tcagggtctc ctccctggag 2880
gccagctctg ccccaggggc tgagctgcca ccagaggtcc tgggaccctc actttagttc 2940
ttggtattta tttttctcca tctcccacct ccttcatcca gataacttat acattcccaa 3000

gaaagttcag cccagatgga aggtgttcag ggaaaggtgg gctgcctttt ccccttgtcc 3060
ttatttagcg atgccgccgg gcatttaaca cccacctgga cttcagcaga gtggtccggg 3120
gcgaaccagc catgggacgg tcacccagca gtgccgggct gggctctgcg gtgcggtcca 3180
cgggagagca ggcctccagc tggaaaggcc aggcctggag cttgcctctt cagtatttgt 3240
ggcagtttta gtttttgtt tttttttt taatcaaaaa acaatttttt taaaaaaaa 3300
gctttgaaaa tggatggttt gggtattaaa aaaaaaaaa aaaaa 3345

<210> 27
<211> 4762
<212> DNA
<213> Homo sapiens

<400> 27

atgcggcgcg gccccggagg cagcagcagc ggcggcggca gccggagcag taggcacccg 60
agcagcgcca gcggccgagc gggcggcttc ctggcctggg cgctccggtg gcggcggagg 120
tgcgcgcgga gccatggtta tcatgtcgga gttcagcgcg gaccccgcgg gccagggtca 180
gggccagcag aagcccctcc gggtgggttt ttacgacatc gagcggaccc tgggcaaagg 240
caacttcgcg gtggtgaagc tggcgcggca tcgagtcacc aaaacgcagg ttgcaataaa 300
aataattgat aaaacacgat tagattcaag caatttggag aaaatctatc gtgaggttca 360
gctgatgaag cttctgaacc atccacacat cataaagctt taccaggtta tggaaacaaa 420
ggacatgctt tacatcgtca ctgaatttgc taaaaatgga gaaatgtttg attatttgac 480
ttccaacggg cacctgagtg agaacgaggc gcggaagaag ttctggcaaa tcctgtcggc 540
cgtggagtac tgtcacgacc atcacatcgt ccaccgggac ctcaagaccg agaacctcct 600
gctggatggc aacatggaca tcaagctggc agattttgga tttgggaatt tctacaagtc 660
aggagagcct ctgtccacgt ggtgtgggag ccccccgtat gccgccccgg aagtctttga 720
ggggaaggag tatgaaggcc cccagctgga catctggagc ctgggcgtgg tgctgtacgt 780
cctggtctgc ggttctctcc ccttcgatgg gcctaacctg ccgacgctga gacagcgggt 840
gctggagggc cgcttccgca tccccttctt catgtctcaa gactgtgaga gcctgatccg 900
ccgcatgctg gtggtggacc ccgccaggcg catcaccatc gcccagatcc ggcagcaccg 960
gtggatgcgg gctgagccct gcttgccggg acccgcctgc cccgccttct ccgcacacag 1020
ctacacctcc aacctgggcg actacgatga gcaggcgctg ggtatcatgc agaccctggg 1080
cgtggaccgg cagaggacgg tggagtcact gcaaaacagc agctataacc actttgctgc 1140
catttattac ctcctccttg agcggctcaa ggagtatcgg aatgcccagt gcgcccgccc 1200
cgggcctgcc aggcagccgc ggcctcggag ctcggacctc agtggtttgg aggtgcctca 1260
ggaaggtctt tccaccgacc ctttccgacc tgccttgctg tgcccgcagc cgcagacctt 1320
ggtgcagtcc gtcctccagg ccgagatgga ctgtgagctc cagagctcgc tgcagtggcc 1380

cttgttcttc ccggtggatg ccagctgcag cggagtgttc cggccccggc ccgtgtcccc 1440
aagcagcctg ctggacacag ccatcagtga ggaggccagg caggggccgg gcctagagga 1500
ggagcaggac acgcaggagt ccctgcccag cagcacgggc cggaggcaca ccctggccga 1560
ggtctccacc cgcctctccc cactcaccgc gccatgtata gtcgtctccc cctccaccac 1620
ggcaagtcct gcagagggaa ccagctctga cagttgtctg accttctctg cgagcaaaag 1680
ccccgcgggg ctcagtggca ccccggccac tcaggggctg ctgggcgcct gctccccggt 1740
caggctggcc tcgcccttcc tggggtcgca gtccgccacc ccagtgctgc aggctcaggg 1800
gggcttggga ggagctgttc tgctccctgt cagcttccag gagggacggc gggcgtcgga 1860
cacctcactg actcaagggc tgaaggcctt tcggcagcag ctgaggaaga ccacgcggac 1920
caaagggttt ctgggactga acaaaatcaa ggggctggct cgccaggtgt gccaggtccc 1980
tgccagccgg gccagcaggg gcggcctgag ccccttccac gcccctgcac agagcccagg 2040
cctgcacggc ggcgcagccg gcagccggga gggctggagc ctgctggagg aggtgctaga 2100
gcagcagagg ctgctccagt tacagcacca cccggccgct gcacccggct gctcccaggc 2160
cccccagccg gcccctgccc cgtttgtgat cgccccctgt gatggccctg ggctgcccc 2220
gctccccagc accctcctca cgtcggggct cccgctgctg ccgcccccac tcctgcagac 2280
cggcgcgtcc ccggtggcct cagcggcgca gctcctggac acacacctgc acattggcac 2340
cggccccacc gccctccccg ctgtgccccc accacgcctg gccaggctgg ccccaggttg 2400
tgagcccctg gggctgctgc aggggactg tgagatggag gacctgatgc cctgctccct 2460
aggcacgttt gtcctggtgc agtgagggca gccctgcatc ctggcacgga cactgactct 2520
tacagcaata acttcagagg aggtgaagac atctggcctc aaagccaaga actttctaga 2580
agcgaaataa gcaatacgtt aggtgttttg gctttttagt ttatttttgt tttatttttt 2640
tcttgcactg agtgacctca actttgagta gggactggaa actttaggaa gaaagataat 2700
tgaggggcgt gtctgggggc gggggcagga ggggagcggg gtggagggaa cacgtgcagt 2760
gccgtggtgt ggggatctcg gcccctctct ctgggttcgt cgtggttgag atgattacct 2820
cggacgtcta cggaaacgag cgggcgcatt gttgtccgct tgtgtgtgtg tgtgtgtgtg 2880
tgtgtgtgcg cgtgcattga ttactatcca tttctttagt caacgctctc cacttcctga 2940
tttctgcttt aaggaaaact gtgaactttc tgcttcatgt atcagtttta aagcagccca 3000
ggcaaagatc atctacagat tctaggaatt ctctcccctg aaatcaaaac ctggaagact 3060
tttttttctt attttagttg agaagtttca taaactgctc aaggattagt tttccaggac 3120
tctgcggagg aacggcagga agaacctcag agagggcaga ggtgacttca aagtgctggg 3180
gactccgtcc tgagggtcac ttggccctga gcccctgcgt gcccttgcgg aagcccagaa 3240
gcttcttcct gctgcacctc ccgtttccgc tgctgctgac gtttatgcat ttcatgatgg 3300

ggtccaacaa gaacacctga cttgggtgaa gttgtgcaat attggaggct gactgtaggg 3360

ctgggcagct gggagacagg ctcatggctc atggctcatg gctcagggcg gtgcctgcca 3420

tgggccggga ccccccctccc caccccccac ctaggctttt tgggttttgt tcaaggaagg 3480

taaagtgaga ggtttaggtc agtgttttta agtttttgtt ttttttttaa agcaaatcct 3540

gtatatgtat ctacatggga gacaggtaga cactacttat ttgttacatt ttgtactaca 3600

cgtttgtgtt ccaggtttca gcttccctcg ctcctgttgt taagaagcgt ccctgtcagc 3660

acaggtgtgc attgaggaag gggccccagg gccttcgctc cctcagcact ggggtggagg 3720

cggcaggaag gggcggccct tacctggcag gtctgggcgc acctttagca ggtggactcc 3780

gtggggctcc accagccaga agcctttgga aggcaacgaa ggcaatgctg ctccctgagt 3840

ccagtccccg cccccaaacc cagcccaggt gccttcagct acttcggctt cttaaaccct 3900

gcagtgttaa acagaggcat tgagaaaggg gaaaggcggg tatttttaaa agccaaagat 3960

tgacccagtt acttgagggt agggaggcgg gcccagtgca ggaggctgca tccctggcct 4020

gctggtgccc accgggggct gtgcctgtgc cgggccgcag ggaagctggc tgccccccatt 4080

cctgctgctg ctgctgctgc tgctctgtgg ctgtttcaaa gactgggcga aaggctgtcc 4140

ggagggcaga ccaggtgcct tgccgcagag aaaacaccaa agtctcctgt tcgctcataa 4200

agaagttttt gggatgggag agaatccaga ccatcttggg gcagccaggc ccttgccttc 4260

atttttacag aggtagcaca attgattcca acacaaaact tccccttttt aaaatgattt 4320

ctgttctaat gccatagatc aaaggcctca gaaaccattg tgtgtttcct ctttgaagca 4380

atgacaagca ctttactttc acggtggttt ttgtttttttc ttattgctgt ggaacctctt 4440

ttggaggacg ttaaaggcgt gttttacttg tttttttaag agtgtgtgat gtgtgttttg 4500

tagatttctt gacagtgctg taatacagac ggcaatgcaa tagcctattt aaagacacta 4560

cgtgatctga ttgagatgta catagttttt tttttacca taactgaatt attttatctc 4620

ttatgttaac atgagaaatg tatgccaaat gattagttga tgtatgtttt ttaatttaat 4680

atttaaataa aatatttggg agtataaaaa aaaaaaaaaa aaaaaaaaaa aaaaaaaaaa 4740

aaaaaaaaaa aaaaaaaaaa aa 4762

<210> 28
<211> 3327
<212> DNA
<213> Homo sapiens

<400> 28

cacacctttc caaggacccc caaactctgc tccgtgcacg tcaaatgctc ctttcccttg 60

tgtccaaccc cctacccctc tccctaacac ccctcttctc aacaagactc agcctctccc 120

cgaggtgggt gagcatcctt gaggtttccc acccttaact gctgtgtccc cggatggagc 180

cagagaaatg tggtgggggg gccggggcag agtttcaaca ttgcccccca gaaggaggag 240
ccagagatgg ggtctgtcca ggaaaacagg atgccggagc ccaggagtcg tcagcctagc 300
agttgcctgg cctccagatg cctcccaggg gagcagatcc tagcatgggc cccaggggtg 360
aggaagggcc tggaaccaga attgtctgga accctgatct gtaccaactt tagggtcacc 420
ttccagccct gtggatggca gtggaatcag gacactccct tgaacagtga atacgatttt 480
gccctggtca acattggacg attagaggct gtgagcggct tgtcccgagt ccagctcctc 540
cgtccagggt ccctgcataa atttatccct gaggagattc tgattcatgg ccgagacttc 600
cggctgctca gagttggttt tgaggctgga ggcctagagc ctcaggcttt tcaggtgacc 660
atggccattg tccaagccag agctcagagc aatcaagccc aacagtattc ggggataacc 720
ctgagcaagg ctggccaggg ttctggctcc agaaaaccac caattcctct catggagaca 780
gcggaagact gggagactga gcggaagaag caggcagcca gaggctggag ggtcagcacg 840
gtcaacgaga ggttcgacgt agccaccagc ctcccccgtt acttctgggt ccctaaccga 900
attctggaca gtgaggtcag gagagcattt ggccactttc atcagggccg tggaccggtc 960
agtgtgatgg ttagggtaat ggctgtggat tagagggtca tgtgggccag ggacatcgtg 1020
gagggaggaa cctctgtgag gtcagtgtgg gggcaagggt agcgtggagc taggcatttc 1080
tcccacaatg accctcttct gccccatgtg aagcgcttgt cctggcatca ccctgggggc 1140
agtgatcttc tccgctgtgg aggcttctat acagccagtg accctaacaa ggaggatatc 1200
agagcagtgg agttgatgca ccaggctggg cattcagatg ttgtcctggt agacactatg 1260
gatgagctgc ccagccttgc agatgtccaa cttgcccacc tgaggctgag ggccctctgc 1320
ctgcctgatt catctgtagc tgaggataaa tgctttcagc cctggaagga acacgatggc 1380
tggactatgt cagggcttgt cttcgaaagg ccagtgacat ttcagtatta gtgacatcca 1440
gggttcgttc tgtaatactt caaggctccg gtgtttctcc tcttccttga ttgtgtctgg 1500
cagctcctcc agcagtttcc agctgatttt gaattctctg agtttttcct tcttgctctt 1560
catgacagtg tcagggttcc tgacaccctt accttcctga gaaatacccc ctgggagcgc 1620
ggaaagcaga gcggacaggt cagtgacttc tatttttgac tcgtgttttt ttttccattg 1680
agatgtactc tctgaagttt ggtcttgatt tgttttatga gaagtgaggt ctgtgagtgg 1740
ggagggggag atttattctc attttcagga cgagactttt gccctacatc tttcctagaa 1800
taagaggtga gaatctcatg atttgtctct agatgtggga ggattgtgtg taaccatcct 1860
ttttcttgct tcctctgtcc agttaaactc ctatacacaa gtctacaccc caggatactc 1920
cagcctccag ctgggaactc ttttaacctg cagctgtctg tctgggactg ggatttacgt 1980
tatagcaatg cacagatact acaattccag aatcctggct atgacccaga acactgtcca 2040
gattcctggc tccctagacc acagccaagc ttcatggttc ctggaccccc cagttttgtg 2100

```
tggctcttct ctagaggagc attgaccccc ctgaatcagc tctgtccttg gcgggacagt      2160
ccttccctgc tggcagtctc ttctcgttgg ctccctcgac ctgctatctc ctctgaaagc      2220
tggctgacca ggaatggggt ctcccctcac attggggagc ttgcccttta cctccagggc      2280
tgctgctgcc tgggtatctg ggaccccaga tcaggctctg gagacgctgc tacctgaggg      2340
gaaggcctga ggtccaggta agaagggaaa atagactggg agtgggacaa gggacttgac      2400
tctgctgaac cagatgaaca ggagctggaa aggcaaggag ctgaagcctc tgggagtctg      2460
ggaagtgaag ttctactcct cttggcatca aacaaggttt gggagtgtag gaggtgcggg      2520
aaagtgcttg tggcttagat taagtggaat ttagggcata gctgaaaggg gaaacagaat      2580
taaagacacc agaagtagca gagaagcagg gggccagagc tacaacagta ttcttctctg      2640
ttcctctttg cctcctcccc agatgggcct ctcatctccc acaatctctg gcctccagga      2700
tgagctatcc catcttcagg agttattacg gaaaggacac caagaatatc tcctgaggat      2760
cactccaaga aaagagatcc acataccatt ctcaatccca ctgaaattgc tggcattctc      2820
aaaggcaggg cagaggggga tctggggtag agggagggtt ctgtctaatc ttttttttt       2880
cttttgtatc tgcacttgca gcctcagctt tcatacttca gcccttaagt tcactaagaa      2940
ggtctgagtt tctgctgcag atagtggtgt taactgctcc aactcttgtc ttgcttagtt      3000
tctacaaata tttttgcttc ttgtcatttg aaggattaag aaacaaaaac aatccagaaa      3060
ttgatcggtt tttttaggcc aatcccatcc cttctggata accagatgtt aaatcatgag      3120
atcagagatg ctgttcatca gtcccaacaa gatggcctag aaatcgcatt ctcacctcgc      3180
cttgctgctg ctttaattcc aagttctatt tcttccctta tagttttcta tgggaatgag      3240
gcggatacag gaaacaccct atctcctctg tatttttgta gtggaatttc tatttaaggg      3300
gctcattaaa gcatagtatt tatacac                                          3327
```

<210> 29
<211> 3061
<212> DNA
<213> Homo sapiens

<400> 29

gagcaattga ttaatagctc ggcgagggga ctcactgact gttataataa cactacacca 60
gcaactcctg gcttcccagc agccggaaca cagacaggag agagtcagtg gcaaatagac 120
atttttctta tttcttaaaa aacagcaact tgtttgctac ttttatttct gttgattttt 180
ttttcttggt gtgtgtggtg gttgttttta agtgtggagg gcaaaaggag ataccatccc 240
aggctcagtc caacccctct ccaaaacggc ttttctgaca ctccaggtag cgagggagtt 300
gggtctccag gttgtgcgag gagcaaatga tgaccgccaa ggccgtagac aaaatcccag 360
taactctcag tggttttgtg caccagctgt ctgacaacat ctacccggtg gaggacctcg 420
ccgccacgtc ggtgaccatc tttcccaatg ccgaactggg aggccccttt gaccagatga 480

acggagtggc cggagatggc atgatcaaca ttgacatgac tggagagaag aggtcgttgg 540
atctcccata tcccagcagc tttgctcccg tctctgcacc tagaaaccag accttcactt 600
acatgggcaa gttctccatt gaccctcagt accctggtgc cagctgctac ccagaaggca 660
taatcaatat tgtgagtgca ggcatcttgc aaggggtcac ttccccagct tcaaccacag 720
cctcatccag cgtcacctct gcctccccca acccactggc cacaggaccc ctgggtgtgt 780
gcaccatgtc ccagacccag cctgacctgg accacctgta ctctccgcca ccgcctcctc 840
ctccttattc tggctgtgca ggagacctct accaggaccc ttctgcgttc ctgtcagcag 900
ccaccacctc cacctcttcc tctctggcct acccaccacc tccttcctat ccatccccca 960
agccagccac ggacccaggt ctcttcccaa tgatcccaga ctatcctgga ttctttccat 1020
ctcagtgcca gagagaccta catggtacag ctggcccaga ccgtaagccc tttccctgcc 1080
cactggacac cctgcgggtg ccccctccac tcactccact ctctacaatc cgtaagccct 1140
ttccctgccc actggacacc ctgcgggtgc cccctccact cactccactc tctacaatcc 1200
gtaactttac cctgggggc cccagtgctg ggtgaccgg accaggggcc agtggaggca 1260
gcgagggacc ccggctgcct ggtagcagct cagcagcagc agcagccgcc gccgccgccg 1320
cctataaccc acaccacctg ccactgcggc ccattctgag gcctcgcaag taccccaaca 1380
gacccagcaa gacgccggtg cacgagaggc cctacccgtg cccagcagaa ggctgcgacc 1440
ggcggttctc ccgctctgac gagctgacac ggcacatccg aatccacact gggcataagc 1500
ccttccagtg tcggatctgc atgcgcaact tcagccgcag tgaccacctc accacccata 1560
tccgcaccca caccggtgag aagcccttcg cctgtgacta ctgtggccga aagtttgccc 1620
ggagtgatga gaggaagcgc cacaccaaga tccacctgag acagaaagag cggaaaagca 1680
gtgccccctc tgcatcggtg ccagccccct ctacagcctc ctgctctggg ggcgtgcagc 1740
ctgggggtac cctgtgcagc agtaacagca gcagtcttgg cggagggccg ctcgcccctt 1800
gctcctctcg gacccggaca ccttgagatg agactcaggc tgatacacca gctcccaaag 1860
gtcccggagg ccctttgtcc actggagctg cacaacaaac actaccaccc tttcctgtcc 1920
ctctctccct ttgttgggca aagggctttg gtggagctag cactgccccc tttccaccta 1980
gaagcaggtt cttcctaaaa cttagcccat tctagtctct cttaggtgag ttgactatca 2040
acccaaggca aaggggaggc tcagaaggag gtggtgtggg gacccctggc caagagggct 2100
gaggtctgac cctgctttaa agggttgttt gactaggttt tgctacccca cttcccctta 2160
ttttgaccca tcacaggttt ttgaccctgg atgtcagagt tgatctaaga cgttttctac 2220
aataggttgg gagatgctga tcccttcaag tggggacagc aaaaagacaa gcaaaactga 2280
tgtgcacttt atggcttggg actgatttgg gggacattgt acagtgagtg aagtatagcc 2340
tttatgccac actctgtggc cctaaaatgg tgaatcagag catatctagt tgtctcaacc 2400

```
cttgaagcaa tatgtattat aaactcagag aacagaagtg caatgtgatg ggaggaacat     2460
agcaatatct gctccttttc gagttgtttg agaaatgtag gctatttttt cagtgtatat     2520
ccactcagat tttgtgtatt tttgatgtac actgttctct aaattctgaa tctttgggaa     2580
aaaatgtaaa gcatttatga tctcagaggt taacttattt aagggggatg tacatatatt     2640
ctctgaaact aggatgcatg caattgtgtt ggaagtgtcc ttggtgcctt gtgtgatgta     2700
gacaatgtta caaggtctgc atgtaaatgg gttgccttat tatggagaaa aaaatcactc     2760
cctgagttta gtatggctgt atatttctgc ctattaatat ttggaatttt ttttagaaag     2820
tatatttttg tatgctttgt tttgtgactt aaaagtgtta cctttgtagt caaatttcag     2880
ataagaatgt acataatgtt accggagctg atttgtttgg tcattagctc ttaatagttg     2940
tgaaaaaata aatctattct aacgcaaaac cactaactga agttcagata atggatggtt     3000
tgtgactata gtgtaaataa atacttttca acaataaaaa aaaaaaaaaa aaaaaaaaaa     3060
a                                                                     3061
```

<210> 30
<211> 1677
<212> DNA
<213> Homo sapiens

<400> 30

```
ggccaagcaa gcttctatct gcacctgctc tcaatcctgc tctcaccatg agcctccgcc       60
tgcagagctc ctctgccagc tatggaggtg gtttcggggg tggctcttgc cagctgggag      120
gaggccgtgg tgtctctacc tgttcaactc ggtttgtgtc tgggggatca gctggggggct     180
atggaggcgg cgtgagctgt ggttttggtg gaggggctgg tagtggcttt ggaggtggct      240
atggaggtgg ccttggaggt ggctatggag gtggccttgg aggtggcttt ggtgggggtt      300
ttgctggtgg ctttgttgac tttggtgctt gtgatggcgg cctcctcact ggcaatgaga      360
agatcaccat gcagaacctc aacgaccgcc tggcttccta cctggagaag gtgcgcgccc      420
tggaggaggc caacgctgac ctggaggtga agatccgtga ctggcacctg aagcagagcc      480
cagctagccc tgagcgggac tacagcccct actacaagac cattgaagag ctccgggaca      540
agatcctgac cgccaccatt gaaaacaacc gggtcatcct ggagattgac aatgccaggc      600
tggctgtgga cgacttcagg ctcaagtatg agaatgagct ggccctgcgc cagagcgtgg      660
aggccgacat caacggcctg cgccgggtgc tggatgagct cactctgtct aagactgacc      720
tggagatgca gatcgagagc ctgaatgaag agctagccta catgaagaag aaccatgaag      780
aggagatgaa ggaatttagc aaccaggtgg tcggccaggt caacgtggag atggatgcca      840
ccccaggcat tgacctgacc cgcgtgctgg cagagatgag ggagcagtac gaggccatgg      900
cagagaggaa ccgccgggat gctgaggaat ggttccacgc caagagtgca gagctgaaca      960
```

aggaggtgtc taccaacact gccatgattc agaccagcaa gacagagatc acggagctca 1020

ggcgcacgct ccaaggcctg gagattgagc tgcagtccca gctgagcatg aaagcggggc 1080

tggagaacac ggtggcagag acggagtgcc gctatgccct gcagctgcag cagatccagg 1140

gactcatcag cagcatcgag gcccagctga gcgagctccg cagtgagatg gagtgccaga 1200

accaagagta caagatgctg ctggacatca agacacgtct ggagcaggag atcgccacct 1260

accgcagcct gctcgagggc caggacgcca agaagcgtca gcccccgtag cacctctgtt 1320

accacgactt ctagtgcctc tgttaccacc acctctaatg cctctggtcg ccgcacttct 1380

gatgtccgta ggccttaaat ctgcctggcg tcccctccct ctgtcttcag cacccagagg 1440

aggagagagc cggcagttcc ctgcaggaga gaggaggggc tgctggaccc aaggctcagt 1500

ccctctgctc tcaggacccc ctgtcctgac tctctcctga tggtgggccc tctgtgctct 1560

tctcttccgg tcggatctct ctcctctctg acctggatac gctttggttt ctcaacttct 1620

ctaccccaaa gaaaagatta ttcaataaag tttcctgcct ttctgcaaac ataaaaa 1677

<210> 31
<211> 8191
<212> DNA
<213> Homo sapiens

<400> 31

```
tttgcttgca acactggcac ctctgccctg caccccggga gtgagcagtg agtgaggctc      60
gggtctgggc gctggctccg aatcttcggg ctgggagaga ctccaccatc tgggggcggc     120
ctgggggagc agccttagtg tcttcctgct gatgcaatcc gctaggtcgc gagtctccgc     180
cgcgagaggg ccggtctgca atccagcccg ccacgtgtac tcgccgccgc ctcgggcact     240
gccccaggtc ttgctgcagc cgggaccgcg ctctgcagcc gcagacccgg tccacacggc     300
caggggctac gacccttggg atctgccctc cgctcagctc gagcttccct cgtggccgac     360
ggaacaatga aggattgcag taacggatgc tccgcagagt gtaccggaga aggaggatca     420
aaagaggtgg tggggacttt taaggctaaa gacctaatag tcacaccagc taccatttta     480
aaggaaaaac cagaccccaa taatctggtt tttggaactg tgttcacgga tcatatgctg     540
acggtggagt ggtcctcaga gtttggatgg gagaaacctc atatcaagcc tcttcagaac     600
ctgtcattgc accctggctc atcagctttg cactatgcag tggaattatt tgaaggattg     660
aaggcatttc gaggagtaga taataaaatt cgactgtttc agccaaacct caacatggat     720
agaatgtatc gctctgctgt gagggcaact ctgccggtat ttgacaaaga agagctctta     780
gagtgtattc aacagcttgt gaaattggat caagaatggg tcccatattc aacatctgct     840
agtctgtata ttcgtcctac attcattgga actgagcctt ctcttggagt caagaagcct     900
accaaagccc tgctctttgt actcttgagc ccagtgggac cttatttttc aagtggaacc     960
tttaatccag tgtccctgtg ggccaatccc aagtatgtaa gagcctggaa aggtggaact    1020
```

```
ggggactgca agatgggagg gaattacggc tcatctcttt ttgcccaatg tgaagcagta    1080
gataatgggt gtcagcaggt cctgtggctc tatggagagg accatcagat cactgaagtg    1140
ggaactatga atctttttct ttactggata aatgaagatg gagaagaaga actggcaact    1200
cctccactag atggcatcat tcttccagga gtgacaaggc ggtgcattct ggacctggca    1260
catcagtggg gtgaatttaa ggtgtcagag agatacctca ccatggatga cttgacaaca    1320
gccctggagg ggaacagagt gagagagatg tttggctctg gtacagcctg tgttgtttgc    1380
ccagtttctg atatactgta caaaggcgag acaatacaca ttccaactat ggagaatggt    1440
cctaagctgg caagccgcat cttgagcaaa ttaactgata tccagtatgg aagagaagag    1500
agcgactgga caattgtgct atcctgaatg gaaaatagag gatacaatgg aaaatagagg    1560
ataccaactg tatgctactg ggacagactg ttgcatttga attgtgatag atttctttgg    1620
ctacctgtgc ataatgtagt ttgtagtatc aatgtgttac aagagtgatt gtttcttcat    1680
gccagagaaa atgaattgca atcatcaaat ggtgtttcat aacttggtag tagtaactta    1740
ccttacctta cctagaaaaa cattaatgta agccatataa catgggattt tcctcaatga    1800
ttttagtgcc tccttttgta cttcactcag atactaaata gtagtttatt ctttaatata    1860
agttacattc tgctcctcaa acaaatgcaa ttttttgtgt gtgtttgaaa gctaatttga    1920
gaaaatttca taggttacat ttcctgcagc ctatctttat ccacagaaag tgttttcttt    1980
tttttaaatc aagactttta aaactggatt tcctcccatc actgtttttt gaaggtcctc    2040
caagtccgtg ttaaggtaaa tatctgtttt cttcctgatg tcacagcctg agcatactct    2100
gtgcattagg aagacctgag tgcatttccc accattgtcc tttccacatt atgttgtagc    2160
tggctggctg tcaggcgact acaagactga gggtcttgtg ccttatagat ctttgtatcc    2220
cccatggctg acatatagta ggtactcagt aaatggtttt ataatgaatc agtgaacatt    2280
ttgcttctat agaagtgtac cttctttgtt tctatattat gaaacctctt tattagaatt    2340
tgtgattgat tctgacagtg tatagattta ccttatattg tctttatttt ccatgagcta    2400
ctaagtcatt agagatactc tgaagcatag ttagtttagg aaatcacttc atattgattg    2460
tattagaatt atcttggaat tgaagatata tccctagagc aggggacccc aacccccagg    2520
ccatgggcca cacagcagga agaggtgagt ggtgggccat tgaggagctt catctgtatt    2580
tatggctact tcccatcact cgaattacca cctgaactcc acctcttgtc agctcagtgg    2640
cagcattaga ttctcatagg agcacaaatc ctattgtgaa ctctgcatgc aagggatcta    2700
ggctatgcgc tccttatgag aatctaatgc ttgatgacct gaggtgtaac agtttcatcc    2760
tgaaaccacc cttcaccctg cagtctgtgg aaaaattgtc ttccacaaaa ctggtccctg    2820
gtgccaaaaa tgttggggac cactgctcta gagagaggtc atgatatcat accaaccaaa    2880
tggaaatgac aaatgtttta tgtcaagtgt taattgcaga aataaatctt tttttttttt    2940
```

ttttggtaga aaacaaagag gcatactctg atttttatac tctgtttttg caggtgctct 3000
tttctttgaa tggagatttg atgagcaagt ggttaggatg cagggagagc tactatgggt 3060
gatattttcc ttgtttagga gctgtgagtt aaaattgtat cctttgtggt ttatctaagg 3120
aaagtcaaat cttgacagaa aacatttttc cttggaaggt caactctcag acattgtatt 3180
ttggtttccc tcagtcctca taacttcctt cttgctgaac atattttatt ctcttttcag 3240
agaaggaaaa taaaaaggat tctaaaagtt tgatgcattg gaaaaatttc cttgaggcat 3300
ttagcaacac atagaaaatg ggctttgatt cttttccaaa acttttagcc atagggtctt 3360
ttatagacag ggatagtaaa atgaaaattg agaaatataa gatgaaaagg aatgataaaa 3420
atatctttta gggggctttt aattggtgat ctgaaatctt gggagaagct gttcttttca 3480
ggcctgaggt gctcttgact gtcgcctgcg cactgtgtac cccgagcaac attctaaggg 3540
tgtgctttcg ccttggctaa ctcctttgac ctcattcttc atatagtagt ctaggaaaaa 3600
gttgcaggta atttaaactg tctagtggta catagtaact aaatttctat tcctatgaga 3660
aatgagaatt atttatttgc catcaacaca ttttatactt tgcatctcca aatttattgt 3720
ggcgagactt gtccattgtg aaagttagag aacattatgt ttgtatcatt tctttcataa 3780
aacctcaaga gcatttttaa gccctttca tcagacccag tgaaaactaa ggatagatgt 3840
ttaaaaactg gaggtctcct gataaggaga acacaatcca ccattgtcat ttaagtaata 3900
agacaggaaa ttgaccttga cgctttcttg ttaaatagat ttaacaggaa catctgcaca 3960
tctttttcc ttgtgcacta tttgtttaat tgcagtggat taatacagca agagtgccac 4020
attataacta ggcaattatc cattcttcaa gacttagtta ttgtcacact aattgatcgt 4080
ttaaggcata agatggtcta gcattaggaa catgtgaagc taatctgctc aaaaagatca 4140
acaaattaat attgttgctg atatttgcat aattggctgc aattatttaa tgtttaattg 4200
ggttgatcaa atgagattca gcaattcaca agtgcattaa tataaacaga actggtggca 4260
cttaaaatga taatgattaa cttatattgc atgttctctt cctttcactt ttttcagttt 4320
ctacatttca gaccgagctt gtcagctttt ttgaaaacac atcagtagaa accaagattt 4380
taaaatgaag tgtcaagaca aaggcaaaac ctgagcagtt cctaaaaaga tttgctgtta 4440
gaaattttct ttgtggcagt catttattaa ggattcaact cgtgatacac caaaagaaga 4500
gttgacttca gagatgtgtt ccatgctctc tagcacagga atgaataaat ttataacacc 4560
tgctttagcc tttgttttca aaagcacaaa ggaaaagtga aagggaaaga gaaacaagtg 4620
actgagaagt cttgttaagg aatcaggttt tttctacctg gtaaacattc tctattcttt 4680
tctcaaaaga ttgctgtaag aaaaaatgta agacaaaaaa aaaaaaaaaa aacaaacaga 4740
ggcagaggca ggcagtagca agaaagcaga gcgtaacatc agctagatgg taacatgcaa 4800
tgtcagctct cttgaagaca tgggaaacct aagttacacc ttgggttaaa attcttcacc 4860

```
atattagttt tgttgcttca taaaatttac ctaagcaagt ggtcttgctt gcctcaaatc 4920
caagcagtct tgaacacttg gaggcaatta atgagtatat cttagtcaaa agaattgttg 4980
gagcttttta ttaaagctac agtttcagtt ctgcttttgg ggaattgtgc tatgaaagca 5040
gctgccaaaa taagctcatt tattttcttc aatcccactc agtgctcagt cactatattc 5100
tgtttccttt tttttttca agttgcatat ttggtttccc cttatgattg ggaaagatga 5160
attttcagca gaaaacattg tttgttcact ttcaaagagt gatagtttct aaaacattta 5220
gagcaataaa tattcatcag aggtaccaag taagccggca gaagagttaa gggttagaga 5280
aatcccttat ttcatgtctt gactctaaaa ttatcaaagt acttttcctt gtaatgtgga 5340
tttcttctta tgcggatatg caaaaacttc agttatacgt agtaatgcta gcaggtaatt 5400
ttagtagaca ttttataaca actgtcactt tgtttcgcca catgtagagt ttgttcagct 5460
attttccaga tatctcccca caaaaggagg caaagggtac cagcttttca atgagcatta 5520
cctattactt ggcaaagatg atgaagactc tattaatagt tcatttgata aatgttgaca 5580
taaccaacaa tagagattag gaagttagtt ttaagaaatc aatggcatat agacattacc 5640
ctcatggagt ttgtattcta ctacttgaac tgattgtagc tataaaagca tagttagata 5700
gctgaatagt tagatcataa gcaaagaagg ccagaacaca tctcttatca agaaatcaat 5760
gaatagttta tctcattttt aaagcaactt tatccttctt taattccttc ctttcttcta 5820
gtgcaaaact acttaataag gttggtgttt aggttagtgt tcacaccatt cctcatctgg 5880
tgtgaattac cttctctttc tttactattt actaccaacc tagtacatgt gttgactgaa 5940
ttcttttcaa acaatgttga gttatcatgg tgcacctaat aaattaacac cacagattac 6000
agcatccttg ctgattttct cagcaaagcc agattagatg gaaataaaca aagaaaatga 6060
tcctagagtg aatttttcta gaaaatatct attatgaacc atgctgttta aagtattagc 6120
ttgaaggtga tggatccagc tattcagaaa ataactttca tataaccatg attttgcaca 6180
gtatgaggtc ttaaatgtgt ggaaagagat aaattttta tcattaccac aaacccttt 6240
taaagattca aaggtggaag aaagtgattt atttttctc ttcagcatac atatataaaa 6300
gacttgtcag atgtttaatt tggggaggtt gataatgaaa catatcaaca gagtatagta 6360
gttatagtag tgtttgtggg taaataattt cctggggtca gacatatata aacatatttg 6420
cttcaaaatg ataaaggcat gaaatcagtc ttaaaaattg aaatgggggt gatgggggag 6480
aaaaagaaga acaaatttga agtgcccttt caaatctgct ggatacaagt attgaagttt 6540
taagtcatct tattctgtct gaaagtgtat ttttcattct acaatagacc caatcaacaa 6600
gacgtataac ttgagttgca tgatgttcag tttatgtaat ctactgttgg gatggtaaga 6660
attgatgtag gctgtggtgt aagaatgaat taaaatatag tttcactggc ttttctctac 6720
atatccacta tcacaatggc taggtttcct gttgctcact attggattct ggagaaaaat 6780
```

ttaatgaaag atgatatcag aggaagaata agtggaggta gagaagaaag gaatgataga 6840
ggaggggaaa aaaacaaaac atatttttgt gttatccaaa ggagcttttt ccttattctg 6900
tcaagcattg agatcttctt cagctttcaa tgtagttgct aaatacaaat aatgctacta 6960
ggtagtgact aaatatagca aacacttcat cagatattag aattaggtca cactattgag 7020
gttataatct gaaggttgtg ttacatagaa accactttag attattatca acttggacta 7080
ggctttattt tataatagca tagtaagtaa tatctattgt gtcatttctt caaccatttt 7140
attctaagat ccatgaagct tcttgaggcc aaataaaata ataagtttag acaagaagta 7200
gattgtgact tttttccctt agagatacta tttactatct cctatcctga taggtggaag 7260
gtttactgaa ttggaaattg gttgactatt agtttttaac taaaatgtgc aataacacat 7320
tgcagtttcc tcaaactagt ttcctatgat cattaaactc attctcaggg ttaagaaagg 7380
aatgtaaatt tctgcctcaa tttgtacttc atcaataagt ttttgaagag tgcagatttt 7440
tagtcaggtc ttaaaaataa actcacaaat ctggatgcat ttctaaattc tgcaaatgtt 7500
tcctggggtg acttaacaag gaataatccc acaatatacc tagctaccta atacatggag 7560
ctggggctca acccactgtt tttaaggatt tgcgcttact tgtggctgag gaaaaataag 7620
tagttcgagg aagtagtttt taaatgtgag cttatagata gaaacagaat atcaacttaa 7680
ttatgaaatt gttagaacct gttctcttgt atctgaatct gattgcaatt actattgtac 7740
tgatagactc cagccattgc aagtctcaga tatcttagct gtgtagtgat tcttgaaatt 7800
ctttttaaga aaaattgagt agaaagaaat aaaccctttg taaatgaggc ttggcttttg 7860
tgaaagatca tccgcaggct atgttaaaag gattttagct cactaaaagt gtaataatgg 7920
aaatgtggaa aatatcgtag gtaaaggaaa ctacctcatg ctctgaaggt tttgtagaag 7980
cacaattaaa catctaaaat ggctttgtta caccagagcc atctggtgtg aagaactcta 8040
tatttgtatg ttgagagggc atggaataat tgtattttgc tggcaataga cacattcttt 8100
attatttgca gattcctcat caaatctgta attatgcaca gtttctgtta tcaataaaac 8160
aaaagaatcc tgtttgtgtg gtttcatgaa a 8191

<210> 32
<211> 2191
<212> DNA
<213> Homo sapiens

<400> 32

cacgggcgga gccggggcca tggagccgcc gctgccgggc taggcaggtc gtgccccgcc 60
gggccggcgg cgatgtcggg ctaccagcgc cacccgggcg ccaccccgct gtcccgagcc 120
cggagcctcg ccattcccga cgctccagcg ttctatgagc gccggtcttg tctcccccag 180
ctaaattgtg agcgccccca tggcagggac ctggactccc ccttcttcgg cattcggccg 240

gcctttatgt gctatgtgcc cagcccggtg ctggcttccg tgggagacac agatgacaga 300
tttgaagatc tggaagaggc aaatccattc tcttttagag agtttctgaa gaccaagaac 360
ctcggcctct cgaaagagga tccggccagc agaatttatg caaaggaagc ctcgaggcat 420
tccctgggac ttgaccacaa ctccccaccc tcccaaaccg gcgggtatgg cctggagtat 480
cagcagccat tttcgagga tccgacaggg gctggtgacc tcctggatgg ggaggaggat 540
gaggacaccg gatggagtgg ggcctacctg ccgtccgcca tcgagcagac tcaccccgag 600
agggtccctg ccggcacgtc gccctgcagc acataccttt ccttttctc caccccgtcg 660
gagctggcag ggcctgagtc tctgccctcg tgggcgttga gtgacactga ttctcgcgtg 720
tctccggcct ctccggcagg gagtcctagc gcagactttg cggttcatgg agagtctctg 780
ggagacaggc acctgcggac gctgcagata agttacgacg cactgaaaga tgaaaattct 840
aagctgagaa gaaagctgaa tgaggttcag agcttctctg aagctcaaac agaaatggtg 900
aggacgcttg agcggaagtt agaagcaaaa atgatcaagg aggaaagcga ctaccacgac 960
ctggagtcgg tggttcagca ggtggagcag aacctggagc tgatgaccaa acgggctgta 1020
aaggcagaaa accacgtcgt gaaactaaaa caggaaatca gtttgctcca ggcgcaggtc 1080
tccaacttcc agcgagagaa tgaagccctg cggtgcggcc agggcgccag cctgaccgtg 1140
gtgaagcaga acgccgacgt ggccctgcag aacctccggg tggtcatgaa cagtgcacag 1200
gcttccatca agcaactggt ttccggagct gagacactga atcttgttgc cgaaatcctt 1260
aaatctatag acagaatttc tgaaattaaa gacgaggagg aagactcttg aggacccctg 1320
ggtgttctca gcatgaagct ccgtgtatac cctgaggtca ccaccgctcg atctaaatgt 1380
gcagttgtgt ccttaaatat gcagtcttca cccagagtaa agtgttgatc gcaagagtcc 1440
agtgtcgtgc cctcagccag ttcttggcca ccacaatggg agcagccctg gccgagttgt 1500
ctctgtggtt tctatgcagc ccttcttggc gaaattcctg cgatcttata gattctaatg 1560
agctcttgga agacattgtc ataaaagcca gtgattttaa gaaaaagagt ggttctggaa 1620
tcagtgtttt ccagtcccat cccagaacat cagttgtaag ataagtacaa ttggttgtcc 1680
ttgatttcat aagtagaaca aacactaaat gtgcctctga gatggccacc ccgggcaggg 1740
acctgtgcct tccaccgatg ctcagggctc cctctggctc ccgggtcact cttgtggccc 1800
cagtgggtgg tccctgcagt catggcctga gtgcgcaggg gccaccgcgt ggctgccgct 1860
gtcctcctcc gggacccacg gggaccaagg tcacacgttc cgtgctgtga agctgtccag 1920
atgtgcctct ttggctgggg gttctggtgg acgtttcaag tggcattttg tacaatgcag 1980
gttagaattc aggaatttca agtatgtgcc cgggtctgtc aggtcccagt tgcctttctg 2040
acggccccc tcagagggac ggcgatgagc actaaatgct tttttgacta ttttcctata 2100
gatttttttt aaaacttttt tttcctcctg ttccaattga tagctttctt atttaataaa 2160

ttctgtagtt caccaaaaaa aaaaaaaaaa a 2191

<210> 33
<211> 463
<212> DNA
<213> Homo sapiens

<400> 33

atatattccc agctagttga aaatgatgat tcccacaaga agcataactc agcttgtttc 60

tgcttactga gtattttcta ctatggtata tattgataac atttcttcca ttatgtatgt 120

tgtataccag agttacagtt actgtgggaa tcataatttg aaattttgac tcctgtgttt 180

ctggaatctt tacaacaaat gttgcattaa catataactt ttttcagttg actttaccaa 240

aattaagccc atctttagta gatactgttt taacatgtga aagaaatacg ttataaacat 300

accacaagat atggctataa aacaatgaga tcagtatcca tttttgcttt aaagaattgg 360

ccttattgct tcagtgtcac atctcatact caagggcatt tactacaaag aaagagttct 420

ccaatattgc tgttctgttg ctgcctgccc tatttacaca tgt 463

<210> 34
<211> 393
<212> DNA
<213> Homo sapiens

<400> 34

tttacactta tagtagactt tatttagtga atccaaatga catgtgataa ttgtttggaa 60

aggcctattg attttatatc tgatcattca atccagagac attaaattca gttgattaat 120

ggagttcccc aactgtaaga cttctttacg agattatttt caagctttga aaagatcttc 180

tgagataaag gggatcagca aacagtaaga gtgtgttgct atacccaagc aaaagaaata 240

aatcttaatc tctcagcaaa tcattcaaaa tgtcagaaat gttagtgttt ctatatcttg 300

gtaaaatgga ttgattgaga agtatgaaaa gtataacagt ggcatgcaga atattgtttt 360

tatgaatatt cagaatttca gttgtttaca taa 393

<210> 35
<211> 10434
<212> DNA
<213> Homo sapiens

<400> 35

```
atggcgaacc ggcgagtggg gcgaggctgc tgggaagtga gcccgaccga gcggaggccg        60
cccgcggggc tgcggggccc cgcggccgag gaggaggcgt cttccccgcc ggtcctgtct       120
ctcagccact tctgcaggtc tcctttcctt tgcttcgggg acgttctcct gggagcctca       180
cggacgctgt ctctggccct agacaaccct aacgaggagg tggcagaagt gaagatctcc       240
cacttcccgg ccgcggacct gggcttcagt gtgtcgcagc gctgtttcgt gttgcagcct       300
aaagagaaaa ttgttatttc tgttaactgg acaccactca aagaaggccg agtaagagag       360
```

attatgacat ttcttgtaaa tgatgttctg aaacaccaag ctatattact aggaaatgca 420
gaagagcaga aaaagaaaaa gaggagtctt tgggatacca ttaaaaagaa gaaaatttca 480
gcctctacaa gtcacaacag aagggtttca aatattcaga atgttaataa aacatttagt 540
gtttcccaaa aagttgacag agttaggagc ccactacaag cttgtgaaaa cttggctatg 600
aatgaaggcg gtcccccaac agaaaacaat tctttaatac ttgaagaaaa taaaataccc 660
atatcaccta ttagccctgc tttcaatgaa tgccatggtg caacttgctt gccactctct 720
gtacgtcgat ctactaccta ctcatctctt catgcatcag aaaataggga actattaaat 780
gtacacagtg ccaacgtttc aaaagtttct tttaatgaga aagctgtaac tgaaacttcc 840
tttaattctg taaatgttaa tggccaaaga ggagagaata gtaaacttag tcttaccccc 900
aactgttctt caactttgaa cattacacaa agccaaatac attttctaag tccagattct 960
tttgtaaata atagtcatgg agctaataat gaactagaat tagtaacatg tctttcatca 1020
gatatgttta tgaaagataa ttcacagcct gtgcatttgg aatcaacaat tgcacatgaa 1080
atttatcaga aaattttaag tccagattct ttcataaaag ataattatgg actaaatcag 1140
gatctagaat cagagtcagt taatcctatt ttatccccta atcaattttt aaaagataac 1200
atggcatata tgtgtacatc tcagcaaaca tgtaaagtac cattatcaaa tgaaaattct 1260
caagtcccac agtctcctga agattggaga aaaagtgaag tttcgccacg tattcctgaa 1320
tgtcagggtt caaaatctcc caaagctatt tttgaagaac tagtagaaat gaagtcaaat 1380
tactacagtt ttataaaaca aaataatcct aaattttctg cagttcagga tatttctagt 1440
catagccaca ataaacaacc taagagacgt ccaatacttt ctgccactgt tactaaaagg 1500
aaggccacct gtaccagaga aaaccaaact gagattaata aaccaaaagc aaaaagatgt 1560
ctcaacagtg cagtgggtga acatgaaaaa gtaataaata atcaaaagga aaaagaagat 1620
tttcattctt atcttccaat tatagatcca atattaagta aatctaagag ttataaaaac 1680
gaggtaacac cctcttcgac aacagcttca gttgctcgga aaagaaagag cgatggaagc 1740
atggaagatg caaatgtgag agttgcaatt acagaacata cagaagtgcg agaaatcaaa 1800
agaatccatt tttctccctc agagcctaaa acatcagctg ttaagaaaac aaaaaatgtg 1860
acaacaccca tctcaaaacg tattagcaac agagagaaat taaacctgaa gaagaaaact 1920
gatttatcaa tattcagaac tccaatttct aaaacaaaca aaaggacaaa acccattatc 1980
gctgtggcac agtccagttt gaccttcata aaaccattaa aaacagatat tcccagacac 2040
ccgatgccat ttgctgcaaa aaacatgttt tatgatgaac gctggaagga aaagcaggaa 2100
cagggcttca cttggtggtt aaattttata ttaacccctg atgacttcac tgtaaaaaca 2160
aatatttctg aagtaaatgc tgctactctt cttttgggaa tagagaatca acataaaata 2220
agtgttccta gagcacctac aaaagaggaa atgtctctca gagcttatac tgctcggtgt 2280

aggttaaaca gactacgtcg tgcagcatgc cgtttgttta cttctgaaaa aatggttaaa 2340
gctattaaaa agcttgaaat tgaaattgaa gctaggcggt taattgttcg aaaagataga 2400
cacctatgga aagatgtggg agaacgtcag aaagtcctga attggctgtt gtcctacaat 2460
cctttgtggc ttcgaattgg tctagagaca acttatggag aactcatatc tttggaagat 2520
aacagtgatg tcacagggtt ggctatgttt attctgaatc gcctactttg gaatcctgat 2580
atagcagctg agtatagaca ccccactgtt cctcacctgt atagagatgg tcatgaagaa 2640
gctttgtcca agtttacatt gaaaaagtta ttgttgttgg tctgttttct tgattatgct 2700
aaaatttcca gactcattga tcatgatcct tgtctcttct gtaaagatgc cgaattcaag 2760
gctagtaaag aaatcctttt ggctttttca cgagatttcc taagtggtga aggtgacctt 2820
tcccgtcacc ttggcttatt gggattacct gttaaccatg ttcagacacc atttgatgaa 2880
tttgattttg ccgttacaaa tcttgccgta gacttgcaat gtggagtgcg ccttgtgcga 2940
accatggaac ttctcacaca gaactgggac ctctcaaaga aactcaggat tccggcaata 3000
agtcgtcttc aaaagatgca caatgttgac attgttcttc aagttcttaa atcacgagga 3060
attgaattaa gtgatgagca tggaaataca attctatcta aggatattgt ggataggcac 3120
agagaaaaaa ctctcaggtt gctttggaaa atagcgtttg cttttcaggt ggatatttcc 3180
cttaacttag atcaattaaa ggaagaaatt gcctttctaa aacacacaaa gagtataaag 3240
aaaacaatat ctctactatc atgccattct gatgatctta ttaataagaa aaaaggcaaa 3300
agggatagtg gttcctttga acaatatagt gaaaacataa agttattgat ggattgggta 3360
aatgctgttt gtgccttcta taataaaaag gtggagaatt ttacagtgtc tttctcagac 3420
ggccgtgtgt tatgttacct gatccaccat taccatcctt gctatgtgcc atttgacgct 3480
atatgtcagc gtactactca aactgtggaa tgtacgcaaa ctggttcagt ggtattaaat 3540
tcatcatctg aatctgatga cagttctctg gatatgtcac ttaaagcatt tgatcatgaa 3600
aatacttcag agctatacaa agagctccta gaaaatgaaa agaaaaattt tcacttggtt 3660
aggtctgcag ttagagacct tggtggaata cctgctatga ttaatcattc agatatgtca 3720
aatacaattc cagatgaaaa ggtggttatt acctatttgt catttctttg tgcaaggctt 3780
ttggatcttc gtaaagaaat aagagctgct cgactcatac aaacaacatg gagaaaatat 3840
aaactaaaaa cagatctcaa acgccatcag gagagagaga aagctgcaag aattattcaa 3900
ttggctgtaa tcaattttct agcaaaacaa agattgagaa aaagagttaa tgcagcactc 3960
gtcattcaga aatattggcg aagagtctta gcacagagaa aattattaat gttaaaaaag 4020
gaaaagctgg aaaaagttca aaataaagca gcatcactta ttcagggata ttggagaaga 4080
tattccacta gacaaagatt tctgaaattg aaatattatt caatcatcct gcaatctagg 4140
ataagaatga taattgctgt tacatcttat aaacgatatc tttgggctac agttacaatt 4200

cagaggcatt ggcgtgctta tttaagaaga aaacaagatc aacaaagata tgaaatgcta 4260

aaatcatcaa ctcttataat ccaatctatg ttcagaaaat ggaagcaacg taaaatgcaa 4320

tcacaagtaa aagctacagt aatattgcaa agagctttta gagaatggca tttaagaaaa 4380

caagctaaag aagaaaattc tgctattatc atacaatcat ggtatagaat gcataaagaa 4440

ttacggaagt atatttatat tagatcttgt gttgttatca ttcagaaaag atttcggtgc 4500

tttcaagccc aaaagttata taaaagaaga aaagagtcca tactaaccat ccagaagtac 4560

tacaaagcat atctgaaagg aaagattgag cgcaccaact atttgcagaa acgagctgca 4620

gccattcaat tacaagctgc ttttaggaga ctgaaagctc ataatttatg tagacaaatt 4680

agagctgctt gtgttattca gtcatactgg agaatgagac aagacagagt tcgattttta 4740

aaccttaaga agactattat caaatttcag gcacatgtaa gaaaacatca acaacgacag 4800

aaatataaga agatgaagaa agcagctgtt ataattcaga ctcatttccg agcttatatt 4860

tttgccatga aagttctagc atcttaccag aaaacacgct ctgctgtcat tgtgctgcag 4920

tctgcatata gagggatgca agccaggaaa atgtatattc acatcctcac atctgttata 4980

aagattcaat catattatcg tgcttatgtt tctaaaaagg aatttttgag cctaaaaaat 5040

gctacaataa aattgcagtc aactgttaag atgaaacaaa cacgtaaaca atatttgcat 5100

ttaagagcag ctgcactatt tatccagcaa tgttaccgtt ccaaaaaaat agctgcacaa 5160

aagagagaag agtatatgca gatgcgggaa tcttgtatca aactgcaagc atttgttaga 5220

ggataccttg tccgaaagca gatgaggtta caaagaaaag ctgttatttc actacagtct 5280

tatttcagaa tgagaaaggc tcggcagtat tatctgaaaa tgtataaagc aattattgtc 5340

attcagaatt actatcatgc atacaaagca caggtcaatc agaggaagaa cttcttgcaa 5400

gtcaaaaaag cagctacttg cttgcaagca gcttacagag gttataaagt acgccagcta 5460

atcaaacaac aatctatagc tgctcttaaa attcagtctg cttttagagg ctataataaa 5520

agggtaaaat atcaatctgt gcttcaatct ataataaaga ttcagagatg gtacagggcg 5580

tacaagactc ttcatgatac aagaacacat tttttgaaga caaaggcagc tgtgatttcc 5640

ctccagtctg cttatcgtgg ctggaaggtt cggaaacaga ttagaaggga acatcaagct 5700

gccttgaaga ttcagtctgc ttttagaatg gccaaggccc agaaacagtt tagattgttt 5760

aaaacagcag cattagtcat ccagcaaaat ttcagagcat ggactgcagg aaggaagcaa 5820

tgtatggagt atattgaact ccgtcatgcg gtactggtgc ttcaatctat gtggaaggga 5880

aaaacactga gaagacagct tcaaaggcaa cataaatgtg ctatcatcat acagtcatac 5940

tatagaatgc atgtgcaaca aaagaagtgg aaaatcatga aaaaagctgc tcttctgatt 6000

caaaagtatt atagggctta cagtattgga agagaacaga atcatttata tttgaaaaca 6060

aaagcagctg tagtaacttt acagtcagct tatcgtggta tgaaagtgag aaaaagaata 6120

aaggattgca acaaagcagc agtcactata cagtctaaat acagagctta caaaaccaaa 6180
aagaaatatg caacctatag agcttcagct attataattc agagatggta tcgaggtatt 6240
aaaattacaa accatcagca taaggagtat cttaatttga agaagacagc aattaaaatc 6300
caatctgttt atagaggtat tagagttaga agacatattc aacacatgca cagggcagcc 6360
acttttatta aagccatgtt taaaatgcat cagtcaagaa taagttacca tacaatgaga 6420
aaagcagcta ttgttattca agtaagatgt agagcatatt atcaaggtaa aatgcagcgt 6480
gaaaagtacc tgacaatttt gaaagctgtt aaagtccttc aggcaagttt tagaggagta 6540
agagttagac ggactcttag aaagatgcag actgcagcaa cactcattca gtcaaactac 6600
agaagataca gacagcaaac atactttaat aagttaaaga aaataacaaa aacagtacag 6660
caaagatact gggcaatgaa agaaagaaac atacaatttc aaaggtataa caaactgagg 6720
cattctgtaa tatacattca ggctattttt aggggaaaga aagctagaag acatttaaaa 6780
atgatgcata tagccgcaac tctcattcag aggagattta gaactctaat gatgagaaga 6840
agattcctct ctctcaagaa aactgctatt ttgattcaga gaaaatatcg ggcacatctt 6900
tgtacaaagc atcacttaca gttccttcag gtacaaaatg cagttattaa aatccagtca 6960
tcatacagaa gatggatgat aaggaaaagg atgcgagaga tgcacagggc tgctactttc 7020
atccagtcta ctttcagaat gcacagatta catatgagat atcgagcttt gaaacaggcc 7080
tccgttgtga tccaacagca ataccaagca aatagagctg caaaactgca gaggcagcat 7140
tatctcagac aaagacactc tgctgtgatc cttcaggctg cattcagggg tatgaaaact 7200
agaagacatt tgaagagtat gcattcctct gcaaccctta ttcagagtag gtttagatca 7260
ttactggtga ggagaagatt catttccctc aaaaaagcta ctatttttgt tcagaggaaa 7320
tatcgagcca ccatttgtgc caaacataaa ttgtaccaat tcttgcactt aagaaaggca 7380
gccattacaa tacagtcatc ttacagaaga ctgatggtaa agaagaagtt acaagaaatg 7440
caaagggctg cagttctcat tcaggctact ttcaggatgc acagaacata tattacattt 7500
cagacttgga aacatgcttc aattctaatt cagcaacatt atcgaacata tagagctgca 7560
aaattgcaaa gagaaaatta tatcagacaa tggcattctg ctgtggttat tcaggctgca 7620
tataaaggaa tgaaagcaag acaactttta agggaaaaac acaaagcttc tatcgtaata 7680
caaagcacct acagaatgta taggcagtat tgtttctacc aaaagcttca gtgggctaca 7740
aaaatcatac aagaaaaata tagagcaaat aaaaagaaac agaaagtatt tcaacacaat 7800
gaacttaaga aagagacttg tgttcaggca ggttttcagg acatgaacat aaaaaaacag 7860
attcaggaac agcaccaggc tgccattatt attcagaagc attgtaaagc ctttaaaata 7920
aggaagcatt atctccacct tagagcaaca gtagtttcta ttcaaagaag atacagaaaa 7980
ctaactgcag tgcgtaccca agcagttatt tgtatacagt cttattacag aggctttaaa 8040

gtacgaaagg atattcaaaa tatgcaccgg gctgccacac taattcagtc attctatcga 8100
atgcacaggg ccaaagttga ttatgaaaca aagaaaactg caattgtggt tatacagaat 8160
tattataggt tgtatgttag agtaaaaaca gaaagaaaaa actttttagc agttcagaaa 8220
tctgtacgaa ctattcaggc tgcttttaga ggcatgaaag ttagacaaaa attgaaaaat 8280
gtatcagagg aaaagatggc agccattgtt aaccaatctg cactctgctg ttacagaagt 8340
aaaactcagt atgaagctgt tcaaagtgaa ggtgttatga ttcaagagtg gtataaagct 8400
tctggccttg cttgttcaca ggaagcagag tatcattctc aaagtagggc tgcagtaaca 8460
attcaaaaag ctttttgtag aatggtcaca agaaaactgg aaacacagaa atgtgctgcc 8520
ctacggattc agttcttcct tcagatggct gtgtatcgga gaagatttgt tcagcagaaa 8580
agagctgcta tcactttaca gcattatttt aggacgtggc aaaccagaaa acagttttta 8640
ctatatagaa aagcagcagt ggttttacaa aatcactaca gagcatttct gtctgcaaaa 8700
catcaaagac aagtctattt acagatcaga agcagtgtta tcattattca agctagaagt 8760
aaaggattta tacagaaacg gaagtttcag gaaattaaaa atagcaccat aaaaattcag 8820
gctatgtgga ggagatatag agccaagaaa tatttatgta aagtgaaagc tgcctgcaag 8880
attcaagcct ggtatagatg ttggagagca cacaaagaat atctagctat attaaaagct 8940
gttaaaatta ttcaaggttg cttctatacc aaactagaga gaacacggtt tttgaatgtg 9000
agagcatcag caattatcat tcagagaaaa tggagagcta tacttcctgc aaagatagct 9060
catgaacact tcttaatgat aaaaagacat cgagctgctt gtttgatcca agcacattat 9120
agaggatata aaggaaggca ggtctttctt cggcagaaat ctgctgcttt gatcatacaa 9180
aaatatatac gagccaggga ggctggaaag catgaaagga taaaatatat tgaatttaaa 9240
aaatctacag ttatcctaca agcactggtg cgtggttggc tagtacgaaa aagattttta 9300
gaacagagag ccaaaattcg acttcttcac ttcactgcag ctgcatatta tcacctgaat 9360
gctgttagaa ttcaaagagc ctataaactt tacctggctg tgaagaatgc taacaagcag 9420
gttaattcag tcatctgtat tcagagatgg tttcgagcaa gattacaaga aaagagattt 9480
attcagaaat atcatagcat caaaaagatt gagcatgaag gtcaagaatg tctgagccag 9540
cgaaataggg ctgcatcagt aatacagaaa gcagtgcgcc attttctcct ccgtaaaaag 9600
caggaaaaat tcactagtgg aatcattaaa attcaggcat tatggagagg ctattcttgg 9660
aggaagaaaa atgattgtac aaaaattaaa gctatacgac taagtcttca agttgttaat 9720
agggagattc gagaagaaaa caaactctac aaaagaactg cacttgcact tcattacctt 9780
ttgacatata agcacctttc tgccattctt gaggccttaa aacacctaga ggtagttact 9840
agattgtctc cactttgttg tgagaacatg gcccagagtg gagcaatttc taaaatattt 9900
gttttgatcc gaagttgtaa tcgcagtatt ccttgtatgg aagtcatcag atatgctgtg 9960

caagtcttgc ttaatgtatc taagtatgag aaaactactt cagcagttta tgatgtagaa 10020
aattgtatag atatactatt ggagcttttg cagatatacc gagaaaagcc tggtaataaa 10080
gttgcagaca aaggcggaag catttttaca aaaacttgtt gtttgttggc tattttactg 10140
aagacaacaa atagagcctc tgatgtacga agtaggtcca aagttgttga ccgtatttac 10200
agtctctaca aacttacagc tcataaacat aaaatgaata ctgaaagaat actttacaag 10260
caaaagaaga attcttctat aagcattcct tttatcccag aaacacctgt aaggaccaga 10320
atagtttcaa gacttaagcc agattgggtt ttgagaagag ataacatgga agaaatcaca 10380
aatcccctgc aagctattca aatggtgatg gatacgcttg gcattcctta ttag 10434

<210> 36
<211> 3581
<212> DNA
<213> Homo sapiens

<400> 36

atacgtggct gccgtctgtc cccgctgagg aggtgcagca gccggagatg gcggcggtgc 60
tgaacgcaga gcgactcgag gtgtccgtcg acggcctcac gctcagcccg gacccggagg 120
agcggcctgg ggcggagggc gccccgctgc tgccgccacc gctgccaccg ccctcgccac 180
ctggatccgg tcgcggcccg ggcgcctcag gggagcagcc cgagcccggg gaggcggcgg 240
ctgggggcgc ggcggaggag gcgcggcggc tggagcagcg ctggggtttc ggcctggagg 300
agttgtacgg cctggcactg cgcttcttca aagaaaaaga tggcaaagca tttcatccaa 360
cttatgaaga aaaattgaag cttgtggcac tgcataagca agttcttatg ggcccatata 420
atccagacac ttgtcctgag gttggattct ttgatgtgtt ggggaatgac aggaggagag 480
aatgggcagc cctgggaaac atgtctaaag aggatgccat ggtggagttt gtcaagctct 540
taaataggtg ttgccatctc ttttcaacat atgttgcgtc ccacaaaata gagaaggaag 600
agcaagaaaa aaaaaggaag gaggaagagg agcgaaggcg gcgtgaagag gaagaaagag 660
aacgtctgca aaaggaggaa gagaaacgta ggagagaaga agaggaaagg cttcgacggg 720
aggaagagga aaggagacgg atagaagaag aaaggcttcg gttggagcag caaaagcagc 780
agataatggc agctttaaac tcccagactg ccgtgcagtt ccagcagtat gcagcccaac 840
agtatccagg gaactacgaa cagcagcaaa ttctcatccg ccagttgcag gagcaacact 900
atcagcagta catgcagcag ttgtatcaag tccagcttgc acagcaacag gcagcattac 960
agaaacaaca ggaagtagta gtggctgggt cttccttgcc tacatcatca aaagtgaatg 1020
caactgtacc aagtaatatg atgtcagtta atggacaggc caaaacacac actgacagct 1080
ccgaaaaaga actggaacca gaagctgcag aagaagccct ggagaatgga ccaaaagaat 1140
ctcttccagt aatagcagct ccatccatgt ggacacgacc tcagatcaaa gacttcaaag 1200

agaagattca gcaggatgca gattccgtga ttacagtggg ccgaggagaa gtggtcactg 1260
ttcgagtacc cacccatgaa gaaggatcat atctcttttg ggaatttgcc acagacaatt 1320
atgacattgg gtttggggtg tattttgaat ggacagactc tccaaacact gctgtcagcg 1380
tgcatgtcag tgagtccagc gatgacgacg aggaggaaga agaaaacatc ggttgtgaag 1440
agaaagccaa aaagaatgcc aacaagcctt tgctggatga gattgtgcct gtgtaccgac 1500
gggactgtca tgaggaggtg tatgctggca gccatcaata tccagggaga ggagtctatc 1560
tcctcaagtt tgacaactcc tactctttgt ggcggtcaaa atcagtctac tacagagtct 1620
attatactag ataaaaatgt tgttacaaag tctggagtct agggttgggc agaagatgac 1680
atttaatttg gaaatttctt tttacttttg tggagcatta gagtcacagt ttaccttatt 1740
gatattggtc tgatggtttg tgaactcttg ctgggaatca aaatttcctt gagactcttt 1800
agcattcata ctttggggtt aaaggagatt cctcagactc atccagccct tgggtgctga 1860
ccagcagagt cactagtgga tgctgaagtt acatgagcta catgttaaat atttaaagtc 1920
tccaaaataa aacaccccaa cgttgacctt acccggctga tggttagccc cttgctgcct 1980
gctccatgtg tcttatgaga gcccgtagtt acagtgtcct ctaatttgaa atccataagt 2040
taacaagtct atatcaggtg cagctggctt tgattaaagg ccatttttaa aacttaaaaa 2100
ctcaacacct cacagattat aatagaaaaa gaaatggcct cagtttgatc tcgttcagaa 2160
tgacccagat tgtttctgct ttgggtgcag ctgtttagtt cagagttata ttacagagaa 2220
ttattttctg agataatctt aaactagaat gttcaaaact aattgataat tgaagtatca 2280
agatacgtag aacacctcag agatttttct tcaggaactt ccacaaactt tgaatccttg 2340
tatctttatt tggtattcat actactagta gcaaaataca ggtttttttgt tttgttttgt 2400
tttgttttgg cttcatagag tatctcaaat tgaaactttt ctgcacaaag aataaaatta 2460
aggattttat aaactcaaat tggcacctac tgaattaaaa tacataaaat catttaaata 2520
taattcagca tatgggaagt aacattgcac taatatggaa atcactgcca gagacagtct 2580
attttctttt aatttgttac tacttagtca caaaccccac attattccag tttggaatta 2640
cttattaagg agaattggaa atacatatgc ccatgcttaa attttatagc tttaatttgt 2700
gttatttctt tattgacggg aagaggtaca tcttttttttc cttactgaaa acaaatatgg 2760
attaattgcc tcaaatttgt ataagtgatt ggctagtgat tcttgttttc agaagggaga 2820
gtggtataga tagaaaatga caaagatggc aatatacact taatgttgtt attgtatgtt 2880
gttactgaag tacttagatt tttaaaattt caaatcctaa atcacttctt gtaggagggt 2940
tttcattaac tgcagtatat acagttcact acatatgggt tgtttgagtt ttttgtgtgc 3000
tgtatttctt tctgtttttt aatacctggt tttgtacata tctaactctg ttctcttttg 3060
gttgttcaga aactggattt tttttttctt aagcagtgct taatttgtgt tttttaattt 3120

tgattcagaa gtagtcccag ctcataggtg ttcatactgt tacatccaga acatttgtca 3180

ggctctctgt cagctttcat gtacatatgg tatagaaacc atggagttag gcacttcctg 3240

gatttttttt ttatgagaaa aatactgtat ttaaaatgta aaataaactt ttaaaaagca 3300

ggcactaata tatatttctt ccagcctttg attacaaatt tgtccttgca catgttaaga 3360

tgaattatct cctaaaaata tcattgttct tgggagcagt gtatgttact ttacatagca 3420

gcggttcctg tcatgtgttc atgtcagaat atttttggtt ttaaactttc ttattgcctt 3480

tggctgttga ttagtacagt acaagtgcga tttcaaaaag atcttgaaag taatatattt 3540

aatcaattaa aatgtttatc tgtaaaaaaa aaaaaaaaaa a 3581

<210> 37
<211> 609
<212> DNA
<213> Homo sapiens

<220>
<221> misc_feature
<222> (565)..(565)
<223> n is a, c, g, or t

<220>
<221> misc feature
<222> (587)..(587)
<223> n is a, c, g, or t

<220>
<221> misc_feature
<222> (598)..(598)
<223> n is a, c, g, or t

<400> 37

ttttttttaca gttttcaaat attttactga aaatgcatat tgtacaatta atgtataatg 60

acacaccagt gtgagaaacc tccataggta tcatttccac aaatatgcta tgaatataga 120

gttcctacac aaaactatac aacttaccag atgtaattcc tgttacgtac catactcaca 180

atcgtcttga agaatatgga gaaaaagtgc tgagtgacaa aaacaggagc catgtgtgat 240

tttaataaat ggaaaacacg gcatttcagc tcagtggtaa agcagtaaac caatcagatg 300

cttagctatc aagtaatcat gtgagaggaa acagaattag atcctacctc atactatatg 360

ttgtcagcta acactgtagc agtggtatat gaatcactaa attacctcca acaaaatgta 420

ttcctgtatt gaaaaaagga ggtatggcca acattgtgtc acgttccaag gtgaattttg 480

cggtcacgat atgacgttca ggaagctact tttattgttc agttgatttc tatgctcaac 540

tattaggtca attccgaaat aatcncatat cacagctaaa ataatgncta ccaagtcnct 600

ctgactgct 609

<210> 38

<211> 2216
<212> DNA
<213> Homo sapiens

<400> 38

```
ctgttagcaa tgcttcctga tgttgtgcgt ggcccttttt ggttgattct ctccaaattc      60
gggtcagctg ctgccacctg gcaaataaca gaggatatgc tgaatctcct gtccatcctt     120
gtaacgatat ccttcttaat gaaattcttc aactggctga gcaattacaa atgtcatctg     180
tccagacaca tgggcttaag gatgtctaca aaattttaga catttttgca aatgggaaaa     240
aaaatagtct tgtaaatact gaaacagatt tccatgaact ttatcctact cttggaaaga     300
aaacaattct ccttggctgc agaaatcaaa taagctgggt ttgcaatgac caaggacata     360
aatgaagatg gattgaagtg gaaaaattct gtctcccaag tgatcagtga catctgccag     420
aggtcattac agctactttt aactgtgaac agtcaccagc taaactactc acttgccaca     480
acaaaataac ctctctcaaa gtaaatccag tgcatctgta tatatgtgta gatagcagca     540
acaaacaatc ctgaaacatt atttttggct gttaggtaag taaacgtgat gataattata     600
aacaacattc aaataacctt ggaccttggt gaaatgactt gtggtggcca gaatggtgca     660
acaagatgtt atttgcaagt ttttttaaga cacaaatatc tcagatacta ataatgagaa     720
taaagactgt tgaatatgaa attaaagcca agcaataatg tgccaaaaag aggcagttat     780
accagcaaat gcatctatta tgggcacacc attatataat gatggtttgc tttatgaaga     840
ctgactgtaa cccacaggat aaaataagca aaggcatagt ttctgctttc ttcctggaaa     900
aacttgttta gaagcttcat aaagaggtac agcactaatg agcattagtc aggatacagt     960
tggcatctat gtttttatgt gagcccagag ggaagaggag ccactcaaag tcttgctggt    1020
ttaaaactca agacagctgc aaccagaagt tttgttgaaa tggagacttt aaacttatgg    1080
taattactct ttctggacac tagcatgtag aaagcaattc agttaactct gcccagagga    1140
ttaccagctt tagctgtgaa aaaatgggct cccggatgta aaatcactaa aacatgagat    1200
cttgtatcca aagaggcttc aaatgatgcc ttacagaaaa cgatgctcca gatgggcact    1260
tctaaatgct aactcttcat caagtatctt tctggattca agctcaaaat taattggctg    1320
caaaatagta ggaataaaaa tcacatattt tacactttag aaaaggatat tgatgatcaa    1380
cctgcatggt gataattatg atgagatacc ccagtgattt aatgatgtta gaaagaatta    1440
aatgggagag aattgctaac agctttcttg atctcttaac tatggagatg tcattcattt    1500
atttctgggg tgaaaattat agcttgcttt ttgacattgc tgctagtatt gttctttgtt    1560
gctttaaaaa ttgtctctct ttagaaaaac tcttgagcag ttaaacagtt ctttttctga    1620
ttcatatcat tgcttttaat aacatgtaaa ggctgtgtgt agagcaaact atataaaatg    1680
agtagaaagg gcttgctcat gttaattggc atccttgatg attttagttg agattcctta    1740
acatttattt tagatcacat ctttacgtaa cttatttttc ctaatgtttt ccatcgtgtc    1800
```

```
ttaaaatgat gctggtatat caggagattg cagtattata gtcatactcc ccaatcccta      1860
gaggagagga aagactaatt cttgttttaa gggcccctgg agataccttt tattaaggtt      1920
gaaaaaggtc aacacagcct gaaaataaga aaaatatata ctagcaatta ctaattttct      1980
aaatgtgtgt atctctgctg tactaatgtg tgaacaatat gtcgtgcata atactgtagc      2040
tggtcgtggt atgtcaatac attctgtgag tgtgtacagt ctgagtgatc agttttctat      2100
ttttatgtgt aaaaaaaata acttgtcgta tcccatttaa aggccaattt ctgtattcag      2160
gcaggcatat gtacatacat gaataaagcc aacaaaagtg tgcacatgta ttcagt          2216
```

<210> 39
<211> 1705
<212> DNA
<213> Homo sapiens

<400> 39

aattcggcac gagaagactt ccagtttgga gtcgtttgct gcggggaggg aatgaatggg 60
cgctgggaac acgcccgcga ggtggggacg cgccggccgt agcgaggtcc ttagcgtgtg 120
agtggccggg gtcgggtcgc ttccccgcag catggaggac gatgcaccag tgatctacgg 180
gctggagttc caggcacgtg ccttaacacc tcaaactgca gaaacagatg ccattcggtt 240
tttggttggg acgcagtctc ttaaatatga taatcagatc catatcatag attttgacga 300
tgaaaacaac attataaata aaaatgtcct cctccatcaa gcgggtgaaa tctggcatat 360
tagcgctagc cctgcagaca gaggtgtgct gacgacctgc tacaacagaa cttcagacag 420
caaagtcctg acatgtgcag ccgtgtggag gatgccgaag gaattggaat caggcagcca 480
cgagtcccct gatgattcat ccagcactgc acagaccctg gagctgctct gtcaccttga 540
caacacagcc catggcaaca tggcctgtgt cgtgtgggag ccaatgggag atgggaagaa 600
aatcatttcc ttggctgata accatatcct gctgtgggat ttacaggaaa gctcgagcca 660
ggctgtgctg gccagctcag cgtccctgga agggaaggga caactgaagt tcacctcagg 720
acggtggagc ccacatcata actgcaccca ggtggccaca gcgaacgaca ccaccctccg 780
tggctgggac acccggagca tgagccagat ctactgcata gagaatgccc acggacagct 840
ggtgcgggac cttgacttta atcccaataa gcagtactac ttggccagct gcggagacga 900
ctgtaaggtg aagttctggg acacccgaaa tgtcaccgaa cccgtgaaga ccctggagga 960
gcactcccac tgggtgtgga acgtccgcta caaccactct catgaccagc tggtcctcac 1020
gggcagcagt gacagcagag tcatcctttc caacatggtg tccatctcgt cggagccctt 1080
cggccacttg gtagacgacg atgacatcag tgaccaggag gaccaccgtt ctgaagagaa 1140
gagcaaggag cccctgcagg acaacgtgat cgccacctac gaggagcacg aggacagcgt 1200
ctatgccgtg gactggtcct cggctgaccc gtggctgttt gcctccctga gctatgacgg 1260
gaggctcgtg atcaacaggg tgcccagggc cctgaagtac cacatcctgc tatgactccc 1320

gggcctgggt tatccaggtc ccattgagtg gttttcctct tggcagattc tcaaacagtc 1380
gcagctcttt ggaggtgact cgtgttccag gtggatccct ctctgggaga gccgctgttc 1440
ccttcctgta gcagcagcat ttatgaatgg ggtgaatggg gctattgtcg acggcacagc 1500
taatgcccga acccagcccc tgtcggcaga gacagagccc cacattatta tgtgaataac 1560
aatgttttct gttttaaggg tgtcaggagt ttcgcttttt aaaaaaatgt ctgttcctgc 1620
agtagtaact cttctttctc ttgagagtaa aaaatgaaat aaaataaatc cacgctgaca 1680
aaaaaaaaaa aaaaaaaaaa aaaaa 1705

<210> 40
<211> 1800
<212> DNA
<213> Homo sapiens

<400> 40

```
gaggaagatg gcggcgtccg cagctgccgc tgagctccag gcttctgggg gtccgcggca      60
cccagtgtgt ctgttggtgt tgggaatggc gggatccggg aaaaccactt ttgtacagag     120
gctcacagga cacctgcatg cccaaggcac tccaccgtat gtgatcaacc tggatccagc     180
agtacatgaa gttccctttc ctgccaatat tgatattcgt gatactgtaa agtataaaga     240
agtaatgaaa caatatggac ttggacccaa tggcggcata gtgacctcac tcaatctctt     300
tgctaccaga tttgatcagg tgatgaaatt tattgagaag gcccagaaca tgtccaaata     360
tgtgttgatt gacacacctg gacagattga ggtattcacc tggtcagctt ctgggacaat     420
tatcactgaa gcccttgcat cctcatttcc aacagttgtc atctatgtaa tggacacatc     480
gagaagtacc aacccagtga ccttcatgtc caacatgctc tatgcctgca gcatcttata     540
caaaaccaag ctgcctttca ttgtggtcat gaataaaact gacatcattg accacagctt     600
tgcagtggaa tggatgcagg attttgaggc tttccaagat gccttgaatc aagagactac     660
atacgtcagt aacctgactc gttcaatgag cctggtgtta gatgagtttt acagctcact     720
cagggtggtg ggtgtctctg ctgttctggg tactggatta gatgaactct ttgtgcaagt     780
taccagtgct gccgaagaat atgaaaggga gtatcgtcct gaatatgaac gtctgaaaaa     840
atcactggcc aacgcagaga gccaacagca gagagaacaa ctggaacgcc ttcgaaaaga     900
tatgggttct gtagccttgg atgcagggac tgccaaagac agcttatctc ctgtgctgca     960
cccttctgat ttgatcctga ctcgaggaac cttggatgaa gaggatgagg aagcagacag    1020
cgatactgat gacattgacc acagagttac agaggaaagc catgaagagc cagcattcca    1080
gaattttatg caagaatcga tggcacaata ctggaagaga aacaataaat aggagacttt    1140
agcacacttc acttgtttct agaagtccag aattttggac ctccacgtga aagaactgtt    1200
cttacctctg aactgggggc tcccataagg gataattttc ctcagagtag caaagtttct    1260

cttattagag aaatcttgtg actcagatga agtcagggat agaagaccct tggacctggc    1320
aggttaatgc tgattattcc ttggcctttc ccttgtattt atgcaaggaa ggatatactg    1380
agctgatact gttccaagcc tacaacttca agttttatca tttgaactca agtacttttg    1440
ctgctgagga atggaatcaa aagaacgtag tctcctggtg accacctcag atctctatta    1500
ttaggctaga tgtatagcct ctactccccc agcttcttgc tcttgaccct gcactgtaag    1560
ttgcccttct attagcagcc aaggaaaagg gaaacatgag cttatccaga acggtggcag    1620
agtctccttg gcaatcaacc aacgttgcta tgaaatatgc ctcacactgt atagctcatt    1680
ataggacgtc aggtttgttg aaaaaagtgg gcaagacatg attaatgaat cagaatcctg    1740
tttcattggt gacttggata aagacttttt aattttaaaa aaaaaaaaaa aaaaaaaaaa    1800
```

<210> 41

<211> 2297
<212> DNA
<213> Homo sapiens

<400> 41

```
ggctgaggcg cgatggcagg tgtcggggct gggcctctgc gggcgatggg gcggcaggcc      60
ctgctgcttc tcgcgctgtg cgccacaggc gcccaggggc tctacttcca catcggcgag     120
accgagaagc gctgtttcat cgaggaaatc cccgacgaga ccatggtcat cggcaactat     180
cgtacccaga tgtgggataa gcagaaggag gtcttcctgc cctcgacccc tggcctgggc     240
atgcacgtgg aagtgaagga ccccgacggc aaggtggtgc tgtcccggca gtacggctcg     300
gagggccgct tcacgttcac ctcccacacg cccggtgacc atcaaatctg tctgcactcc     360
aattctacca ggatggctct cttcgctggt ggcaaactgc gtgtgcatct cgacatccag     420
gttggggagc atgccaacaa ctaccctgag attgctgcaa aagataagct gacggagcta     480
cagctccgcg cccgccagtt gcttgatcag gtggaacaga ttcagaagga gcaggattac     540
caaaggtatc gtgaagagcg cttccgactg acgagcgaga gcaccaacca gagggtccta     600
tggtggtcca ttgctcagac tgtcatcctc atcctcactg gcatctggca gatgcgtcac     660
ctcaagagct tctttgaggc caagaagctg gtgtagtgcc ctctttgtat gacccttcct     720
ttttacctca tttatttggt actttcccca cacagtcctt tatccacctg gatttttagg     780
gaaaaaaatg aaaaagaata agtcacattg gttccatggc cacaaaccat tcagatcagc     840
cacttgctga ccctggttct taaggacaca tgacattagt ccaatctttc aaaatcttgt     900
cttagggctt gtgaggaatc agaactaacc caggactcag tcctgcttct tttgcctcga     960
gtgattttcc tctgtttttc actaaataag caaatgaaaa ctctctccat taccttctgc    1020
tttctctttg tccacttacg cagtaggtga ctggcatgtg ccacagagca ggccctgcct    1080
cactgtctgc tggtcagttc tgggttcact taatggcttt gtgaatgtaa ataaggggca    1140
ggtcttggcc ctagaggatt gagatgtttt tctatatctt agaactattt ttggataaat    1200
```

tatatatttt ccttcctagt agaagtgtta ctgcctgtaa ctagctcaaa ataccaatgc 1260

agtttctgca ttctgggttt tgtttttcct tttttttttt tttttttttt ttttgagttt 1320

tgctctcgtc gcccaggctg gagtgcaatg gcgtgatctc agctcactgg caacatctgc 1380

ctcccgggtt caaatgattc tcctgcctca gtctcctgag tagctgggat tacaggtgcc 1440

cgccaccacg ctcagctaat ttttgtattt ttagtagaga tggggtttta ccatgttggc 1500

caggctggtc ttagactcct gacctcagtt gatccacctg cctcagcctc tgcattcagt 1560

ttattcacat atttttggta actcccatgg cagctcctag gatttcagcg gtctgtgggc 1620

cagaaagcag gcaccagggc tgacctcaag gccgtatcag agggccaagc agagttcttt 1680

tggatacctg cttttcatcc cacagggcct tagagtcaga ggtaaggtag caacagagct 1740

agaatggggc aatgcactct taccctcctt ctcaactttt atttaagctg tgctaaatgt 1800

tttcttcaag ggaaccagat ttagttcttt acagaatttt ccagtgaaat aaaacatgtt 1860

gtaatagctg tgtttgagat gaaataagag gttgtgggta gaggggaggc acctaaagga 1920

aaagaggaaa ggtgcctggg ctacctatgc agataacctg gagtggactt cactgtggac 1980

tcgtggtact aaggcttggc ctggacaggc agtctagggg gtatgggaat acacggtgtg 2040

gttgttcaac tatttgcaaa ggtcaaccaa atagaccaca tgttcgcaaa gtatcatctg 2100

aggaaattaa gtaccttctt agccctctca gtcataaatt tgaacaaatt ttaatacact 2160

tccctcatgc ccttctatat aaaacttaat accattagtt ccccattctt gacattttat 2220

ttcagttttt attatatatt tatttgaaat atttattaaa ttatctgacc tacagaacta 2280

aaaaaaaaaa aaaaaaa 2297

<210> 42
<211> 653
<212> DNA
<213> Homo sapiens

<220>
<221> misc_feature
<222> (621)..(621)
<223> n is a, c, g, or t

<220>
<221> misc_feature
<222> (653)..(653)
<223> n is a, c, g, or t

<400> 42

gggacatttc caagggtatt taaactctca ctctgccacc tttctaaggg tgggaggctg 60

gcagagatgc tgcaatgctt gataatcatt tggccacact gaaatttcca aagggagctc 120

ttgccggtgc ttaaaaccaa aactcctgga cacttagaaa attccatgaa tctagcacaa 180

aatatccatt cttgcccaag tgtatcccct ttctctccag cttaatcttt tttttttttt 240

```
tttttaaag cccaggccaa gggtactttt aactggaaac tggggaggag ggaagaacac     300
tagcagggag ctaagaggca ggttgctggg taagccatcc tgctcctacc tggtgcctgt    360
atctacattg ctgagtgctg tgcgccagtg cctttccttc atctgcagat ggagcccatc    420
tctttccacc tgggtgagga gaccctctgc tactccaggg gtaaacctta aagaaggtgt    480
cttgaagagc ccaaaggaca ctcacgtgct aaggtgtcca ttttatgcat ctttaaaata    540
ttttatttaa aaaaaaaaat agccctgccc tgtcttagtg ccactaacgg cccagattca    600
ttcattctga atggaaaaac ngagactgcc agcactttcc tttggtcctt ccn           653
```

<210> 43
<211> 1955
<212> DNA
<213> Homo sapiens

<400> 43

```
catggaggcg ctgctgctgg gcgcggggtt gctgctgggc gcttacgtgc ttgtctacta     60
caacctggtg aaggccccgc cgtgcggcgg catgggcaac ctgcggggcc gcacggccgt    120
ggtcacgggt gagtgcggag gcgggtgagt gcgagctggc ggggcgcgcg gagaggaggc    180
cgggccggcg gtagcagcgg cccgccgggc tcagctcagc tcggctcccg cccgcggtcc    240
gcaggcgcca acagcggcat cggaaagatg acggcgctgg agctggcgcg ccggggagcg    300
cgcgtggtgc tggcctgccg cagccaggag cgcggggagg cggctgcctt cgacctccgc    360
caggagagtg ggaacaatga ggtcatcttc atggccttgg acttggccag tctggcctcg    420
gtgcgggcct ttgccactgc ctttctgagc tctgagccac ggttggacat cctcatccac    480
aatgccggta tcagttcctg tggccggacc cgtgaggcgt ttaacctgct gcttcgggtg    540
aaccatatcg gtccctttct gctgacacat ctgctgctgc cttgcctgaa ggcatgtgcc    600
cctagccgcg tggtggtggt agcctcagct gcccactgtc ggggacgtct tgacttcaaa    660
cgcctggacc gcccagtggt gggctggcgg caggagctgc gggcatatgc tgacactaag    720
ctggctaatg tactgtttgc ccgggagctc gccaaccagc ttgaggccac tggcgtcacc    780
tgctatgcag cccacccagg gcctgtgaac tcggagctgt tcctgcgcca tgttcctgga    840
tggctgcgcc cacttttgcg cccattggct tggctggtgc tccgggcacc aagagggggt    900
gcccagacac ccctgtattg tgctctacaa gagggcatcg agcccctcag tgggagatat    960
tttgccaact gccatgtgga agaggtgcct ccagctgccc gagacgaccg ggcagcccat   1020
cggctatggg aggccagcaa gaggctggca gggcttgggc ctggggagga tgctgaaccc   1080
gatgaagacc cccagtctga ggactcagag gccccatctt ctctaagcac cccccaccct   1140
gaggagccca cagtttctca accttacccc agccctcaga gctcaccaga tttgtctaag   1200
atgacgcacc gaattcaggc taaagttgag cctgagatcc agctctccta accctcaggc   1260
```

```
caggatgctt gccatggcac ttcatggtcc ttgaaaacct cggatgtgtg cgaggccatg      1320
ccctggacac tgacgggttt gtgatcttga cctccgtggt tactttctgg ggccccaagc      1380
tgtgccctgg acatctcttt tcctggttga aggaataatg ggtgattatt tcttcctgag      1440
agtgacagta accccagatg gagagatagg ggtatgctag acactgtgct tctcggaaat      1500
ttggatgtag tattttcagg ccccaccctt attgattctg atcagctctg gagcagaggc      1560
agggagtttg caatgtgatg cactgccaac attgagaatt agtgaactga tccctttgca      1620
accgtctagc taggtagtta aattaccccc atgttaatga agcggaatta ggctcccgag      1680
ctaagggact cgcctagggt ctcacagtga gtaggaggag ggcctgggat ctgaacccaa      1740
gggtctgagg ccagggccga ctgccgtaag atgggtgctg agaagtgagt cagggcaggg      1800
cagctggtat cgaggtgccc catgggagta aggggacgcc ttccgggcgg atgcagggct      1860
ggggtcatct gtatctgaag cccctcggaa taaagcgcgt tgaccgccaa aaaaaaaaaa      1920
aaaaaaaaaa aaaaaaaaaa aaaaaaaaaa aaaaa                                 1955
```

<210> 44
<211> 3098
<212> DNA
<213> Homo sapiens

<400> 44

```
tcctgcttgt cggcatcgct ccccacaggc cgacgtcgag agggcctgct ttactcctcc      60
tctttctcct ccttctcccg cggcttctgc gcggagaggc gtcgcccggg atctgggttt     120
tggaagaagg atctttgtgg gaagacaggg tgaatttatc acagaggaat aacgagggag     180
aggagaaagg tttcctaaag acaaaaaaaa aaatggagga atctgtaaac caaatgcagc     240
cactgaatga gaagcagata gccaattctc aggatggata tgtatggcaa gtcactgaca     300
tgaatcgact acaccggttc ttatgtttcg gttctgaagg tgggacttat tatatcaaag     360
aacagaagtt gggccttgaa aatgctgaag ctttaattag attgattgaa gatggcagag     420
gatgtgaagt gatacaagaa ataaagtcat ttagtcaaga aggcagaacc acaaagcaag     480
agcctatgct ctttgcactt gccatttgtt cccagtgctc cgacataagc acaaaacaag     540
cagcatttaa agctgtttct gaagtttgtc gcattcctac ccatctcttt acttttatcc     600
agtttaagaa agatctgaag gaaagcatga aatgtggcat gtggggtcgt gccctccgga     660
aggctatagc ggactggtac aatgagaaag gtggcatggc ccttgctctg gcagttacaa     720
aatataaaca gagaaatggc tggtctcaca aagatctatt aagattgtca catcttaaac     780
cttccagtga aggacttgca attgtgacca aatatattac aaagggctgg aaagaagttc     840
atgaattgta taaagaaaaa gcactctctg tggagactga aaaattatta aagtatctgg     900
aggctgtaga gaaagtgaag cgcacaagag atgagctaga agtcattcat ctaatagaag     960
aacatagatt agttagagaa catcttttaa caaatcactt aaagtctaaa gaggtatgga    1020
```

aggctttgtt acaagaaatg ccgcttactg cattactaag gaatctagga aagatgactg 1080
ctaattcagt acttgaacca ggaaattcag aagtatcttt agtatgtgaa aaactgtgta 1140
atgaaaaact attaaaaaag gctcgtatac atccatttca tattttgatc gcattagaaa 1200
cttacaagac aggtcatggt ctcagaggga aactgaagtg gcgccctgat gaagaaattt 1260
tgaaagcatt ggatgctgct ttttataaaa catttaagac agttgaacca actggaaaac 1320
gtttcttact agctgttgat gtcagtgctt ctatgaacca aagagttttg ggtagtatac 1380
tcaacgctag tacagttgct gcagcaatgt gcatggttgt cacacgaaca gaaaaagatt 1440
cttatgtagt tgcttttcc gatgaaatgg taccatgtcc agtgactaca gatatgacct 1500
tacaacaggt tttaatggct atgagtcaga tcccagcagg tggaactgat tgctctcttc 1560
caatgatctg ggctcagaag acaaacacac ctgctgatgt cttcattgta ttcactgata 1620
atgagacctt tgctggaggt gtccatcctg ctattgctct gagggagtat cgaaagaaaa 1680
tggatattcc agctaaattg attgtttgtg gaatgacatc aaatggtttc accattgcag 1740
acccagatga tagaggcatg ttggatatgt gcggctttga tactggagct ctggatgtaa 1800
ttcgaaattt cacattagat atgatttaac cataagcagc agcacgatcc agagatccat 1860
tgccatcagt gatctcacta aaaatataca gctacttccc agctaatctc cacccaatga 1920
atgatgatgg tatagtatgt gcataatgga aagttacctt actgaaaaaa aaaaaagaag 1980
gaaaaataag atgggcccaa aggtctatct actaaactag ctcttgggga aatagcttca 2040
ggatactgta gtttcctcta tctaatagag aactttttgt taacagacac tgtaaaatag 2100
ttttgctttg ttgaataata catgtgtacc taaaagaggt aagagcaaaa agtgtaattc 2160
cacatcatgt tacttgagaa gtgcttaacg ttttcttaaa tgttttcatt gggaaaggac 2220
agctttgata atgtccaaat actctgaaat gcactagacc atataactgt gatgaaatat 2280
gaaactcatc tgtaaacttt tataccaagg gggtaaaaaa aaaaactaag gcatttgatt 2340
aaattatgaa tgagttttac aaattccttt cagagtttta ctaagatcac acaaataaca 2400
gctttcttat tcagtgaaaa agatatttta tttctgatgt tttatttgca ctcgtggaat 2460
atgttaccat taatcagaaa catcatggca acccctaaga atagactaag tttgtgttgg 2520
ctgagggatt ctatttggtt tgcttttttt tttttgcttt gttatatttt attgctacaa 2580
ggggtgtgac ttgataatga tttcctctga attataataa catagccaga tgtagtctca 2640
cactgttttt catactctta agtgtaaata atataaaatg tttcaagcgc ttaactcccc 2700
ctcattcaca aagtataaca attaaaatct caactataac cagtttagct ttttccttac 2760
ttttaaaata aaattttttta cttttaacta ttttttttagt taatattttt aaaagtatac 2820
atgtcaatgg cctctttgtc cattattcat tttgtggcaa aatattcttc tttgatagtg 2880
taaacaaata ataaagcaat ctaggtcctt taggtttgaa aggcaatttt tgagtagcat 2940

attaccagct agccagtcac taggaatttt tttcagtatt atttgtatgt attaaacttt 3000

tcattacact aaagtgcatt attttattga gcaagtatcc ttcattgtga ggtttgacat 3060

taaagcaatc tgttgaaatg ccaaaaaaaa aaaaaaaa 3098

<210> 45
<211> 2689
<212> DNA
<213> Homo sapiens

<400> 45

gatcttgggc tgaggttccc gggcgggcgg gcgcggagag acgcgggaag caggggctgg 60

gcgggggtcg cggcgccgca gctagcgcag ccagcccgag ggccgccgcc gccgccgccc 120

agcgcgctcc ggggccgccg gccgcagcca gcacccgccg cgccgcagct ccgggaccgg 180

ccccggccgc cgccgccgcg atgggcaacg ccgccgccgc caagaagggc agcgagcagg 240

agagcgtgaa agaattctta gccaaagcca aagaagattt tcttaaaaaa tgggaaagtc 300

ccgctcagaa cacagcccac ttggatcagt ttgaacgaat caagaccctc ggcacgggct 360

ccttcgggcg ggtgatgctg gtgaaacaca aggagaccgg gaaccactat gccatgaaga 420

tcctcgacaa acagaaggtg gtgaaactga aacagatcga acacaccctg aatgaaaagc 480

gcatcctgca agctgtcaac tttccgttcc tcgtcaaact cgagttctcc ttcaaggaca 540

actcaaactt atacatggtc atggagtacg tgcccggcgg ggagatgttc tcacacctac 600

ggcggatcgg aaggttcagt gagccccatg cccgtttcta cgcggcccag atcgtcctga 660

cctttgagta tctgcactcg ctggatctca tctacaggga cctgaagccg gagaatctgc 720

tcattgacca gcagggctac attcaggtga cagacttcgg tttcgccaag cgcgtgaagg 780

gccgcacttg gaccttgtgc ggcacccctg agtacctggc ccctgagatt atcctgagca 840

aaggctacaa caaggccgtg gactggtggg ccctgggggt tcttatctat gaaatggccg 900

ctggctaccc gcccttcttc gcagaccagc ccatccagat ctatgagaag atcgtctctg 960

ggaaggtgcg cttcccttcc cacttcagct ctgacttgaa ggacctgctg cggaacctcc 1020

tgcaggtaga tctcaccaag cgctttggga acctcaagaa tggggtcaac gatatcaaga 1080

accacaagtg gtttgccaca actgactgga ttgccatcta ccagaggaag gtggaagctc 1140

ccttcatacc aaagtttaaa ggccctgggg atacgagtaa ctttgacgac tatgaggaag 1200

aagaaatccg ggtctccatc aatgagaagt gtggcaagga gttttctgag ttttaggggc 1260

atgcctgtgc ccccatgggt tttctttttt cttttttctt ttttttggtc gggggggtgg 1320

gagggttgga ttgaacagcc agagggcccc agagttcctt gcatctaatt tcacccccac 1380

cccaccctcc agggttaggg ggagcaggaa gcccagataa tcagagggac agaaacacca 1440

gctgctcccc ctcatcccct tcaccctcct gccccctctc ccacttttcc cttcctcttt 1500

ccccacagcc ccccagcccc tcagccctcc cagcccactt ctgcctgttt taaacgagtt 1560

tctcaactcc agtcagacca ggtcttgctg gtgtatccag ggacagggta tggaaagagg 1620

ggctcacgct taactccagc ccccacccac accccccatcc cacccaacca caggccccac 1680

ttgctaaggg caaatgaacg aagcgccaac cttcctttcg gagtaatcct gcctgggaag 1740

gagagatttt tagtgacatg ttcagtgggt tgcttgctag aatttttta aaaaaacaac 1800

aatttaaaat cttatttaag ttccaccagt gcctccctcc ctccttcctc tactcccacc 1860

cctcccatgt ccccccattc ctcaaatcca ttttaaagag aagcagactg actttggaaa 1920

gggaggcgct ggggtttgaa cctccccgct gctaatctcc cctgggcccc tccccgggga 1980

atcctctctg ccaatcctgc gagggtctag gcccctttag gaagcctccg ctctcttttt 2040

ccccaacaga cctgtcttca cccttgggct ttgaaagcca gacaaagcag ctgcccctct 2100

ccctgccaaa gaggagtcat cccccaaaaa gacagagggg gagccccaag cccaagtctt 2160

tcctcccagc agcgtttccc cccaactcct taattttatt ctccgctaga ttttaacgtc 2220

cagccttccc tcagctgagt ggggagggca tccctgcaaa agggaacaga agaggccaag 2280

tccccccaag ccacggcccg gggttcaagg ctagagctgc tggggagggg ctgcctgttt 2340

tactcaccca ccagcttccg cctcccccat cctgggcgcc cctcctccag cttagctgtc 2400

agctgtccat cacctctccc ccactttctc atttgtgctt ttttctctcg taatagaaaa 2460

gtggggagcc gctggggagc caccccattc atccccgtat ttccccctct cataacttct 2520

ccccatccca ggaggagttc tcaggcctgg ggtggggccc cgggtgggtg cgggggcgat 2580

tcaacctgtg tgctgcgaag gacgagactt cctcttgaac agtgtgctgt tgtaaacata 2640

tttgaaaact attaccaata aagttttgtt taaaaaaaaa aaaaaaaaa 2689

<210> 46
<211> 1936
<212> DNA
<213> Homo sapiens

<400> 46

```
ccggagcctc ctggaccagg agaactgtaa cgcgagcccc gagccatggg cgaaaggcgg        60
ggccgagacg ggttgggggc gccgacggtt tcccggccct ggctgcagct tggaggagaa       120
gctgagcctg tgcttccgcc cctcggatcc gggcgccgag cccgaggacg gccgtgcggc       180
catcacggag ctcaactcct gcagggggac gagatttgga atgccctgac agataattat       240
gggaatgtga tgcctgtaga ctggaagtca tcgcatacta ggaccttgca cttgcttact       300
ctgaacctct cagaaaaagg ggtaagtgac agtttgctct ttgatacatc agatgatgaa       360
gagctgagag aacagctgga tatgcactca atcatcgtct cctgtgttaa tgatgaaccc       420
ctcttcacgg cagaccaggt tattgaagaa attgaagaaa tgatgcagga atcaccggac       480
ccagaagatg atgaaacccc tacacagtca gatcggcttt caatgctttc ccaggaaatt       540
```

caaactctca agaggtctag taccggcagt tatgaagaga gagtgaaaag gctctcagtg 600

tctgagttaa atgaaatcct ggaagaaatt gagactgcca ttaaggagta ctctgaggag 660

ctggtgcagc agttggcttt acgagatgaa ctggagtttg aaaaggaagt gaaaaacagc 720

tttatttctg ttcttattga agtgcaaaac aaacagaaag agcacaaaga aacagcaaaa 780

aagaaaaaga aactaaaaaa tggcagctct cagaatggga agaatgagag aagtcatatg 840

cccggcacat atttgactac agtcattcct tatgagaaaa aaaacggacc accgtctgtt 900

gaagatcttc aaatattaac aaaaattctt cgtgccatga aggaggacag tgaaaaagtt 960

ccgagcttgt taactgatta tattctgaaa gttctgtgtc ctacatagag cagcaacttt 1020

atctgcggtg ggctccaagc tagatttccg acagcattat tctgagagct ggctaccatt 1080

acccttcttg ctattggaaa ctcagcacat ttgaacttgg gtttgattca gtattaacag 1140

atcttgacta cactaattct ttatattata gaaccaacgg aaatatgggc actattttga 1200

attctagaga tggtttttgt taaatctact aataaactgt tctcttagta gattaagaga 1260

gagtaatatt aattgtgcat gtgcagttgt atttctcatt aactgacagt atgcccattt 1320

gtttttatgg ctttcttatc taaactgcac tgatgaacta gattaaagcc ttgggagatt 1380

tatactataa attcagtgat ggcaagaacc aacactgttt ttttgtgaga attgtcagtg 1440

taactattac ctaccagtat tgttcagaga gattgaaaca gaataaacgg gctgttcttg 1500

aagaagcaaa accagaatat gcattacttt ggtttaatac ttagtgctaa cattgaaact 1560

gttggtggtg atggattttg tagcttgctg cttgtttcac cactggtcaa attttaacca 1620

ttaaattgcc attcactttt agaatcttgt atttaagtaa gttttgattt tcaaatgttc 1680

tgcttcatgt gtctgtgaag aattgtactt ttttaaaagt gtgtgtcctc tgaggtgctt 1740

gagaaagtgt acactgcaga actgcccatt ctcattactg tgtcctattt tattcatgcc 1800

tgtgtgtttt tcttaagtat gaattctaga tacagctact tatggattca tcaatatcat 1860

gagcactttt gctggttcca gtcaaatcaa tggcatttaa taaatttttt aagaagtaaa 1920

aaaaaaaaaa aaaaaa 1936

<210> 47
<211> 3990
<212> DNA
<213> Homo sapiens

<400> 47

ggagtttagg gcctgacaga agcccgcccc cgctggcgct cgtgcgcacg cgtggcgggc 60

tctcggcgca ctgagcaggc gcggcctcgt gtcggccgga gggggcgggc gcaacgacgc 120

gcgctgcgtc ccggcgctcg gctttccctc cgccggtccc gccctccgtc gcggcggcgc 180

ggtgtaccct gggataggga gcgatctccg agcgaggcgg caagatggac gcgggatttt 240

```
tccgcggaac aagtgcagaa caggataatc ggttcagcaa caaacagaag aaactactga      300
agcagctgaa atttgcagaa tgcctagaaa aaaaggtgga catgagcaaa gtaaatttgg      360
aggttataaa gccttggata acaaaaagag taacggaaat ccttgggttt gaagatgatg      420
ttgtgattga gtttatattc aaccagctgg aagtgaagaa tccagactcc aaaatgatgc      480
aaatcaacct gactggattt ttgaatggaa aaaatgctcg agaatttatg ggagaactgt      540
ggcccctgct gctaagtgca caagaaaaca tcgcgggaat cccttctgct ttcctagaac      600
tgaagaaaga agaaataaaa caaagacaga ttgaacaaga aaaactggca tctatgaaaa      660
agcaagatga agacaaagat aaaagagata aggaagaaaa agaaagcagc agagaaaaaa      720
gggagcggtc tcgtagccca agaagacgca aatccagatc tccttcccct agaagacgat      780
cttcccctgt caggagagag agaaagcgca gtcattctcg atctccccgt cacagaacca      840
agagccggag tccttcccct gctccagaaa agaaggaaaa aactccagag ctcccagaac      900
cttcagtgaa agtaaaagaa ccttcagtac aagaggctac ttctactagt gacattctga      960
aagttcccaa acctgaacct ataccagagc ctaaagaacc ttctccggaa aaaaattcca     1020
aaaaagaaaa ggagaaggag aagacccgac cacgatctcg gtcacgctcc aaatcaagat     1080
cccggacgcg gtcccgctct ccttctcaca ctcgacctag acggcgccat agatcccgat     1140
caagatcgta ttcacctaga aggcggccaa gcccaagaag gcggccatct cctcgaagaa     1200
gaactccgcc aagaagaatg cctcctccac caaggcatag aaggagtaga tctccagtaa     1260
gacgaagaag acgttcgtca gcatccttgt ctgggagtag ctcatcatcc tcttcatctc     1320
gttcacggtc accaccaaag aagcctccca agaggacatc cagccccccct cggaaaactc     1380
gtaggttatc tccttcagca agtcctccaa ggcgaaggca caggccatca cctcctgcaa     1440
ctccaccacc caaaactcgg cattccccta caccccagca gtcaaaccgt acaagaaaaa     1500
gtcgtgtttc tgtgtctcca gggagaactt caggtaaagt gacaaaacat aaaggtactg     1560
agaaaagaga atccccttca ccagcaccga agcctagaaa agtagagtta tctgaatcgg     1620
aagaagataa aggtggcaaa atggctgcag cagattctgt gcagcagaga cgccaataca     1680
gacgacaaaa ccagcagtct tcatctgact ctggctcctc ctcctcctca gaagatgaac     1740
gacccaagag atcccatgtg aagaatggtg aggttggcag gcggcggaga cattcccctt     1800
cccggagtgc ttctccatca ccacgaaagc gccaaaaaga gacttcccct cgtggtagac     1860
ggaggagaag tccatcccca ccacccacca gaaggcgacg gtctccttct cccgcccctc     1920
ctcctcgacg gcgcaggact cccacaccac caccacgacg aaggactcct tctcctcccc     1980
cacgtcggcg ctcaccttct cctagaagat actctcctcc aatacagagg agatactctc     2040
cttctccacc tccaaagaga agaacggctt cacctcctcc ccctcctaaa cgaagagcat     2100
caccatctcc accaccaaag cggcgggtct cccattctcc acctcccaaa caaagaagct     2160
```

ccccagtcac caagagacgt tcaccttcat tatcatccaa gcataggaaa gggtcttccc 2220
caagccgctc tacccgggag gcccgatcac cacaaccaaa caaacggcat tcgccctcac 2280
cacggcctcg agctcctcag acctcctcaa gtcctccacc cgttcgaaga ggagcgtcgt 2340
catcacccca aagaaggcag tccccgtctc caagtactag gcccattagg agagtctcca 2400
ggactccgga acctaaaaag ataaaaaagg ctgcttcccc aagcccacag tctgtaagaa 2460
gggtctcatc ctcccgatct gtctccgggt ctcctgagcc agcagctaaa aagcccccag 2520
cacctccatc ccccgtccag tctcagtcac cgtctacaaa ctggtcacca gctgtaccgg 2580
tcaaaaaggc caaaagccca acaccgagcc catcaccgcc aagaaattca gatcaggaag 2640
gaggtggaaa gaaaaagaag aaaaagaagg acaagaaaca caaaaaggat aagaagcaca 2700
agaagcacaa aaaacacaag aaggaaaagg ctgtggctgc agctgctgca gctgctgtga 2760
cccctgcagc cattgcagct gccacaacca cattagcaca ggaagagcca gtggcagcgc 2820
cagagccgaa gaaggagact gaaagtgaag ctgaagataa ccttgatgat ttagaaaagc 2880
acctgcgtga aaaggccctg agatcaatga ggaaggccca agtgtcccca cagtcttagg 2940
gggaaatgtt tgttatgatg taaattttat ttggtttgta cgcagttcaa tttcaaaatt 3000
gctaaaatgt gtttgagctt tagactataa catttgttgt aataattgct aggttgaagt 3060
tcaacatgta aaaaaagggg gcatggattt acattgcaaa aggtgtccac agtgtattag 3120
tgacattctt tcattgacag ctgacataat tcattgagtg aaatatttta agccaaaaaa 3180
aaattccctt tttaaaaaag ggggtttaaa tactgttggc atttttatgg ttcctttaaa 3240
tgccctagct attcccagag gggttttttt gtttgttttt ttggttttga ttttcttttt 3300
gtttttcttt cttcttctta tttttttcat ttgagtctta gctcccattt aagttatgct 3360
tctgaccttg tatggtctgt aagcttgccc agaaataaga ccactgtttt gaactaccac 3420
aaaagtataa atgaatattt taatgccaca atctttcctg ttgcctgtgg agtctctgct 3480
gaaatgaatc aggattcgag ctctaggatg agacagaaaa tgaaagcatg ttgtttgcca 3540
ggacactgtg ggtttatatt gatgtgtaac aagttgattt ggaacactgg actctcattc 3600
tgttattctg gttttgtttt ttttgttttg tttttttttct tttgtaaagg caatgagcta 3660
gtcccagaaa ggatccttca gttacataca atttgtttaa tgaaatgtca tggctctgtt 3720
catatttttg tcttgttctt ccaattggta tatacaactt tcagagcctc ttgtatttgg 3780
aaggctggaa gggcccagac tttggaatag tgtcttggtt tcactgtttt tgttttgatt 3840
tttttttgt tttgattttt tttaaactaa agctatataa agcttgtgga ttaaacagaa 3900
taaatttcta aatttaaaaa tttaaaaaaa aaaaaaaaaa aaaaaaaaaa aaaaaaaaaa 3960
aaaaaaaaaa aaggaaaaaa aaaaaaaaaa 3990

<210> 48
<211> 1502
<212> DNA

<213> Homo sapiens

<400> 48

```
cctgcgtccc cgccccgcgc agccgccgcg ctcctgcgct ccgaggtccg aggttcccga      60
gatgaaggtc tggctgctgc ttggtcttct gctggtgcac gaagcgctgg aggatgttac     120
tggccaacac cttcccaaga acaagcgtcc aaaagaacca ggagagaata gaatcaaacc     180
taccaacaag aaggtgaagc ccaaaattcc taaaatgaag gacagggact cagccaattc     240
agcaccaaag acgcagtcta tcatgatgca agtgctggat aaaggtcgct tccagaaacc     300
cgccgctacc ctgagtctgc tggcggggca aactgtagag cttcgatgta aagggagtag     360
aattgggtgg agctaccctg cgtatctgga cacctttaag gattctcgcc tcagcgtcaa     420
gcagaatgag cgctacggcc agttgactct ggtcaactcc acctcggcag acacaggtga     480
attcagctgc tgggtgcagc tctgcagcgg ctacatctgc aggaaggacg aggccaaaac     540
gggctccacc tacatctttt ttacagagaa aggagaactc tttgtacctt ctcccagcta     600
cttcgatgtt gtctacttga acccggacag acaggctgtg gttccttgtc gggtgaccgt     660
gctgtcggcc aaagtcacgc tccacaggga attcccagcc aaggagatcc cagccaatgg     720
aacggacatt gtttatgaca tgaagcgggg ctttgtgtat ctgcaacctc attccgagca     780
ccagggtgtg gtttactgca gggcggaggc cgggggcaga tctcagatct ccgtcaagta     840
ccagctgctc tacgtggcgg ttcccagtgg ccctccctca acaaccatct tggcttcttc     900
aaacaaagtg aaaagtgggg acgacatcag tgtgctctgc actgtcctgg gggagcccga     960
tgtggaggtg gagttcacct ggatcttccc agggcagaag gatgaaaggc ctgtgacgat    1020
ccaagacact tggaggttga tccacagagg actgggacac accacgagaa tctcccagag    1080
tgtcattaca gtggaagact tcgagacgat tgatgcagga tattacattt gcactgctca    1140
gaatcttcaa ggacagacca cagtagctac cactgttgag ttttcctgac ttggaaaagg    1200
aaatgtaatg aacttatgga aagcccattt gtgtacacag tcagctttgg ggttcctttt    1260
attagtgctt tgccagaggc tgatgtcaag caccacaccc caaccccagc gtctcgtgag    1320
tccgacccag acatccaaac taaaaggaag tcatccagtc tattcacaga agtgttaact    1380
tttctaacag aaagcatgat tttgattgct tacctacata cgtgttccta gtttttatac    1440
atgtgtaaac aattttatat aatcaatcat ttctattaaa tgagcacgtt tttgtaaaaa    1500
at                                                                   1502
```

<210> 49
<211> 385
<212> DNA
<213> Homo sapiens

<400> 49

aaaaaaatta aggctaacca agtgcatcca ttgttcaatg gcacaattga tttcagcaac 60
tatttggaat atcctaatta taggaaatgc ccatctaagt gatatattta aataatacaa 120
tcaatttttt aaggtgaata aactatgatg gtttctaaat agtgtacatg ttacctgaaa 180
aatcagaaaa cacaaagaat gattaatttc gaaagttctt gcctaaaggc accactgact 240
taaaaaacat tcaaaatcaa ataccacaag acataaagcc tcttcatgta tatattcata 300
tatgcaataa atgcattaaa tgtaacttta ttaaacatag tacactgtac ttgacttatg 360
gttaaatatt ttacacacag cttga 385

<210> 50
<211> 6313
<212> DNA
<213> Homo sapiens

<400> 50

gccaggtccc tgaggggcgg gcagatgagg cctaggggtg ccgatcccta gtgtcgacta 60
tgcgagatct gattccggag ctgccatgat tgaagtggta gcagagctca gccggggtcc 120
tgtatttttg gctggggagg cgctggagtg tgtagtgacc gtcaccaacc cccttccgcc 180
cacggccact tctgcatcca gtgaggccct ggcctgggcc agtgcccaaa tccactgcca 240
gttccatgcc agtgagagtc gagtagcact gcctcctcct gactctagtc agccagatgt 300
ccagcccgac agccagactg tctttctgcc acaccgaggt gagaggggcc agtgtatcct 360
ttctactcca ccgaaaattc tattctgtga cctgaggctt gatcctggag agtccaaatc 420
atactcctac agtgaagtgc tgcccataga gggaccaccc tcctttcggg gtcagtcagt 480
caagtacgtc tacaaactga ccattggctg ccagcgtgtc aactccccta tcactttact 540
cagagtccct ctgagggttc ttgtgctgac tggccttcag gatgtccggt ttccccagga 600
tgaggctgta gccccatcca gtccattctt ggaggaggat gaaggtggga agaaagattc 660
atggctagct gagctggctg gggaacgcct aatggctgcc acatcctgcc gcagcctcca 720
tctatacaat atcagtgatg gccgagggaa agttgggacg tttggcatct tcaaatctgt 780
gtacagactt ggcgaggacg tggtggggac cttaaactta ggggaaggaa ccgtagcttg 840
tttgcagttt tcagtcagct tacagaccga ggagcgtgta cagcctgagt accagcggcg 900
acgtggggca gggggtgtcc cctctgtgtc acatgtgact cacgcccggc accaggaatc 960
ctgcctacat acaactagaa ccagcttctc cctcccaatc cctctcagct ccacccccagg 1020
cttctgtaca gccattgtgt ccttgaagtg gagattgcat tttgaatttg taacgtcccg 1080
agaaccagga ttggtactcc taccccctgt ggaacagccc gaacctacca cctggacagg 1140
acctgagcaa gtacctgtag acaccttcag ctgggacctg cccatcaagg tgctgcctac 1200
tagccccacc ctggcctcat atgctgcccc aggccccagc accagcacca taaccatctg 1260
aaactggccc accctggtgc tagttccttc cggatactga gaactcagca cctggactct 1320

aatgggaccc actttttcca cctggggtcc aatgtcgtgg acagtgagag tcgggctttc 1380
agctatagca ttaatttatt tgttcagaat acattggcag ctgctagtgg tttccctgga 1440
agtggcagca gcagtgagca gtcagcagat ggatgatcag ttgagtttag ctggagtggg 1500
gagcaggagc cccaggaaca ggggtgttgg ctgagcccca ttctgggtca ggccctcccc 1560
ctttgcaggg cagccgaggg tcagattttt gcaccaagga gaactggcag gttcctgcct 1620
cctgacgtac ctcacaccca gccgggaagt cgatgggatg ctgggacctg gggaaccaag 1680
gataggggaa ggagtcagca cagtgaaagg ctgcctttat ccctgcccac atgttccctc 1740
tctcacagtt ttccccccac agagcccctt tcagtggccc cttggtcctc ctaactaagc 1800
tgtcacctac catatgtggg cctttttgtt ttataacagg agtattttct ctccaggtcc 1860
accccaacct cccctgattt atagcctgaa gccttatctt tcacactagt gttggtccct 1920
tcaggtttgg cccatcttgt attgctcttc tgttcattct tacatcacag caatctagtc 1980
actccctggt catccctcag tcactcatat cagagtcatt ctctctggcc atctttggtc 2040
actcacgtgt cacagcagcc cacgccaaca ggatgcagac aggtgcaatg gaaacagtcc 2100
ttgcggagcc aagactcacc cagggtaaaa tatttcccct catagtgaca gggggctagg 2160
gaagaacggg aaatgttagt aggtgtagga gtgctgatga gaggcagagg ctcttctggt 2220
ctggggtgga gacagtaagt acgcactatc cccgtattta gtttgtcttt cctgtttcac 2280
agctggagga agcctgggta ttttgacacg ggatcatctg taaggcccca tcctccctgt 2340
gccctctctg ctgctcctcc attcctaacg cttcacccca ctttaccttg agcttggaag 2400
tagcacttgc tgtagactcc tgggtgctgg aggagtagag acatcaccaa gcagatgatc 2460
ccccagcctc ctaggatccc cttggcctgt ccagcccaga gcatccttag ggccattgct 2520
gctgcacagc cctctcagac ccttcttggc ctctgctcag ctactctggt cttgactcct 2580
tgactttgct ttgcgttgct ccttgagtct tagtttctgt ctttctcccc tgggctcctg 2640
tctcacacta tctccctgcc ctctgctctc acaggctggg gatgtttata aagtgaggac 2700
cctggccccc tgctgagtag agctggaaaa gttgtaactc tgtttcctga ggtgagggca 2760
tgaaaacaag aggtctagct ttaacaagct gtgagagctg attcatgccc cggcacagct 2820
agagggaggg aggtggccat ggagggggca ctggactggg cacttcccca gcaaggaggc 2880
aggaggggcg agggccccca ggtggtcccc agatctcttc cctgacctgg agagaaggaa 2940
gcattccacc ttcccccttt ctcccccact gccaccacca ggggtgtgta tgctgggatc 3000
cctgcctgga ccggagggag gcatttcctg gggatggtta atcctgtgcc ccagccaaac 3060
ccaggagctg caatagggtg cgacggccag aagctccagg agagtgagca ggcacctgga 3120
gtggagactg tgtttccctc agatcctagg gcagggtttc cctaatgtat ccaagaaata 3180
gggctgcccc tcagagatgg tggggagggt ctcttttcct caggcattcc agaggtgaac 3240

tgtccattgc ttatcacctt caaacataca gcagatgtgg gatcacccca catctgggga 3300

tggttctttc ccctttcaaa gaggagcatc tctaagtgcc ctgatgggat gaatcactcc 3360

aggttcacag aggtgtcctc tctttcctcc catatataat ggagtgaggt ttttaggaat 3420

ttatcatttg gcatcctctg agtttcccac aggttctgga ggagcccagg atggattatt 3480

gagagcatgg gctgtagaga cagtcttctt ggattcagat cctgactcca cttagctatg 3540

taacctggtc agattacttc acctctctga gcctgtttcc tcatctataa attggggata 3600

gtaatgccaa ctcattgggc tgttatgagg attactgaga taatgcgtgc agtgctctta 3660

tcaccatctc tggtgcgtaa gcgtcaggaa atagcagttg ctgtgattgg ggctaaagct 3720

ctgaggcaaa atgggcgaca ttattttctt tgaatgacat taagcagttt gtgcatagct 3780

gagggcttct attggggatg gctgtctcct ggcatagacc tctgcacctt tcacactcat 3840

actccttgtc agcagtcccc aacctttttg gtaccaggga ccggttttgt ggaaaacaat 3900

ttttccacca gtggatggag ggggatagca gcggggagat gattttggga tgaaactgtt 3960

tcatctcaga tcatcaggca ttagattctc ataaggagtg tgcaatctag atcccttgca 4020

tgcggagttc acagtggggt ttgcactcct gtgagaatct aatgcctctg ctgatctgcc 4080

aggaggagga gctcaggcgg taatgctcac tcgcctgccg cccacctcct gctttgtgct 4140

cccgcttcct aacaggccac agactggtac tggcctgtgg cctgggggat ggagacccct 4200

aatccatgtc acctttccca cctctttcaa aaacaggtac ctccaggaac attttggttt 4260

tggcccttgt attgacttct gaatgtctag tttgagaaac tgttcccaaa taagccttct 4320

tcccccagat ctgcaccctc gcctctaccc taggacaaga tgtccttttc tcatcatcct 4380

gccaggctaa ctttaagtct cctgcttttt ctcacttgga tttggatcca tttcttccta 4440

tttccgctca tgtgaactct ccagttctcc tttctcacca ctctcctgct agccatctct 4500

ttggcactaa aggccctggt caaattggat ttctttcatt tttccacact tcaaagaccc 4560

atgttctagg tattctccat agggatagtc tctttggcat ttatttggtt tttctacgtt 4620

ttcagtccca tttactccaa gactcactcc ctgccaccta gtgcatcaga tacagctact 4680

tctggctgac ttttcaaggg ggaccaccct acctgtcatc tcttcactgt tcagaaatga 4740

ctgtgtcagt gcacctcaaa ctcccttgct gtccttttcc aaggagacag ctaaggtgga 4800

tggagatgca gaatggacct cacgttcgcc ctagtcagga ctgataccct ttccgtttca 4860

gaggattgcc aagaaaaaac tcacagttga ggcagggtgc tctgaggtcg gctgcggtgt 4920

gggaggcacg gcctgggcct gctctctggg ctggagcagg tggattcgaa ggcctgtcta 4980

gcacgagggc ccaaaggtct tgtcagtggc cagtagctct gccgcctttc ccagagaggg 5040

ggtccagggg acatcctgga aggctgggcc ctgggccacc ttctgctctt gcaagctaga 5100

gccagcccaa taggggggcgg atgtgagtgg ggagctgggg cgcatgaagg tgggggtgat 5160

gccgaagggg aagggatcgc cagtggggat tggtgcgtgt gcggaaacgg ggacagaagt 5220
gaaggttcat cgcctataac gaagatgagg taggcatata ggggcttctg gaaagctaga 5280
ggctgggctg agccaggagt cctctcccag aagttggggg gcggtgcaga ggtgtgggtc 5340
gagcccgcat gcgtgcctgc tggggagggg gtgagtggtg aggaccaggc ccgctgggtc 5400
ctgggggcgc ggtggctggc gcgcaggtcc cggagggggc ggctggcgcg cactacacgc 5460
ttgggaacaa ggaaaacatc cgccggaggc ccggccgggc ggcgctccag cctcggggca 5520
ggtgcgcgga gaggaagtga gagcattccg gcccccccac cccaaccccg gccgctggcc 5580
ctctggtgag tcacagccga cccccgccgc cggagggaga ggggagctgc gggccagagc 5640
cccggagggt ctggaggagc caggagggtt tctgggagca gagggtcact tagtgggctt 5700
ctgtcgtggt gtcgctacgg gcgcgaaacg gacactgaac acagtctgac tgtatggagg 5760
caggtgggga gggatcccct gggagaactt ggcgggccga gagcagaccc cagggcaagg 5820
aggggccccc gaggggggaaa ccgggagtcg ggcaggtggc gtaacccaga aagggaagga 5880
gagccggatt gattggggtg agagaggaag gaagcacgcc aagttaggcc tgggagaact 5940
gagggacctg aggagggagg agggagacca acacagggtg ggaaggcgga aatggccaaa 6000
ccccaggcat caggtctgtc cagaggctga cgtagacagt gaagggtgaa gggtaggttt 6060
taggagtagg gggagttatg attatttggt tacattttgg gattatttgg tctcacaggt 6120
agaagggagc ctgctggtct ctgtgtaacg gatggcttaa aagcaaggtt gtctgcgtct 6180
tggattactg tctgccattc agcctttgcc aaaaaatttg gcactgatct gcacattttt 6240
atagtcattt aaaattgtat gactctgtca aatgatttaa gtaattttgg tggattttta 6300
aaaataaaaa aat 6313

<210> 51
<211> 1133
<212> DNA
<213> Homo sapiens

<400> 51

ggtaaggggt cctccctgcg ccacacggcc gtcgccatgg tgaagctgag caaagaggcc 60
aagcagagac tacagcagct cttcaagggg agccagtttg ccattcgctg ggctttatc 120
cctcttgtga tttacctggg atttaagagg ggtgcagatc ccggaatgcc tgaaccaact 180
gttttgagcc tactttgggg ataaaggatt atttggtctt ctggatttgg aggcaatcag 240
cggacagcat ggaagatgtg tgctctggct cggataagag atgggacatc attcagtcac 300
tagttggatg gcacaaggct cttcacagac gcatctgtag cagagtggat cttgtactaa 360
cttatgatag aatgtatcag aataaatgtt tttaacagtg taaacaccac aaacaaaaaa 420
cacaacacac acatcataca cacaaaaaac acaaaaaaaa caaacaaatc acacaaaagc 480

tacggtagac ctactattat gcggtgggcc gaaacaagac gggtattata gacaagggaa 540

acgagtcgtc aaatcgtcgt agcctgacac acatcatatt gttagaccca gcgtgtgcaa 600

tatctcgccg gggtagctcc ctcatatgag ggacacgtta tatatgtctc agatagggcg 660

ccggggtata acctgcagtt ttatagatat gctggcaaca gaaaaaagcg atgtaaaaaa 720

aaaaatgaag acaacataaa cacacacaca aagatactat cacatatata ctataaccaa 780

aaaatctcaa agcgtaaatc aaaaatacac taaaacaatt cacagccata ttcactacac 840

cctatccacc ccacacaaaa aaaataagac acaaaacatc acacatatac acactaccta 900

tcattttata ctttaatcta atataattaa gtaacaaatc aacacaaata tacacacgat 960

cgatagatac actgataaaa ttcaacaaac aaaataccaa ataaaatata ctaaacacac 1020

cactagacga gcatcttata ttgcactttt acgtagacct ctgatcaata acaacagacc 1080

tactccacaa atatactact aacacacaaa caatgcaaac agcacagaat aac 1133

<210> 52
<211> 20
<212> DNA
<213> Artificial

<220>
<223> Oligonucleotide primer

<400> 52
gccataagtg gtcccacagt 20

<210> 53
<211> 23
<212> DNA
<213> Artificial

<220>
<223> Oligonucleotide primer

<400> 53
gtcttctagt ccgtcatctc cct 23

<210> 54
<211> 22
<212> DNA
<213> Artificial

<220>
<223> Oligonucleotide primer

<400> 54
ccatagctgc ctttatgtct gc 22

<210> 55
<211> 22
<212> DNA
<213> Artificial

<220>
<223> Oligonucleotide primer

<400> 55
ctttttacct tcgtgcacct tt 22

<210> 56
<211> 23
<212> DNA
<213> Artificial

<220>
<223> Oligonucleotide primer

<400> 56
taatctgctg aggacctttt gtc 23

<210> 57
<211> 23
<212> DNA
<213> Artificial

<220>
<223> Oligonucleotide primer

<400> 57
taattcactg tcctcttctg gga 23

<210> 58
<211> 21
<212> DNA
<213> Artificial

<220>
<223> Oligonucleotide primer

<400> 58
gctcacagca gtaaatgcct a 21

<210> 59
<211> 23
<212> DNA
<213> Artificial

<220>
<223> Oligonucleotide primer

<400> 59
tgctatgctg taaacactgg cta 23

<210> 60
<211> 23
<212> DNA
<213> Artificial

<220>
<223> Oligonucleotide primer

<400> 60
ggatccttta ttggtggtag agc 23

<210> 61
<211> 23
<212> DNA
<213> Artificial

<220>
<223> Oligonucleotide primer

<400> 61
ccagagtgac cctgaagata aat 23

<210> 62
<211> 23
<212> DNA
<213> Artificial

<220>
<223> Oligonucleotide primer

<400> 62
acctgattct ctaggtgcag ttt 23

<210> 63
<211> 23
<212> DNA
<213> Artificial

<220>
<223> Oligonucleotide primer

<400> 63
gtcgtttcaa ccaggtagtt ttg 23

<210> 64
<211> 20
<212> DNA
<213> Artificial

<220>
<223> Oligonucleotide primer

<400> 64
gggcgcctta agttattgga 20

<210> 65
<211> 22
<212> DNA
<213> Artificial

<220>
<223> Oligonucleotide primer

<400> 65
ggatggtaga aaagcaaact gg 22

<210> 66
<211> 23
<212> DNA
<213> Artificial

<220>
<223> Oligonucleotide primer

<400> 66
tgtaatggag attgtacagg ttg 23

<210> 67
<211> 23
<212> DNA
<213> Artificial

<220>
<223> Oligonucleotide primer

<400> 67
aggaacagta caaatgctgt ggt 23

<210> 68
<211> 23
<212> DNA
<213> Artificial

<220>
<223> Oligonucleotide primer

<400> 68
gcactccttg aaggtacact aac 23

<210> 69
<211> 22
<212> DNA
<213> Artificial

<220>
<223> Oligonucleotide primer

<400> 69
atttgtattc actcagccat gc 22

<210> 70
<211> 23
<212> DNA
<213> Artificial

<220>
<223> Oligonucleotide primer

<400> 70
acccaacttc aaaactagga ctc 23

<210> 71
<211> 23
<212> DNA

<213> Artificial

<220>
<223> Oligonucleotide primer

<400> 71
acagcttgat gtcctttcta tgc 23

<210> 72
<211> 22
<212> DNA
<213> Artificial

<220>
<223> Oligonucleotide primer

<400> 72
tcatgaaagg cactgagttt tg 22

<210> 73
<211> 23
<212> DNA
<213> Artificial

<220>
<223> Oligonucleotide primer

<400> 73
gttagctgaa gcagctttat tgc 23

<210> 74
<211> 22
<212> DNA
<213> Artificial

<220>
<223> Oligonucleotide primer

<400> 74
atatgcacaa tcctggaagt ga 22

<210> 75
<211> 23
<212> DNA
<213> Artificial

<220>
<223> Oligonucleotide primer

<400> 75
tgccttacta gcattaccac cat 23

<210> 76
<211> 21
<212> DNA
<213> Artificial

<220>

<223> Oligonucleotide primer

<400> 76
gaggtgatag cattgctttc g 21

<210> 77
<211> 21
<212> DNA
<213> Artificial

<220>
<223> Oligonucleotide primer

<400> 77
caagtcagtg tacaggtaag c 21

<210> 78
<211> 21
<212> DNA
<213> Artificial

<220>
<223> Oligonucleotide primer

<400> 78
tcttacaccc agtggagaag c 21

<210> 79
<211> 20
<212> DNA
<213> Artificial

<220>
<223> Oligonucleotide primer

<400> 79
gtctttgtgt tcccggacat 20

<210> 80
<211> 20
<212> DNA
<213> Artificial

<220>
<223> Oligonucleotide primer

<400> 80
actatgtccg ggaacacaaa 20

<210> 81
<211> 18
<212> DNA
<213> Artificial

<220>
<223> Oligonucleotide primer

<400> 81

ttccgtcata tggcttgg 18

<210> 82
<211> 23
<212> DNA
<213> Artificial

<220>
<223> Oligonucleotide primer

<400> 82
cgtcgctatc aaggaattaa gag 23

<210> 83
<211> 23
<212> DNA
<213> Artificial

<220>
<223> Oligonucleotide primer

<400> 83
gtagctccag acatcactct ggt 23

<210> 84
<211> 1951
<212> DNA
<213> Homo sapiens

<300>
<308> NM 024829
<309> 2001-03-18
<313> (1)..(1951)

<400> 84

```
gagcccggag caggccgggc tcaagaaaga ggaaagttga ggacgcccca cctcggagag     60
gcggcgcccc tgagtaggcc aggagcctct cttgcaactt ctgccaccgc gggccaccgc    120
ggccgcctga tcccgcagag gaaggtcgcg gccgtggagc gatgacccgc ggcggtccgg    180
gcgggcgccc ggggctgcca cagccgccgc cgcttctgct gctgctgctg ctgctgccgc    240
tgttgttagt caccgcggag ccgccgaaac ctgcaggagt ctactatgca actgcatact    300
ggatgcctgc tgaaaagaca gtacaagtca aaaatgtaat ggacaagaat ggggacgcct    360
atggctttta caataactct gtgaaaacca caggctgggg catcctggag atcagagctg    420
gctatggctc tcaaaccctg agcaatgaga tcatcatgtt tgtggctggc tttttggagg    480
gttacctcac tgccccacac atgaatgacc actacacaaa cctctaccca cagctgatca    540
cgaaaccttc catcatggat aaagtgcagg attttatgga gaagcaagat aagtggaccc    600
ggaaaaatat caaagaatac aagactgatt cattttggag acatacaggc tatgtgatgg    660
cacaaataga tggcctctat gtaggagcaa agaagagggc tatattagaa gggacaaagc    720
caatgaccct gttccagatt cagttcctga atagtgttgg agatctattg gatctgattc    780
cctcactctc tcccacaaaa aacggcagcc taaaggtttt taagagatgg gacatgggac    840
attgctccgc tcttatcaag gttcttcctg gatttgagaa catccttttt gctcactcaa    900
gctggtacac gtatgcagcc atgctcagga tatataaaca ctgggacttc aacgtcatag    960
ataaagatac cagcagtagt cgcctctctt tcagcagtta cccagggttt ttggagtctc   1020
tggatgattt ttacattctt agcagtggat tgatattgct gcagaccaca aacagtgtgt   1080
ttaataaaac cctgctaaag caggtaatac ccgagactct cctgtcctgg caaagagtcc   1140
gtgtggccaa tatgatggca gatagtggca agaggtgggc agacatcttt tcaaaataca   1200
actctggcac ctataacaat caatacatgg ttctggacct gaagaaagta aagctgaacc   1260
acagtcttga caaaggcact ctgtacattg tggagcaaat tcctacatat gtagaatatt   1320
ctgaacaaac tgatgttcta cggaaaggat attggccctc ctacaatgtt cctttccatg   1380
aaaaaatcta caactggagt ggctatccac tgttagttca gaagctgggc ttggactact   1440
cttatgattt agctccacga gccaaaattt tccggcgtga ccaagggaaa gtgactgata   1500
cggcatccat gaaatatatc atgcgataca acaattataa gaaggatcct tacagtagag   1560
gtgacccctg taataccatc tgctgccgtg aggacctgaa ctcacctaac ccaagtcctg   1620
gaggttgtta tgacacaaag gtggcagata tctacctagc atctcagtac acatcctatg   1680
ccataagtgg tcccacagta caaggtggcc tccctgtttt tcgctgggac cgtttcaaca   1740
aaactctaca tcagggcatg ccagaggtct acaactttga ttttattacc atgaaaccaa   1800
ttttgaaact tgatataaaa tgaaggaggg agatgacgga ctagaagact gtaaataaga   1860
taccaaaggc actattttag ctatgttttt cccatcagaa ttatgcaata aaatatatta   1920
atttgtcact ttaaaaaaaa aaaaaaaaaa a                                  1951
```

<210> 85
<211> 1274
<212> DNA
<213> Homo sapiens

<300>
<308> BC009799
<309> 2001-07-05
<313> (1)..(1274)

<400> 85

```
ggggagacgt tcgcacacct gggtgccagc gccccagagg tcccgggaca gcccgaggcg      60
ccgcgcccgc cgccccgagc tccccaagcc ttcgagagcg gcgcacactc ccggtctcca     120
ctcgctcttc caacacccgc tcgttttggc ggcagctcgt gtcccagaga ccgagttgcc     180
ccagagaccg agacgccgcc gctgcgaagg accaatgaga gccccgctgc taccgccggc     240
gccggtggtg ctgtcgctct tgatactcgg ctcaggccat tatgctgctg gattggacct     300
caatgacacc tactctggga agcgtgaacc attttctggg gaccacagtg ctgatggatt     360
tgaggttacc tcaagaagtg agatgtcttc agggagtgag atttcccctg tgagtgaaat     420
gccttctagt agtgaaccgt cctcgggagc cgactatgac tactcagaag agtatgataa     480
cgaaccacaa atacctggct atattgtcga tgattcagtc agagttgaac aggtagttaa     540
gcccccccaa aacaagacgg aaagtgaaaa tacttcagat aaacccaaaa gaaagaaaaa     600
gggaggcaaa aatggaaaaa atagaagaaa cagaaagaag aaaaatccat gtaatgcaga     660
atttcaaaat ttctgcattc acggagaatg caaatatata gagcacctgg aagcagtaac     720
atgcaaatgt cagcaagaat atttcggtga acggtgtggg gaaaagtcca tgaaaactca     780
cagcatgatt gacagtagtt tatcaaaaat tgcattagca gccatagctg cctttatgtc     840
tgctgtgatc ctcacagctg ttgctgttat tacagtccag cttagaagac aatacgtcag     900
gaaatatgaa ggagaagctg aggaacgaaa gaaacttcga caagagaatg gaaatgtaca     960
tgctatagca taactgaaga taaaattaca ggatatcaca ttggagtcac tgccaagtca    1020
tagccataaa tgatgagtcg gtcctctttc cagtggatca taagacaatg gacccttttt    1080
gttatgatgg ttttaaactt tcaattgtca ctttttatgc tatttctgta tataaaggtg    1140
cacgaaggta aaaagtattt tttcaagttg taaataattt atttaatatt taatggaagt    1200
gtatttattt tacagctcat taaacttttt taaccaaaca gatcaaaaaa aaaaaaaaaa    1260
aaaaaaaaaa aaaa                                                      1274
```

<210> 86
<211> 3828
<212> DNA
<213> Homo sapiens

<300>

<308> NM 014325
<309> 2000-04-27
<313> (1)..(3828)

<400> 86

```
cagtgccagg ctggaggcgg cagcggttgg aggcttcgcc cggctttgca gcggggactt      60
cggcggcggc gcctcaggca cctcggcccg gacacgatga ggcgagtggt acgacagagc     120
aagtttcggc atgtatttgg gcaagcggtg aaaaatgacc agtgctatga tgacatccgg     180
gtttctcgtg tgacctggga tagttccttt tgtgctgtca atcccagatt tgttgccata     240
atcatagagg caagtggggg aggagcgttc cttgtcctcc ctctgcacaa gactggtcga     300
attgacaaat cttaccctac agtatgtggc cacacaggac cagtgctgga catagactgg     360
tgcccacata acgatcaggt cattgccagc ggttcagagg actgcacggt catggtatgg     420
cagatcccag aaaatggact caccctttcc ctgactgaac ctgtggtgat tttggaaggc     480
cactcaaaga gagtcggcat cgtggcttgg catccaacgg cccgcaatgt gcttcttagt     540
gcaggctgtg ataatgccat tatcatctgg aatgtgggaa caggggaagc ccttataaac     600
ttggacgata tgcattcaga catgatttac aatgtgagct ggaaccggaa tggcagtctg     660
atctgcacag cttccaaaga caagaaagtg agagtcattg atcccaggaa acaagagatt     720
```

```
gttgctgaga aggagaaagc acatgaagga gcaagaccca tgagagccat cttcctggcc      780
gatggcaatg tcttcaccac tgggttcagc cgcatgagcg agcggcagct ggctctctgg      840
aatccgaaaa atatgcagga accaattgct cttcatgaga tggacactag caatggggtg      900
ttgctgcctt tctatgaccc tgacaccagc atcatttact tatgtggaaa gggtgacagc      960
agtattcgct attttgagat cacggatgaa tccccgtacg tccactacct caacacattc     1020
agcagcaagg agcctcagag agggatgggt tacatgccca agaggggact tgatgttaac     1080
aaatgtgaga ttgccagatt cttcaaactt catgagagaa agtgtgaacc tattattatg     1140
actgttccca ggaagtctga ccttttccaa gatgacctgt atcctgacac agcggggcca     1200
gaggccgcgc tggaggcaga agagtggttc gaaggcaaga atgcagaccc aatcctcatc     1260
tccttgaagc acgggtacat tccaggcaaa aacagggatc tcaaggtggt caagaagaac     1320
attctggata gcaagcccac tgcaaacaag aagtgcgacc tgatcagcat ccccaagaaa     1380
accacagaca cggccagtgt gcaaaatgaa gccaagttgg atgagatttt aaaagagatc     1440
aaatctataa aagacacaat ctgcaatcaa gatgagcgta tttccaagtt agaacagcag     1500
atggcaaaga tagcagcctg aaggtcccac ccccacccct acagaaaaaa tgggagcaag     1560
aacttgtgct tgggagctgg ttattggtgt ggtcctaggg agggcggaaa gggaggcact     1620
gccatttgga gacattccat ttcagatttg tcaaccagcg ataggccaca ttccagtaag     1680
aactcaattt gtctcccaaa tttgcagaaa caaaacgtga tttaaaagct gagcttttta     1740
tcagaaagct tttttgatgt tttaagtgtt atgtgacttg ttgaactttt taaaaagtgc     1800
tacttttaaa atcccagata ctctgaattt tagaaaacaa actaattctg attgtgtcgt     1860
gcccaagtac cctttttttt ttaatgaata gggaccaatg ccacattgct ttttatattt     1920
ctttcttttt taatgttgcc aaaaccaaaa gtagctttgt tttcctttgt attttgctac     1980
tttgcagtat ttgtgtgtgt ggtttttttt ccttaatttg aaagggacag cactgtgtat     2040
gtttataaac taaatgaaga taagatatta ttttgtataa acattcatct gagaacaatc     2100
aaagcagtag ccacatggtg ctggctcctt tgcagcacaa acctggtcat tttgatgact     2160
gtacaacagg aagacttgaa aaatcacgtg gattcatatt accaccgctc tcatttcatg     2220
gagtcttctg atcaaaaaag ctcacgtcgt atttcttctt ttcctttctc ttttctagaa     2280
attgggtgtt tgtaccagaa tggaattttg cttctcggtt atcctgtgct tcagatgatt     2340
ataatctaac ccaaactagc atgtgtttct gcagtttgtt acacacctag gatcatattg     2400
cattcatcac tttaaacatc atgtttcagg ttttggtcaa tacttgacaa gggtgcccag     2460
gacaggaaga cgtgtactgc tgagtgttcc ttcttgccct tttcagcagc ttgcccagct     2520
cttgagtaca gtggtgggga ctaaaaatgt gggcatgtgg agaggggtat ttgccctggg     2580
tgatcctgtt tccctgtgct gtccccatgc tgtgttggag gaggaagtgg ctctccttcc     2640
```

accaacaaag ctcctgctct accctcttcc tcacatgtgc tgcgacctct ctcagggctc 2700
ccccagccat tccttctttc cttcctgcct tttagctcta accacattaa gctaagacaa 2760
ggccagaggg tgcgattgaa tgagtattga gactgaggag aatgatagag agtgaagcag 2820
aaacaggagc acagacctct gctgtagctt taatgcatac aaacatgtcc ctccgcacaa 2880
ctaacctgcc ctgcctctcc atctctcacc aaggctgcgt caaagcacag aggctccccg 2940
gactcggagg gggccagaga ctgagctctg gtcacctgtt cattcctcgg ttagctggaa 3000
ctttgccccg tttccagttt cttatagtgc atgcttggga aacaagattt aaggagcctc 3060
tgttttggaa gggctgtctg tgattgaacg tgaaatgtgt agtgccattg ggaccacgaa 3120
gggaattctt gcacatgctc gtgctggtgt gggcatggga ctggctggaa acgtctgtat 3180
gcagggagcc agggtgaggg cagagtgtgg tgacagccga acttggagta atgtccgtgt 3240
agaaaaagga ccatgttctt atccagccaa tactgggagt gctgtctcca caatttcagg 3300
gcatctgaat gtttgatgtg gttttgtgtg tgtgtatgta tgtgtttaat attgaagtgg 3360
atcatgagat gtaaagaaaa caataatggc aatgacttat attcaaatct gtatttgttt 3420
ctttatcaat gtaatctgct gaggaccttt tgtctaagat tcagtagtgt tttaaggttc 3480
tgatatcgaa ttaatgaagt aaagttgttg atggtggtga aacaccgtag ggcatgtggt 3540
tcaaagagaa gcaggagggc aagggaaagt taccctgatc ttagtttgta gcttatgact 3600
tatttaatga atggatgccc agccaagctc agagtaggcg cccaaagcat tgtggattat 3660
tttcctgttt tgtctttttt tttttttttt tttaagccat gacatcccag aagaggacag 3720
tgaattactc ctaggtcggc tcttatagag tggccatagt gttctgtcaa aacacttgct 3780
tccattttca gagataaaaa tcattgatta caaaaaaaaa aaaaaaaa 3828

<210> 87
<211> 1549
<212> DNA
<213> Homo sapiens

<300>
<308> BC010488
<309> 2001-07-12
<313> (1)..(1549)

<400> 87

ctccgccgca gctcggctcc cgcgcgctca gcaccgccag cggcggccag atgcaggcgg 60
agcgaggagc tcggggaggc cgtgggcggc ggccaggccg cggccggcct ggcggagatc 120
gccacagcga gcggcccgga gccgcagcgg cggtagccag aggcggcggc ggaggcggcg 180
gcggggacgg aggcggacgc cggggccgtg gccgtggccg gggcttccgc ggcgctcgcg 240
gaggccgagg aggaggaggc gcccccgcgag gcagccgccg ggagccggga ggctggggcg 300
caggggccag cgcgccggtt gaagatgaca gcgatgcaga gacctatgga gaagagaatg 360

atgaacaggg aaattattct aaaagaaaga ttgtctctaa ctgggatcga tatcaagata 420
ttgaaaaaga ggtcaataat gaaagtggag agtcacagag gggaacagat ttcagtgtcc 480
tccttagctc tgcaggggac tcattctcac agttccggtt tgctgaggag aaagaatggg 540
atagtgaagc ttcttgtcca aaacagaatt cagcatttta tgtggatagt gagttattgg 600
ttcgagccct tcaagagctg cctctctgcc tccgactcaa cgttgctgcc gaactggtcc 660
agggtacagt tcctttagag gttcctcagg tgaaaccaaa gagaactgat gatggcaagg 720
gattagggat gcagttaaag gggcccttgg ggcctggagg aaggggggccc atctttgagc 780
tgaaatctgt ggctgctggc tgccctgtgt tgctgggcaa agacaaccca agcccgggtc 840
cttcaaggga ttctcagaaa cccacttccc cactgcagtc agcaggagac catttggaag 900
aagaactaga tctgttgctt aatttagatg cacctataaa agagggagat aacatcttac 960
cagatcagac gtctcaggac ctgaaatcca aggaagatgg ggaggtggtc caagaggaag 1020
aagtttgtgc aaaaccatct gtgactgaag aaaaaaacat ggaacctgag caaccaagta 1080
cctccaaaaa tgttaccgag gaagagctgg aagactggtt ggacagcatg atttcctaaa 1140
aaggggaaaa aaagtgcctg aagcaaatct tggttgcctt ctaacggcag gtgggcataa 1200
ggctgtcctt caggaccagc cagtttacaa gcatgtctca agctagtgtg ttccattatg 1260
ctcacagcag taaatgccta cctctgtgtt tgacatctga aagaatacat tgaagcagct 1320
tgttgcattt gtttttctgg cttagtaatc taatagattt ccttaagggc aggagataga 1380
ctctggccct tgtttctagc ctccttcctt gcagtgttta caacatagcc agtgtttaca 1440
gcatagcaga tgctgctgct gattaagaga atagatgcaa acaaggcatg catttggcca 1500
aaataaacaa atgctggtct gtcaaaaaaa aaaaaaaaaa aaaaaaaaa 1549

<210> 88
<211> 4139
<212> DNA
<213> Homo sapiens

<300>
<308> NM_004090
<309> 1999-05-07
<313> (1)..(4139)

<400> 88

gcgagccccc gcccagctga tccgccgctc tccgcctcgc ttgctcctgc cgggcgtgca 60
gggccccgcc gccgccatgt cgggctcgtt cgagctctcg gtgcaggatc tcaacgacct 120
gctctcggac ggcagcggct gctacagcct cccgagccag ccctgcaacg aggtcacccc 180
gcggatctac gtgggcaacg cgtctgtggc tcaggacatc cccaagctgc agaaactagg 240
catcacccat gtgctgaacg cggctgaggg caggtccttc atgcacgtca acaccaatgc 300
caacttctac aaggactccg gcatcacata cctgggcatc aaggccaacg acacacagga 360

```
gttcaacctc agcgcttact ttgaaagggc tgccgacttc attgaccagg ctttggctca     420
aaagaatggc cgggtgctcg tccactgccg ggaaggttat agccgctccc caacgctagt     480
tatcgcctac ctcatgatgc ggcagaagat ggacgtcaag tctgccctga gcatcgtgag     540
gcagaaccgt gagatcggcc ccaacgatgg cttcctggcc cagctctgcc agctcaatga     600
cagactagcc aaggagggga agttgaaacc ctagggcacc cccaccacct ctgctcgaga     660
ggtccgtggg ggaggccgtg ggcaaaggtg tcccgagctg ccatgtttag gaaacacact     720
gtaccctgct cccagcatca caaggcactt gtctacaagt gtgtcccaac acagtcctgg     780
gccactttcc ccaccctggg gagcacataa agaagcttgc caaggggggc gtccttgctc     840
cccagttgtc ctgtttctgt aacttatgat gtcttttccc tgagatgggg gctcagaggg     900
ggaaggcctg tggcctgcat gcttcccgat ggcccacggc aggaggtgtg tggaagtgta     960
aggcctaaga tgctcacaga ggtccctcat gacctcccct ccccaactcc cgaatcctct    1020
cttgagtgtg gacctcaaca ccttgagccc tagtaaagga actatgcaaa tgcaggccac    1080
tctccccacc acgtctgtgc cctgcactgt ccccacagcc ttccacaccc tgtgcatagg    1140
cagccctctc acgtcttgag gtccgaagct ggggtggggg tgtccgtcag ttattagtgg    1200
atggagattc ccacagcaag gctgcatttg aatgatttcc ttaggatgaa tggtccctac    1260
acaaagaggc cttgtgggca aacctggaga accctcctaa atccatagag ttttcaaaat    1320
gtgaatcttt ggaagccttg agttcagaat ctgctgctct ggaatatttc ccttcgatct    1380
tatctcagtc acttcgtttt tgagaagagt gatgccttgg gcatgctttt tttttttct     1440
tttttagaaa acagggagtt gaagtccaac ctatttaaaa accccaccat ttggagaatt    1500
acaagggttt tgtcctgaat tgtagtgttg gcaagcccaa gccactcgtg ctaactgctt    1560
tttgtctcgg ttgctattcc aagaacagaa ggaggaagtt ggccaattac agcgtgtgtg    1620
catggatgtg tgtgggggc gtgcctctca gaaacgcggc cagaagacaa gcagggaagt     1680
gaaaggtccc aggcacacac cctgcccatt gcaggtggct cttacagctc tctggtgcca    1740
gcacgggatc cctgaagtga ctcagccagg cagacatgag acatggcgga gtgtccaaat    1800
ggatccttta ttggtggtag agcaaaaaaa cccaaacacg ataaaccttt caaaagactt    1860
tctaaggatg atattggaat gcaccagccc tcacatgtgt atgcacattt gccagaatat    1920
aagagttttg ttttaaatac agtcttgtta ggattttacg ttattgttat tatggaaagt    1980
gattgtgatg ctatttatct tcagggtcac tctgggcaaa gagaaggtcc tcagccatgc    2040
ccccagcacc ttgcacatag gtgtctgata aaagtttaag aaagtaaaca ctttttgagc    2100
accaaatata tatagggcat tgttctggtg ggtgtgtcac gctcccagaa gactgaattc    2160
atggtaggat cactcgcaag gccttgtgaa ggagtcttac ctaaaacgaa agaaatatca    2220
gggacttttg ttgactattt acaactcagt tttacattta aattcaggca gtgttaatat    2280
```

gccaaggtag ggaatgtgcc tttttcagag ttggccagga gctcctggct gggacacgga 2340
gaggcaggtg tggcgtaagg cctcactccc ggctgggaag gtctctgatc acacagaagc 2400
agccctgccc agcctggtca tttgctgtcc gcttttctct gtgaccacag cagccctgaa 2460
caaccagtat gtgtcttctt ctccagatag tgaaaaaggt gtccagataa acccacctaa 2520
gtgaaatggc catcctctaa actgggtacc tcactgcaca gcttctaggt agccttccaa 2580
cttaatctaa cttgagcctc acagtaaccc tgtaaagtta gtagagcttg ttcttgtatt 2640
gtgacctttt ttaaaaaaaa ggaactgagg ttcagaatga ttaagggcct ggcccccagg 2700
gttgtccagc tccataaggt ggagctgggc aagattttgg gtttgctgct ccctgaagct 2760
ggattctttc atacgatact ctttctcaag aagggggctc cctgggatct ccaggtgtac 2820
tgcacttacc ctcaatccag ccccggagaa gcaagtgaaa agggtgggtc cctcataggc 2880
tagaatgtgc agctctttct ccaggtggga tgtagcaccc cgaagtagag ctttctgctc 2940
tgctcctgga aaaggctagg gagctggggc tggggctccc ctcccatgac caggcagtgg 3000
tcaccccatg ggacaggcac agctacttac gcgaacacag caggttggtg tggctggcta 3060
actaggacct ctcgaaagtc tctgtggggg catgagggag aaaaggccat tgggagaatt 3120
actgccttta ctttgggact acttttatgc tgataacttg ggatttcttg atagtccttc 3180
acccctgaaa ccccgtattt acttaacaag atttagctct tagttcttca agtaaaatta 3240
aagtctcttg tgtaagagcc aacacatgcc cagctgcgga tgggagctgt tcctggacag 3300
ccttctactg cctgggaagt gatggaacag gaactcaggg tgcccttacc ccctccccag 3360
acctgttccc tttctttgac tgacagagca ccatccaggc aaaattagag cgccaaatgg 3420
ttttcttctc aatcttaaag cagtatacct ttccacaggc tcgtctgtgt ccctgccact 3480
ctgagttatc cagaaaccac cacctacaaa tgaggggact catctagaag acctctaagg 3540
tccccttttg gctctgaggg gtctctaata atccccactt ggaattcagc accgcaagga 3600
aattatgggt atgtgagcca taatatgatg ggcagcaggt ggcgctgcct tccacccatg 3660
gtgatggatg gtttggaaag ggaatgttgg tgccttttgt gccacaagtt aagatgctac 3720
tgttttaaag gaaaaaaaaa aaaaaaagta ctgatcttca atatgaagac atgagctttt 3780
ctcgcaggaa attttctttt tcacagaact ggtgtcagga atcactgaag ggctaaccgt 3840
gatagtcctt gcaagtaagt caaggtttta tcctgattgg aaatagaaga catttccggt 3900
tgagagaaca gattcgttgg aagcttaact tttgttgcct cttaacgcca ccaaatttta 3960
gggtaatttg attatgaaag agtgaatttt tctggacaga aaagggagag ctaccaaatt 4020
gtttttttct ttttaaaagg aagtttaatg tccgttgtat cacaaatcag tgttaaaaca 4080
ccagaacttt agccaaaata aatgtcttac attacaaagg taaaaaaaaa aaaaaaaaa 4139

<210> 89
<211> 521
<212> DNA

<213> Homo sapiens

<300>
<308> AI026836
<309> 1998-05-20
<313> (1)..(521)

<400> 89

```
tttttttttt tttttcaaaa taatatgaac agtgcactaa gcttggccta ttgccaaaca        60
caatctctct cttccttgga actaattaaa tgagaaagga tataggaaag tcatggaaac       120
aaaagtatag gaactgccaa attgtactag cttcataaag tatggtgcct gcagtcttag       180
atatatttat gtttatgttt aaatacagat atatttaaaa ggctacagca acatctaccc       240
gctccttaca gcctggtcca gtgaggggta ttccctcctc tcaaagtcca cctccaggct       300
tcttgttcta cttcaggtac ctgattctct aggtgcagtt ttgaagtagt tgaccatgtt       360
tgaagtggta gatgattttt ctagtaatta ttcttagctg cctgattatt ttaaagttaa       420
taatgtgtaa gacctcaaaa gggctttaaa aacaacccaa aactacctgg ttgaaacgac       480
tgctcaaaaa ataaactttc agttaaatgt tcctttaatc t                           521
```

<210> 90
<211> 932
<212> DNA
<213> Homo sapiens

<220>
<221> misc_feature
<222> (816)..(816)
<223> n is a, c, g, or t

<300>
<308> BU500509
<309> 2002-09-12
<313> (1)..(932)

<400> 90

aagaagcgaa gcgtaccgcc cgcattgccg ctcccagtcc cgcgctcggc acgacatgaa 60
atccccgac gaggtgctac gcgagggcga gttggagaag cgcagcgaca gcctcttcca 120
gctatggaag aagaagcgcg gggtgctcac ctccgaccgc ctgagcctgt tccccgccag 180
cccccgcgcg cgccccaagg agctgcgctt ccactccatc ctcaaggtgg actgcgtgga 240
gcgcacgggc aagtacgtgt acttcaccat cgtcaccacc gaccacaagg agatcgactt 300
ccgctgcgcg ggcgagagct gctggaacgc ggccatcgcg ctggcgctca tcgatttcca 360
gaaccgccgc gccctgcagg actttcgcag ccgccaggaa cgcaccgcac ccgccgcacc 420
cgccgaggac gccgtggctg ccgcggccgc cgcaccctcc gagccctcgg agccctccag 480
gccatccccg cagcccaaac cccgcacgcc atgagcccgc cgcgggccat acgctggacg 540

agtcggaccg aggctaggac gtggccggcg ctctccagcc ctgcagcaga agaacttccc 600
gtgcgcgcgg atcctcgctc cgttgcacgg gcgccttaag ttattggact atctaatatc 660
tatgtattta tttcgctggt tctttgtagt cacatatttt atagtcttaa tatcttgttt 720
ttgcatcact gtgcccattg caaataaatc acttggccag tttgcttttc taccatccgg 780
ctgtggctca gtgagactcc tgctgggagg gtggangccc aggaatgggc gggcaggaca 840
ccctcatcca gtcctgcggg ggctggtgtg aaaggcgctg gggaccgggc tttggatgaa 900
taaatgaatc gtgtcatctg gaaaaaaaaa aa 932

<210> 91
<211> 2025
<212> DNA
<213> Homo sapiens

<300>
<308> NM_016090
<309> 2000-09-08
<313> (1)..(2025)

<400> 91

```
cgacgctgag atgggggcgg cggcggcgga agcggatcgc actctctttg tgggcaacct      60
tgaaacgaaa gtgaccgagg agctcctttt cgagcttttc caccaggctg ggccagtaat     120
aaaggtgaaa attccaaaag ataaggatgg taaaccaaag cagtttgcgt ttgtgaattt     180
caaacatgaa gtgtctgttc cttatgcaat gaatctactt aatggaatca aactttatgg     240
aaggcctatc aaaattcaat ttagatcagg aagtagtcat gccccacaag atgtcagttt     300
gtcatatccc caacatcatg ttggaaattc aagccctacc tccacatctc ctagcaggta     360
cgaaaggact atggataaca tgacttcatc agcacagata attcagagat ctttctcttc     420
tccagaaaat tttcagagac aagcagtgat gaacagtgct ttgagacaaa tgtcatatgg     480
tggaaaattt ggttcttcac ctctggatca atcaggattt tcaccatcag ttcaatcaca     540
cagtcatagt ttcaatcagt cttcaagctc ccagtggcgc caaggtacac catcatcaca     600
gcgtaaagtc agaatgaatt cttatcccta cctagcagat agacattata gccgggaaca     660
gcgttacact gatcatgggt ctgaccatca ttacagagga aagagagatg atttcttcta     720
tgaagacagg aatcatgatg actggagcca tgactatgat aacagaagag acagtagtag     780
agatggaaaa tggcgctcat ctcgacacta acacatgtta aaaggacatt gtttttatag     840
ggtcatttta ggccctttga ctaagttgat atggaaatat tttgttgaaa aactgtacag     900
agcagcttta caagttgtca catttcttta taaatttttt taaagctaca gtttaataca     960
aaatgaattg cggttttatt acattaataa cctttcacct cagggtttta tgaagaggaa    1020
agggttttat gcaaaagaaa gtgctacaat tcctaatcat tttagacact ttaggagggg    1080
gtgaagttgt atgataaagc agatatttta attatttgtt atctttttgt attgcaagaa    1140
```

```
atttcttgct agtgaatcaa gaaaacatcc aggttgacag tctaaaatgg ctactggtat      1200
tttagttaat tcaaaaatga aacttttcag tgattcactt tactaacatt ctatttgaga      1260
aggcttattg gtaaagtttg gggataaagg cattgcttaa cttcttatat aatttaggta      1320
taaattctgt gacatgctct tgagctttac cctagttgaa catacatgtg tagatttaca      1380
catactgttt cattctaaaa tttagaaatt gttcattaaa tcccatttga ggtataagtc      1440
actcaggaag ttaaaatatc tctacacgta tatttttaca ttaaaaatac agtgttagca      1500
taaatcccct tttcaggaag aacaaaaatg tcagtgcata gttagataaa atggtaaaat      1560
gttttactga aagcatactt ttttggaaaa tagattcatg aagcctttaa gtgctgcttc      1620
tgtcagtcaa acgttaaaaa ctttaacatt ttcaaagtgc ccagactgtg tacaaagaca      1680
catgtaatgg agattgtaca ggttgttttt ttgtttgaac ctttgaaaga gtttaatctt      1740
aacgttttct aattttaaaa ttttaaaatc ttgtttaaca aaagcttgta ttaagatact      1800
gttttcattt cattacagaa ttgtttataa aagttcattt gttgaaaaat aaggatcctt      1860
tttaatacca cagcatttgt actgttcctt tttaatatac tgaaaatata aaaggaaaaa      1920
aaaaaaaaaa aaaaaaaaaa aaaaaaaaaa aaaaaaaaaa aaaaaaaaaa aaaaaaaaaa      1980
aaaaaaaaaa aaaaaaaaaa aaaaaaaaaa aaaaaaaaaa aaaaa                      2025
```

<210> 92
<211> 513
<212> DNA
<213> Homo sapiens

<300>
<308> BX092512
<309> 2003-01-22
<313> (1)..(513)

<400> 92

```
agagcaccca agccagaacc tctctgagat gtagggcctg gtgttccagc cacacacttt        60
ctgtctgtgt aactcgggga caggacgtcc cacccttttct tagtccgtaa cgcacctttg      120
gacatgatgg aatatacgaa ggggcctcca cccacagcat gaccaatccc cagcatccaa      180
aatcgcacac ataccaatat gtgcacacac acactcacac ccacacccat aaagattttg      240
cactccttga aggtacacta actcaccatt tttatcatac ttatcccagt gtgccacagt      300
tactggctta tatgcctgtc tctgctatct tattttatct gtctccacaa cacagcaaac      360
tacctggcct tcaataaagg gcttatgaat tattcatgaa tccattttgc caggtgccta      420
gccctgtgtc tggcttgaag caggtgttcc caaggtgtgg catggctgag tgaatacaaa      480
taaaaggtat acaaaagcaa aaaaaaaaaa aaa                                   513
```

<210> 93
<211> 621

<212> DNA
<213> Homo sapiens

<220>
<221> misc_feature
<222> (520)..(520)
<223> n is a, c, g, or t

<300>
<308> AI936027
<309> 1999-02-12
<313> (1)..(621)

<400> 93

```
tatgggcaaa tattagcaat ttaatattat ataaacataa ttactgaatt attacataca        60
gcttgatgtc ctttctatgc attaaaacca aacaaaataa aggcattttt accatcacat       120
aagaaatgag aatacacacg cacacaattt aaagtgataa ttatcttggc ggaaggagcg       180
aaggtggggg cgttacagga tgagatatga cagaagttat tgttagagtc ctagttttga       240
agttgggtag taagctcatt ggtgctcgtg acattattaa aagaaacaaa taatagggga       300
tgcgtgcatc catgaggaga aacgtggcat gaacaatagg tataaattct aactctcacc       360
tccgctgtga atgaggcaaa gatctacagc acatttactt gatcacacag tctgtgaaac       420
aaagacacgt tagtggccac ggtgggctgt gacaactcta gtctcacctc gatgagtcag       480
gttaaaaagg atattgacac ttctctttct taagtaattn tcagctatga gtgtgaagtt       540
ataggtttct tgtgagtctt gagttatttg ccaattacac cagtttttgt gtgtacaaag       600
tttctttttc ccagaaaatc c                                                 621
```

<210> 94
<211> 619
<212> DNA
<213> Homo sapiens

<220>
<221> misc feature
<222> (525)..(525)
<223> n is a, c, g, or t

<300>
<308> AI971137
<309> 1999-08-25
<313> (1)..(619)

<400> 94

```
tttttctga atgaaagcta attccattct tttatttcct cataccacaa acatttcaat 60
ttatctgcct ttttacaacc tgatttacac aagcaaattg caaacgaaaa ccaggcattc 120
tttaatcatc caaaatgcat gtataaaata tagaacaaac cctagtattt aaacataaac 180
agggttagct gaagcagctt tattgcaatc tcttcaagtt agcatattac agtttaaata 240
tttatgcctg taaagatctg cataatctac aatacagagt tatttcagaa gcagttgact 300
taactagttg agaaaaaaaa acaacaaact tcaacgcaaa gctataataa ttatctgaaa 360
cttatttaca attaaacatt tagggtcctg atttacaaaa ctcagtgcct ttcatgattt 420
attgatgagt tttatagaga aagtaagcag tatgtagaat attccccagg taaaatctgg 480
agtgaatggc ttttagagga aagtctatca gtatgcttaa ctaanaacta aagagtgaca 540
aaactgtata cagcagcaat cagaataaca ggccacaaga gaagaacgcc atttttgaca 600
atatcctttg tatgtatag 619
```

<210> 95
<211> 697
<212> DNA
<213> Homo sapiens

<220>
<221> misc_feature
<222> (665)..(665)
<223> n is a, c, g, or t

<300>
<308> BQ024877
<309> 2002-03-27
<313> (1)..(697)

<400> 95

```
tttttttttt ttttttttaa acatcacaat ataaaatgtt aactggtgat gtagtaaacc     60
aacatgataa tttgtcttat ttgccttact agcattacca ccatataggc acttttccac    120
gtagtcaaat tatgaacagc atttttctat aaggcatatt ccatgcacat catttacaga    180
aaggtaaatt aaaaaataga aaagattatg gagcatgaaa tccgcgtgca tgtaatgtaa    240
gcatatgttt tatatgtatt tttctctctg tacagaataa taaatatcac tatatctgat    300
gcaagtaatt cacttccagg attgtgcata ttaagcgatc agactaatcc catcattaaa    360
ttattatctt gcacttatca ttaagactcc attaccatgt atgtgatata tacaggacat    420
gttccatgta acatgatttt ccgctttaca aaatgggtct gatttctgtg tttttacaga    480
gctgaacaag tcagcaaagc atctacaaaa tgcctctgat ttgtataaat acactggtgt    540
gtaaacacaa tgaagatttt gaagtgggca gtcatttcaa cttcagtgag tactattcct    600
tgcctcatct ccatgcttca atgttgttta caggacttaa actgaaaaag tattttacat    660
tttangttct tacagcaaca aagaaaagca ttcagtt                              697
```

## Description

Field

The present description relates to a method of personalized cancer therapy which employs a set of marker genes to predict whether a patient will respond to a chemotherapeutic agent and a kit for use in said method.

In particular, the method predicts patient response to erbB tyrosine kinase inhibitors. More particularly the method relates to those patients with cancers mediated alone or in part by erbB tyrosine kinase, especially patients with advanced Non-small Cell Lung Cancer (NSCLC), for example adenocarcinoma, using the levels of a set of marker genes having differential expression between responders and non responders to the erbB tyrosine kinase inhibitor.

Background to the Invention

Lung cancer is the leading cause of cancer death and is therefore a major health problem worldwide. In the treatment of this disease, chemotherapy is the mainstay, because the majority has locally advanced stage 3 (44%) or metastatic stage 4 (32%) disease at diagnosis [1]. Nevertheless, the findings of large meta-analysis revealed that platinum-based chemotherapy contributed to prolong the median survival time of patients with advanced non-small cell lung cancer (NSCLC) by only about 6 weeks compared with best supportive care [2].

In the last decade, many new cytotoxic agents have been developed including paclitaxel, docetaxel, gemcitabine, and vinorelbine, and have offered multiple choices for patients with advanced lung cancer. However, each regimen served only modest survival benefit compared with the cisplatin-based therapies [3], [4]. More recently, new therapeutic strategies including a number of molecular-targeted agents have been developed in an effort to overcome the limitations of conventional cytotoxic agents [5] [6].

In recent years it has been discovered that certain growth factor tyrosine kinase enzymes are important in the transmission of biochemical signals which initiate cell replication. They are large proteins which span the cell membrane and possess an extracellular binding domain for growth factors such as epidermal growth factor (EGF) and an intracellular portion which functions as a kinase to phosphorylate tyrosine amino acids in proteins and hence to influence cell proliferation.

Various classes of receptor tyrosine kinases are known (Wilks, Advances in Cancer Research, 1993, 60, 43-73) based on families of growth factors which bind to different receptor tyrosine kinases. The classification includes Class I receptor tyrosine kinases comprising the EGF family of receptor tyrosine kinases. This includes receptors for the ligands EGF, TGF$\alpha$ (also referred to as TGFA), amphiregulin (also referred to as AREG), betacellulin, heparin binding EGF, epiregulin and the neuregulins (including NRG-1, NRG-2, NRG-3 and NRG-4) . More specifically, these receptors include those with a functional kinase domain called erbB1 (EGFR), erbB2 (Neu, Her2) and erbB4 (Her 4), and erbB3 (her3), which does not), Class II receptor tyrosine kinases comprising the insulin family of receptor tyrosine kinases such as the insulin and IGFI receptors and insulin-related receptor (IRR) and Class III receptor tyrosine kinases comprising the platelet-derived growth factor (PDGF) family of receptor tyrosine kinases such as the PDGF$\alpha$, PDGF$\beta$ and colony-stimulating factor 1 (CSF1) receptors.

It is known that the erbB family of receptor tyrosine kinases, which include EGFR, erbB2, erbB3 and erbB4, are frequently involved in driving the proliferation and survival of tumour cells (reviewed in Olayioye et al., EMBO J., 2000, 19 3159). One mechanism by which this can occur is over expression of the receptor at the protein level, generally as a result of gene amplification. This has been observed in many common human cancers (reviewed in Klapper et al., Adv. Cancer Res., 2000, 77, 25) such as, non-small cell lung cancers (NSCLCs) including adenocarcinomas (Cerny et al., Brit. J. Cancer, 1986, 54, 265; Reubi et al., Int. J. Cancer 1990, 45 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7, 347. As a consequence of the mis-regulation of one or more of these receptors, it is widely believed that many tumours become clinically more aggressive and so correlate with a poorer prognosis for the patient (Brabender et al, Clin. Cancer Res., 2001, 7, 1850; Ross et al, Cancer Investigation, 2001, 19 554, Yu et al., Bioessays, 2000, 22.7, 673). In addition to these clinical findings, a wealth of pre-clinical information suggests that the erbB family of receptor tyrosine kinases are involved in cellular transformation. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550).

Thus it has been recognised that inhibitors of these receptor tyrosine kinases should be of value as a selective inhibitor of the proliferation of mammalian cancer cells (Yaish et al. Science, 1988, 242, 933, Kolibaba et al, Biochimica et Biophysica Acta, 1997, 133, F217-F248; Al-Obeidi et al, 2000, Oncogene, 19, 5690-5701; Mendelsohn et al, 2000, Oncogene, 19, 6550-6565). In addition to this pre-clinical data, findings using inhibitory antibodies against EGFR and erbB2 (c-225 and trastuzumab respectively) have proven to be beneficial in the clinic for the treatment of selected solid tumours (reviewed in Mendelsohn et al, 2000, Oncogene, 19, 6550-6565).

A number of small molecule inhibitors of erbB family of receptor tyrosine kinases are known, particularly inhibitors of EGF and erbB2 receptor tyrosine kinases. For example European Patent Application No. 0566226 and International Patent Applications WO 96/33980 and WO 97/30034 disclose that certain quinazoline derivatives which possess an anilino substituent at the 4-position possess EGFR tyrosine kinase inhibitory activity and are inhibitors of the proliferation of cancer tissue including prostate cancer. It has been disclosed by J R Woodbum et al. in Proc. Amer. Assoc. Cancer Research. 1997, 38, 633 and Pharmacol. Ther., 1999, 82, 241-250 that the compound N-(3-chloro-4-fluorophenyl)-7-methoxy-6-(3-morpholinopropoxy)quinazolin-4-amine is a potent EGFR tyrosine kinase inhibitor. This compound is also known as Iressa (registered trade mark), gefitinib (United States Adopted Name), by way of the code number ZD1839 and Chemical Abstracts Registry Number 184475-35-2. The compound is identified hereinafter as gefitinib. Gefitinib has recently been approved in Japan for the treatment of inoperable or recurrent non-small cell lung cancer (NSCLC) and in the USA as a monotherapy for the treatment of patients with locally advanced metastatic NSCLC after failure of both platinum and docetaxel chemotherapies.

It is further known from International Patent Application WO 96/30347 that certain structurally-related quinazoline derivatives possessing an anilino substituent at the 4-position also possess EGFR tyrosine kinase inhibitory activity. It has been disclosed in WO 99/55683 that the compound N-(3-ethynylphenyl)-6,7-bis(2-methoxyethoxy)quinazolin-4-amine, or a pharmaceutically-acceptable salt thereof (linked to the code numbers CP 358774 and OSI-774, identified hereinafter by the code number OSI-774) is an EGFR TKI.

It is further known from International Patent Application WO 97/38983 that certain other structurally-related quinazoline derivatives possessing an anilino substituent at the 4-position also possess EGFR tyrosine kinase inhibitory activity. It has been disclosed in J.Med. Chem., 1999, 42, 1803-1815 and WO 00/31048 that the compound 6-acrylamido-N-(3-chloro-4-fluorophenyl)-7-(3 morpholinopropoxy) quinazolin-4 - amine (linked to the code numbers PD 183805 and CI 1033, identified hereinafter by the code number CI 1033) is an EGFR TKI.

It is further known from International Patent Application WO 97/02266 that certain other structurally-related heterocyclic derivatives also possess EGFR tyrosine kinase inhibitory activity. For example, the compound 4-[(1R)-1-phenylethylamino]-6-(4-hydroxyphenyl)-7H-pyrrolo[2,3-d]pyrimidine (linked to the code numbers PKI-166, CGP 75166 and CGP 59326, identified hereinafter by the code number PKI-166) is an EGFR TKI.

It is further known from European Patent Application No. 0787722 and International Patent Applications WO 98/50038, WO 99/09016 and WO 99/24037 that certain other structurally-related quinazoline derivatives possessing an anilino substituent at the 4-position also possess EGFR tyrosine kinase inhibitory activity. For example, the compound N-[4-(3-bromoanilino)quinazolin-6-yl]but-2-ynamide (linked to the code numbers CL-387785 and EKB-785, identified hereinafter by the code number CL-387785) is an EGFR TKI.

It is further known from Nature Medicine, 2000, 6, 1024-1028 and United States Patent No. 6,002,008 that certain other structurally-related quinoline derivatives possessing an anilino substituent at the 4-position also possess EGFR tyrosine kinase inhibitory activity. For example, the compound 4-(3-chloro-4-fluoroanilino)-3-cyano-6-(4-dimethylaminobut-2(E)-enamido)-7-ethoxyquinoline (identified hereinafter by the code number EKB-569) is an EGFR TKI. It is also known from WO 99/35146 and WO 01/04111 that certain other quinazoline derivatives are inhibitors of one or more of the erbB receptor tyrosine kinase inhibitors. For example the compound N-{3-chloro-4-[(3-fluorobenzyl)oxy]phenyl}-6-[5-({[2-(methylsulfonyl)ethyl]amino}methyl)-2-furyl]quinazolin-4-amine (also identified as lapatinib or GW2016 identified hereinafter by the code GW2016) is thought to be an inhibitor of both EGF and erbB2 receptor tyrosine kinases.
Novartis AE788 is another suitable inhibitor compound.
Inhibition of erbB receptor tyrosine kinase may also be achieved by inhibition of the extracellular ligand binding to a receptor using suitable antibodies against an erbB receptor. For example using the anti-erbB2 antibody trastuzumab [Herceptin™] and the anti-erbb1 antibody cetuximab [C225]). The use of such inhibitory antibodies have proven to be beneficial in the clinic for the treatment of selected solid tumours (reviewed in Mendelsohn et al, 2000, Oncogene, 19, 6550-6565).

As mentioned above, gefitinib is an oral active inhibitor of epidermal growth factor receptor-tyrosine kinase (EGFR-TK), which blocks signalling pathways responsible for driving proliferation, invasion, and survival of cancer cells [7]. Potent anti-tumour effects as well as rapid improvements in NSCLC-related symptoms and quality of life have been observed in clinical studies that enrolled patients with advanced NSCLC who did not respond to platinum-based chemotherapy. In the randomized double-blind phase II monotherapy trial (the IDEAL 1 trial), use of gefitinib as 2nd or 3rd line of chemotherapy to advanced NSCLC achieved tumour response rate of 18.4% (95%CI: 11.0-25.9%), and in the IDEAL 2 trial, use as 3rd or 4th line of chemotherapy achieved that of 11.8% (95%CI: 6.2-19.7%) [8],[27],[28].

Moreover in these trials, the treatment of this drug achieved high disease control rate (54.4% in IDEAL 1, 42.2% in IDEAL 2) and overall symptom improvement rate (40.3% in IDEAL 1, 43.1 % in IDEAL 2).

Those results were promising when compared with responses to conventional cytotoxic agents, but the fact remained that about half of the patients enrolled in these studies received non-effective treatment with no improvement in symptoms. Moreover, the medication exposed non-responders to adverse effects, including life threatening ones such as interstitial pneumonia [11].

Patients responses to the various chemotherapy treatments differ, therefore there is a need to find methods of predicting which treatment regimes best suit a particular patient.

There is an increasing body of evidence that suggests that patients responses to numerous drugs may be related to a patients genetic profile and that determination of the genetic factors that influence, for example, response to a particular drug could be used to provide a patient with a personalised treatment regime. Such personalised treatment regimes offer the potential to maximise therapeutic benefit to the patient, whilst minimising, for example side effects that may be associated with alternative and less effective treatment regimes. There is therefore a need for methods that can predict a patients response to a drug.

Summary of the Invention

It has been found that the sensitivity of certain cancers to chemotherapeutic agents can be predicted by gene expression and hence that the suitability of cancer patients for treatment with such chemotherapeutic agents can be determined by measuring the relative levels of particular genes in patient tissue.

The present invention provides an *in vitro* method of predicting the responsiveness of a patient with cancer to the treatment with an erbB receptor kinase inhibitor, or for selecting a patient that will respond to an erbB receptor kinase inhibitor, comprising comparing the differential expression of the AREG gene.

Accordingly, the present description provides an isolated set of marker genes comprising at least one gene identified as having differential expression as between patients who are responders and non responders to an erbB receptor tyrosine kinase inhibitor, said gene set comprising one or more genes selected from at least the group consisting of the 51 genes listed in Table 4 herein including gene-specific oligonucleotides derived from said genes. In Table 4, accession numbers are given for the genes on the GenBank database. As will be appreciated by those skilled in the art, sequences available at the given accession numbers represent only examples of sequences of the genes referred to in the table; alternative sequences, including sequences which comprise sequencing error corrections, allelic or other variations, splice mutants and the like are also included in the definition of the gene represented by the name usesd. In a most preferred ebodiment, the sequences referred to are the sequences set forth at the accession numbers and specific sequences given and set out in detail in Table 4a.

In a further aspect the present description provides a set of isolated marker genes comprising at least one gene identified as having differential expression as between patients who are responders and non responders to an erbB receptor tyrosine kinase inhibitor; said gene set selected from the group consisting of the 51 genes listed in Table 4 herein including gene-specific oligonucleotides derived from said genes.

The present invention permits the improved prognosis and hence quality of life of cancer patients by matching the treatments to individual patients and so making more effective use of the types of drug available.

A preferred set is at least one or more of the first 40 genes listed in Table 4 herein.

A further preferred set is at least one or more of the first 20 genes listed in Table 4 herein.

A further preferred set is at least one or more of the first 12 genes listed in Table 4 herein.

A preferred set is at least one or more of the first 5 genes listed in Table 4 herein.

An especially preferred set is the first 12 genes listed in Table 4a herein, namely FLJ22662 (e.g. GenBank NM_024829), AREG (e.g. GenBank BC009799), CORO1C (e.g. GenBank NM_014325), AVEN (e.g. GenBank BC010488), DUSP3 (e.g. GenBank NM_004090, DJ473B4 (e.g. GenBank AI026836), PHLDA2 (e.g. GenBank BU500509), RBM7 (e.g. GenBank NM_016090), EST (GenBank BX092512), OSMR (e.g. GenBank AI436027), GCLC (e.g. GenBank AI971137), COL4A3BP (e.g. GenBank BQ024877).

Preferably the inhibitor is selected from gefitinib, OSI-774, PKI-166, EKB-569, GW2016, CI-1033 and an anti-erbB antibody such as trastuzumab and cetuximab.

Most preferably the inhibitor is gefitinib.

The present invention is particularly suitable for use in predicting the response to the aforementioned chemotherapeutic agents in those patients or patient population with a cancer mediated alone , or in part, by an erbB tyrosine kinase. Such cancers include, for example, non-solid tumours such as leukaemia, multiple myeloma or lymphoma, and also solid tumours, for example bile duct, bone, bladder, brain/CNS, breast, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine and vulval tumours.

The present invention is particularly suitable for identifying those patients with NSCLC, more particularly advanced NSCLC including advanced adenocarcinoma that will respond to treatment with chemotherapeutic agents such as an erbB receptor tyrosine kinase inhibitor as hereinbefore defined.

The present invention offers considerable advantages in the treatment of cancers such as NSCLC, especially advanced NSCLC by identifying "individual cancer profiles" of NSCLC and so determining which tumours would respond to gefitinib. This includes 1st line treatment and any other treatment regimen, such as, for example chemotherapy failed patients.

The present invention is particularly useful in the treatment of patients with advanced NSCLC who have failed previous chemotherapy, such as platinum-based chemotherapy.

The present invention is also particularly useful in the treatment of patients with locally advanced (stage IIIB) or metastasized (stage IV) NSCLC who have received previous chemotherapy, such as platinum-based chemotherapy.

The present description also provides a method of predicting the responsiveness of a patient or patient population with cancer-, for example lung cancer, to treatment with chemotherapeutic agents, especially erbB receptor tyrosine kinase inhibitors, comprising comparing the differential expression of a set of marker genes said marker genes selected from the gene sets as defined above.

Preferably the assessment of expression is performed by gene expression profiling using oligonucleotide-based arrays or cDNA-based arrays of any type; RT-PCR (reverse transcription- Polymerase Chain Reaction), real-time PCR, *in-situ* hybridisation, Northern blotting, Serial analysis of gene expression (SAGE) for example as described by Velculescu et al Science 270 (5235): 484-487, or differential display. Details of these and other methods can be found for example in Sambrook et al, 1989, Molecular Cloning: A Laboratory Manual). Preferably the assessment uses a microarray assay.

Alternatively, or in addition, the assessment uses an immunohistochemical assay.

In a further aspect, the present description provides a kit for use in a method of predicting the responsiveness of a patient or patient population with cancer, to treatment with chemotherapeutic agents, especially erbB receptor tyrosine kinase inhibitors, comprising a marker gene set as defined above on a suitable support medium. Preferably the marker gene is attached to a support material or membrane such as nitrocellulose, or nylon or a plastic film or slide.

Preferably the kit comprises a microarray.

Detailed Description Including Preferred Embodiments

The invention will be described in more detail and illustrated by the following examples which are meant to serve to assist one of ordinary skill in the art in carrying out the invention and are not intended in any way to limit the scope of the invention. Certain elements of the invention are also described in more detail below.

"Set of isolated marker genes"

These are, according to the context of the embodiments described herein, a group of genes which can be used in classification or categorisation of patent response according to the description.

"Differential expression"

Genes that are either expressed at a higher or lower level as between groups of responders or nonresponders.

"Responders/Non responders"

Objective tumour responses according to Union International Contre le Cancer/World Health Organization (U ICC/WHO) Criteria are categorised as follows: complete response (CR): no residual tumour in all evaluable lesions; partial response (PR): residual tumour with evidence of chemotherapy-induced 50% or greater decrease under baseline in the sum of all measurable lesions and no new lesions; stable disease (SD) residual tumour not qualified for CR; and progressive disease (PD): residual tumour with evidence of 25% or greater increase under baseline in the sum of all measurable lesions or appearance of new lesions. As defined herein, non responders are PD.

The present invention is particularly effective for determining those patients which are CR or PR

"ErbB receptor inhibitors including, without limitation, ErbB receptor tyrosine kinase inhibitors"

This family includes EGF, erbB2 (HER), erbB3 (note that erbB3 does not have a functional kinase domain) and erbB4 as described in the background to the invention above.

"Gene-specific oligonucleotides"

These are intended to be unique to the respective genes so that, for example, fragments of the gene that uniquely identify the gene. Advantageously, a gene-specific oligonucleotide is between 5 and 50 nucleotides in length, preferably about 15 to 30 nucleotides, and most preferably about 23 nucleotides.

"Arrays or microarrays"

Array technology and the various techniques and applications associated with it are described generally in numerous textbooks and documents. Gene array technology is particularly suited to the practice of the present invention. Methods for preparing micoarrays are well known in the art. These include Lemieux et al., (1998), Molecular Breeding 4, 277-289, Schena and Davis. Parallel Analysis with Biological Chips. in PCR Methods Manual (eds. M. Innis, D. Gelfand, J. Sninsky), Schena and Davis, (1999), Genes, Genomes and Chips. In DNA Microarrays: A Practical Approach (ed. M. Schena), Oxford University Press, Oxford, UK, 1999), The Chipping Forecast (Nature Genetics special issue; January 1999 Supplement), Mark Schena (Ed.), Microarray Biochip Technology, (Eaton Publishing Company), Cortes, 2000, The Scientist 14[17]:25, Gwynne and Page, Microarray analysis: the next revolution in molecular biology, Science, 1999 August 6; and Eakins and Chu, 1999, Trends in Biotechnology, 17, 217-218.

The technology is described in PCT/US01/10063 and US 2002 090979 and references therein.

Commercial suppliers include Affymetrix (California) and Clontech Laboratories (California).

Major applications for array technology include the identification of sequence (nucleotide sequence/nucleotide sequence mutation) and the determination of expression level (abundance) of nucleotide sequences. Gene expression profiling may make use of array technology, optionally in combination with proteomics techniques (Celis et al, 2000, FEBS Lett, 480(1):2-16; Lockhart and Winzeler, 2000, Nature 405(6788):827-836; Khan et al., 1999, 20(2):223-9). Other applications of array technology are also known in the art; for example, nucleotide sequence discovery, cancer research (Marx, 2000, Science 289: 1670-1672; Scherf, et al, 2000, Nat Genet;24(3):236-44; Ross et al, 2000, Nat Genet. 2000 Mar;24(3):227-35), SNP analysis (Wang et al, 1998, Science, 280(5366):1077-82), drug discovery, pharmacogenomics, disease diagnosis (for example, utilising microfluidics devices: Chemical & Engineering News, February 22, 1999, 77 (8):27-36), toxicology (Rockett and Dix (2000), Xenobiotica, 30(2):155-77; Afshari et al., 1999, Cancer Res1;59(19): 4759-60) and toxicogenomics (a hybrid of functional genomics and molecular toxicology). The goal of toxicogenomics is to find correlations between toxic responses to toxicants and changes in the nucleotide sequencetic profiles of the objects exposed to such toxicants (Nuwaysir, et al (1999), Molecular Carcinonucleotide sequencesis, 24:153-159).

In general, any library may be arranged in an orderly manner into an array, by spatially separating the members of the library. Examples of suitable libraries for arraying include nucleic acid libraries (including DNA, nucleotide sequence, oligonucleotide, etc libraries), peptide, polypeptide and protein libraries, as well as libraries comprising any molecules, such as ligand libraries, among others. Accordingly, where reference is made to a "library" such reference includes reference to a library in the form of an array.

The members of a library are generally fixed or immobilised onto a solid phase, preferably a solid substrate, to limit diffusion and admixing of the samples. In particular, the libraries may be immobilised to a substantially planar solid phase, including membranes and non-porous substrates such as plastic and glass. Furthermore, the samples are preferably arranged in such a way that indexing (i.e. reference or access to a particular sample) is facilitated. Typically the samples are applied as spots in a grid formation. Common assay systems may be adapted for this purpose. For example, an array may be immobilised on the surface of a microplate, either with multiple samples in a well, or with a single sample in each well. Furthermore, the solid substrate may be a membrane, such as a nitrocellulose or nylon membrane (for example, membranes used in blotting experiments). Alternative substrates include glass, or silica based substrates. Thus, the samples are immobilised by any suitable method known in the art, for example, by charge interactions, or by chemical coupling to the walls or bottom of the wells, or the surface of the membrane. Other means of arranging and fixing may be used, for example, pipetting, drop-touch, piezoelectric means, ink-jet and bubblejet technology, electrostatic application, etc. In the case of silicon-based chips, photolithography may be utilised to arrange and fix the samples on the chip. The samples may be arranged by being "spotted" onto the solid substrate; this may be done by hand or by making use of robotics to deposit the sample. In general, arrays may be described as macroarrays or microarrays, the difference being the size of the sample spots. Macroarrays typically contain sample spot sizes of about 300 microns or larger and may be easily imaged by existing gel and blot scanners. The sample spot sizes in microarrays are typically less than 200 microns in diameter and these arrays usually contain thousands of spots. Thus, microarrays may require specialised robotics and imaging equipment, which may need to be custom made. Instrumentation is described generally in a review by Cortese, 2000, The Scientist 14[11]:26.

Techniques for producing immobilised libraries of DNA molecules have been described in the art. Generally, most prior art methods describe how to prepare single-stranded nucleic acid molecule libraries, using for example masking techniques to build up various permutations of sequences at the various discrete positions on the solid substrate. US 5,837,832 describes an improved method for producing DNA arrays immobilised to silicon substrates based on very large scale integration technology. In particular, US 5,837,832 describes a strategy called "tiling" to prepare specific sets of probes at spatially-defined locations on a substrate which may be used to produced the immobilised DNA libraries of the present description. US 5,837,832 also provides references for earlier techniques that may also be used.

To aid detection, targets and probes may be labelled with any readily detectable reporter such as a fluorescent, bioluminescent, phosphorescent, radioactive reporter. Labelling of probes and targets is disclosed in Shalon et al., 1996, Genome Res 6(7):639-45.

The materials for use in the methods of the present invention are ideally suited for preparation of kits. A set of instructions will typically be included.

GENERAL RECOMBINANT DNA METHODOLOGY TECHNIQUES

The present invention employs, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press.

In a specific embodiment, a cDNA microarray system representing 27, 648 genes was used to select a set of genes predicating the responsiveness to gefitinib for advanced NSCLC. Statistical analysis of the expression profiles identified dozens of genes differentially expressed between responders and non-responders to gefitinib. A drug response scoring (DRS) system based on the expression of these genes successfully predicted the response to gefitinib therapy.

Description of the Figures

**Figure 1:** Images illustrating laser-microbeam microdissection of four representative lung adenocarcinomas. The upper row shows the samples before dissection; the lower row, dissected cancer cells (H.E. stain X100). TBB indicates transbronchial biopsy; LN, lymph-node.

**Figure 2:** Establishing a scoring system to predict the efficacy of gefitinib treatment.

**A.** Different prediction scores appear when the number of discriminating genes is changed. The number of the discriminating gene sets (from 5 to 51) corresponds to the number of selected genes from the top of the rank-ordered list in table 4. A larger value of classification score (CS) indicates better separation of the two groups.
**B.** Hierarchical clustering of 17 "learning" cases using 51 candidate genes for gefitinib-sensitivity (left), and 12 prediction genes that were finally selected for the GRS (right). The dendrograms represent similarities in expression patterns among individual cases; longer branches indicate greater differences. The two groups were most clearly separated by the 12-gene set.
**C.** Schematic distinction of responder, non-responder and "test cases" verified on the basis of the GRS. Red diamonds denote prediction scores for learning PR cases and blue diamonds represent learning PD cases. A pink triangle indicates a test PR case that had not been used for establishing GRS, and blue triangles indicate test PD cases. Yellow triangles indicate test SD cases that kept the SD status throughout the 4-month observation period, and green triangles indicate test cases once judged as SD at a **certain-time** point of the study but showed progression of the disease within three or four months after the start of treatment.

**Figure 3:** Validation of GRS with semi-quantitative RT-PCR and immunohistochemical analyses.

**A.** Representative image of semi-quantitative RT-PCR analysis ofRNAs from the PR and PD groups. *OSMR* and *GCLC* genes were over-expressed in non-responders (PD). The integrity of each cDNA template was controlled through amplification of *ACTB.*
**B.** Immunohistochemical staining of representative samples from fiberscopic transbronchial biopsy (TBB) and lymph-node (LN) biopsy from the same PD-patient (No. LC21), using anti-AREG antibody (X 200).
**C.** Immunohistochemical staining of representative samples from PD patients, using antibodies for other 4 prediction markers (TGFA, ADAM9, CD9, and OSMR) (X200).

**Figure 4:** Serologic concentration of TGFA determined by ELISA in 5 PR, 10 SD, and 20 PD adenocarcinoma cases. The averaged serum levels of TGFA were shown as black bars: 19·0 $\pm$ 2·8 pg/ml (mean$\pm$SE) in PD patients, 13·9 $\pm$ 1·9 pg/ml in SD patients, and 12·8 $\pm$ 1·4 pg/ml in PR patients.

**Figure 5:** Anti-apoptotic effect of secreted AREG on gefitinib-sensitive PC-9 cells.

A. Expression of *AREG* transcript examined by semi-quantitative RT-PCR in lung-adenocarcinoma cell lines PC-9, NCI-H358, and -H522.

**B**. PC-9 cells cultured in medium supplemented with 10% FCS, in serum-free medium, or in serum-free conditioned medium (CM) obtained from cultures of NCI-H358 or -H522 cells. Each medium was replaced once with the same medium at the 48-hour time point; 72 hours after adding gefitinib at concentrations of 0·5 or 1·0 μM, cell viability was measured by MTT assays. The experiments were done in triplicate. The Y-axis indicates the relative MTT value (MTT in the presence of 0·5 or 1·0 μM gefitinib/MTT in the absence of gefitinib) of the cells incubated in different media.

**C**. Effect of AREG, secreted in an autocrine manner, on the resistance of NSCLC cells to gefitinib. At the start of culture, PC-9 cells were inoculated into medium containing 1·0 μM gefitinib and recombinant AREG protein (final concentrations of 1-100 ng/ml); 72 hours later, cell viability was measured by triplicate MTT assays (blue bars). The Y-axis indicates the relative MTT values (MTT at individual concentrations of AREG/MTT without AREG) of the cells.

Effect of AREG on the viability of NSCLC cells in the absence of 1·0 μM gefitinib was also studied. Individual PC-9 cells were added to medium containing recombinant AREG protein but no gefitinib; 72 hours later, viability was measured by triplicate MTT assays (red bars).

**Figure 6:** Immunohistochemical analysis of amphiregulin expression in sections derived from PD and PR patients.

Materials and Methods

***Patients and tissue samples***

A phase II clinical study was carried out comprising a multi-center trial to explore the dominant biological factors responsible for clinical anti-tumor effect, adverse drug reactions (ADR) and pharmacokinetics of ZD1839 dosed 250mg daily in patients with advanced non-small-cell lung cancer who have failed previous chemotherapy. The primary endpoint was to clarify a gene-expression profile that could determine in advance a potential anti-tumor effect of gefitinib. At the start of the study, the sample size was estimated using studies conducted thus far as a rationale.[12,13] Since the response rate for gefitinib has been less than 20% in patients with lung cancer,[8-10] about 50 patients were estimated to be required to obtain learning cases estimated above. Patients whose locally advanced (stage IIIB) or metastasized (stage IV) NSCLCs were resistant to one or more regimens of conventional chemotherapy were enrolled in this trial. Inclusion criteria were (1) age greater than 20 years, (2) Performance Status (PS) 0-2, (3) adequate liver and kidney function tests. All patients were treated with 250mg of gefitinib orally once a day at the Tokushima University or Kinki University hospitals in Japan. The treatment was continued until the patient was dropped from the study due to (1) progression of disease, (2) intolerable toxicity, or (3) withdrawal of consent.

Objective tumor responses were assessed every 4 weeks after the beginning of treatment, according to criteria outlined by the *Union International Contre le Cancer*/World Health Organization (UICC/WHO). Response categories were as follows: complete response (CR), no residual tumor in any evaluable lesion; partial response (PR), residual tumor with evidence of 50% or greater decrease under baseline in the sum of all measurable lesions, and no new lesions; progressive disease (PD), residual tumor with evidence of 25% or greater increase under baseline in the sum of all measurable lesions, or appearance of new lesions; and stable disease (SD), residual tumor not qualified for CR, PR, or PD. All evaluable lesions were measured bi-dimensionally (sum of products of longest diameter and its longest perpendicular of measurable lesions) using the same techniques as baseline, e.g. plain X-ray, CT, or MRI.

At the end of 4-month treatment (or withdrawal), the best overall response was evaluated for each patient based on definitions as follows: CR, patients who qualified for CR at two sequential examination points with an interval of at least 28 days between them; PR, patients judged as PR or better at two sequential examination points with an interval of at least 28 days between them; SD, patients who were SD or better at two sequential examination points at least 28 days apart but who did not qualify as CR or PR. The first judgment of an SD case must be done at or after the first tumor assessment point (28 days after randomization); PD, the patients determined as PD at or before the first tumor assessment point (28 days after randomization); Unknown, the patient does not qualify for a best response of increased disease, and all objective statuses after baseline (before randomization) and before progression are unknown.

Prior to the gefitinib treatment, tumor specimens were taken by transbronchial (TBB), skin, or lymph-node biopsy with written informed consent from each patient. Ethics approval was obtained from the ethics committee of the individual institutes. Biopsy samples were frozen immediately, embedded in TissueTek OCT medium (Sakura, Tokyo, Japan), and stored at -80 °C. All samples were examined microscopically, and samples from 28 patients (17 learning and 11 test cases) that contained enough cancer cells for analysis of expression profiles were initially selected for further analysis. For validation of the prediction system, a blinded set of samples from 5 newly enrolled cases (4 PD and 1 SD) were also added to the 11 test cases. Clinical and histological information about these patients is summarized in Table 1-3.

***Microdissection***

In view of significant differences in the proportions of cancer cells and various types of parenchymal cells that are present from one tumor to another, microdissection is a necessary means of obtaining precise gene-expression profiles on cDNA microarrays. Therefore we stained 8 μm-thick frozen sections with hematoxylin and eosin and collected cancer cells selectively, using the μCUT laser-microbeam microdissection system (Molecular Machines & Industries AG, Glattbrugg, Switzerland).[14] In this system tissue sections are mounted on a thin supporting polyethylene membrane that will be cut together with the target tissue; a pulsed-ultraviolet (UV) narrow-beam-focus laser cuts out cancer cells along a pre-selected track that can be observed on a video screen. The material to be extracted is never directly exposed to the laser but only circumscribed by it; unlike other LMM systems, this one allows recovery of dissected cells to proceed without radiation. Moreover, the membrane protects the tissue on the slide against cross-contamination. Using this system we were able to isolate small areas of tissue rapidly, and to isolate single cells from histological sections (figure 1).

***RNA extraction and T7-based RNA amplification***

Total RNA was extracted from individual microdissected populations of cancer cells using RNeasy mini kits and RNase-free DNase kits (QIAGEN, Hilden, Germany) according to the manufacturer's protocols. Total RNAs were subjected to T7-based RNA amplification, as described previously.[15] Two rounds of amplification yielded 40-200 μg of aRNA (amplified RNA) (>100,000-fold) from each sample. As a control probe, normal human lung poly(A)$^+$RNA (BD Biosciences Clontech, Palo Alto, CA and BIOCHAIN, Hayward, CA, USA) was amplified in the same way. Aliquots (2·5 μg) of aRNA from individual samples and from the control were reversely transcribed in the presence of Cy5-dCTP and Cy3-dCTP respectively.

***cDNA microarray***

Our "genome-wide" cDNA microarray system contains 27,648 cDNAs selected from the UniGene database of the National Center for Biotechnology Information.[15] Fabrication of the microarray, hybridization, washing, and detection of signal intensities were described previously.[15] To normalize the amount of mRNA between tumors and controls, the Cy5/Cy3 ratio for each gene's expression was adjusted so that the averaged Cy5/Cy3 ratio of 52 housekeeping genes was equal to one. We assigned a cutoff value to each microarray slide using analysis of variance, and the Cy5/Cy3 ratio of the gene was calculated as follows: (1) if Cy5 (cancer sample) was lower than the cut off level, then the Cy5/Cy3 ratio of the gene was substituted by 2·5 percentile among the Cy5/Cy3 ratios of other genes whose Cy5 and Cy3 were higher than the cut off level; (2) if Cy3 (control sample) was lower than the cut off level, then the Cy5/Cy3 ratio of the gene was substituted by 97·5 percentile among the Cy5/Cy3 ratios of other genes whose Cy5 and Cy3 were higher than the cut off level; (3) if both Cy5 and Cy3 were lower than the cut off level, then the Cy5/Cy3 ratio of the gene was left blank.

*Extraction of genes for predicting responsiveness to gefitinib*

To discover genes that might be associated with sensitivity to gefitinib, individual measurements of about 27,648 genes were compared between the two groups of patients, one classified as responders to gefitinib (PR) and the other as non-responders (PD). To reduce the dimensionality of the number of potent genes that could discriminate between the two classes, we extracted only genes that fulfilled two criteria: 1) signal intensities were higher than the cut-off level in at least 60% of either group, and 2)| $MED_{PR}$ - $MED_{PD}$ |□1, where MED indicates the median calculated from log-transformed relative expression ratios in each group. Then random-permutation tests were applied to estimate the ability of individual genes to distinguish between the two classes (PR and PD); mean (μ) and standard deviations (σ) were calculated from the log-transformed relative expression ratios of each gene in both groups. A discrimination score (DS) for each gene was defined as follows:

$$DS = (\mu_{PR} - \mu_{PD}) / (\sigma_{PR} + \sigma_{PD}).$$

The samples were randomly permutated 10,000 times for each pair of groups. Since the DS dataset of each gene showed a normal distribution, we calculated a p-value for the user-defined grouping.

***Calculation of drug-response scores***

We calculated gefitinib response scores (GRS) reflecting the expression levels of candidate prediction-genes according to procedures described previously.[16-18] Each gene (gi) votes for either responder (PR) or non-responder (PD) depending on whether the expression level (xi) in the sample is closer to the mean expression level of one group or the other in reference samples. The magnitude of the vote (vi) reflects the deviation of the expression level in the sample from the average of the two classes:

$$Vi = | xi - (\mu_{PR} + \mu_{PD}) /2 |.$$

We summed the votes to obtain total votes for responders ($V_{PR}$) and non-responders ($V_{PD}$), and calculated GRS values as follows:

GRS = (($V_{PR}$ - $V_{PD}$) / ($V_{PR}$ + $V_{PD}$)) X 100, where the GRS value reflects the margin of victory in the direction of either responder or non-responder. GRS values range from -100 to 100; the higher an absolute value of GRS, the stronger the prediction.

***Cross-validation of scores and evaluation of the prediction system***

The prediction scores of all samples were obtained by a leave-one-out approach, in which one sample at a time was removed from the sample set; permutational p-values and mean values of the two classes were calculated for each gene using the remaining samples. The drug-response of the withheld sample was predicted by calculating the prediction score. These procedures were repeated for each sample.[16-17]

To evaluate the reliability of the prediction system, we calculated a "classification score" (CS) using the GRS values of responders and non-responders in each gene set, as follows:

$$CS = (\mu_{GRSpr} - \mu_{GRSpd})/(\sigma_{GRSpr} + \sigma_{GRSpd}).$$[17]

A larger value of CS indicates better separation of the two groups by the prediction system.

***Hierarchical clustering***

We used web-available software ("Cluster" and "TreeView") written by M. Eisen (http://genome-www5.stanford.edu/MicroArray/SMD/restech.html) to create a graphic representation of the microarray data and to create a dendrogram of hierarchical clustering. Before the clustering algorithm was applied, the fluorescence ratio for each spot was first log-transformed and then the data for each sample were median-centered to remove experimental biases.

***Semi-quantitative RT-PCR analysis***

*Aliquots (5.0 μg) of the same aRNA hybridized to the microarray slides from individual samples and from the normal control lung were reversely transcribed using oligo(dT)12-18 primer and SuperScript II reverse transcriptase (Invitrogen, Carlsbad, CA, USA). Semi-quantitative RT-PCR experiments were carried out with the following sets of synthesized primers specific to the 12 top-ranked genes used for establishing a GRS or with beta-actin (ACTB)-specific primers as an internal control:* FIJ22662, *5'-GCCATAAGTGGTCCCACAGT-3' and 5'-GTCTTCTAGTCCGTCATCTCCCT-3', Amphiregulin* (AREG), *5'-CCATAGCTGCCTTTATGTCTGC-3' and 5'-CTTTTTACCTTCGTGCACCTTT-3'; coronin, actin binding protein, 1C* (CORD1C), *5'-TAATCTGCTGAGGACCTTTTGTC-3' and 5-TAATTCACTGTCCTCTTCTGGGA-3',-apoptosis, caspase activation inhibitor (*AVEN*), 5'-GCTCACAGCAGTAAATGCCTA-3' and 5'-TGCTATGCTGTAAACACTGGCTA-3'; dual specificity phosphatase 3 (*DUSP3*), 5'-GGATCCTTTATTGGTGGTAGAGC-3' and 5'-CCAGAGTGACCCTGAAGATAAAT-3';* DJ473B4, *5'-ACCTGATTCTCTAGGTGCAGTTT-3' and 5'-GTCGTTTCAACCAGGTAGTTTTG-3'; pleckstrin homology-like domain, family A, member 2 (*PHLDA2*), 5'-GGGCGCCTTAAGTTATTGGA-3' and 5'-GGATGGTAGAAAAGCAAACTGG-3'; RNA binding motif protein* 7 *(*RBM7*), 5'-TGTAATGGAGATTGTACAGGTTG-3' and 5'-AGGAACAGTACAAATGCTGTGGT-3')*- BX092512 *(EST), 5'-GCACTCCTTGAAGGTACACTAAC-3' and 5' -ATTTGTATTCACTCAGCCATGC-3'; oncostatin M receptor (*OSMR*), 5' -ACCCAACTTCAAAACTAGGACTC-3' and 5 '-ACAGCTTGATGTCCTTTCTATGC-3'; glutamate-cysteine ligase, catalytic subunit (*GCLC*), 5'-TCATGAAAGGCACTGAGTTTTG-3' and 5'-GTTAGCTGAAGCAGCTTTATTGC-3'; collagen, type IV, alpha*

*3 binding protein (*COL4A3BP*), 5'-ATATGCACAATCCTGGAAGTGA-3' and 5'-TGCCTTACTAGCATTACCACCAT-3';* ACTB, *5'-GAGGTGATAGCATTGCTTTCG-3' and 5'-CAAGTCAGTGTACAGGTAAGG3'. PCR reactions were optimized for the number of cycles to ensure product intensity within the logarithmic phase of amplification. We did phosphorimager quantification analysis (Molecular Imager FX: Bio-Rad Laboratories, Hercules, CA, USA), and RT-PCR band intensities were quantitatively compared with normalized Cy5/Cy3 ratio of gene expression from the microarray data.*

RT-PCR was performed to screen the mutation at entire region of codon 709 - 870 (from p-loop to activation loop) of *EGFR* which was recently reported as a hot spot of mutation,[18] using three primer sets: fragment-1, 5'-TCTTACACCCAGTGGAGAAGC-3' and 5'-GTCTTTGTGTTCCCGGACAT-3'; fragment-2, 5'-ACTATGTCCGGGAACACAAA-3' and 5'-TTCCGTCATATGGCTTGG-3'; firagment-3, 5'-CGTCGCTATCAAGGAATTAAGAG-3' and 5'-GTAGCTCCAGACATCACTCTGGT-3'. RT-PCR products from 19 NSCLC patients treated with gefitinib were analyzed by direct sequencing.

### *Immunohistochemical analysis*

To confirm the differential expression of AREG and transforming growth factor-alpha (TGFA) proteins, both of which encode the ligand for EGFR and other ERBB members, and other 3 candidate markers (a disintegrin and metalloproteinase domain 9 (ADAM9), CD9 antigen (p24), and OSMR), which are also known to relate to the EGFR signaling, for predicting responders vs non-responders to gefitinib, we stained clinical tissue sections obtained by fiberscopic transbronchial biopsy (TBB) and lymph-node biopsy using ENVISION+ Kit/HRP (DakoCytomation, Glostrup Denmark). Briefly, after endogenous peroxidase and protein blocking reactions, anti-human AREG polyclonal antibody (Neo Markers, Fremont, CA, USA), anti-human TGFA monoclonal antibody (Calbiochem, Darmstadt, Germany), anti-human ADAM9 monoclonal antibody (R&D Systems Inc. Minneapolis, MN, USA), anti-human CD9 monoclonal antibody (Novocastra Laboratories Ltd, Newcastle upon Tyne, UK), or anti-human OSMR monoclonal antibody (Santa Cruz Biotechnology, Inc., Santa Cruz, CA, USA), was added, and then HRP-labeled anti-rabbit or antimouse IgG as the secondary antibody. Substrate-chromogen was then added and the specimens were counterstained with hematoxylin.

Frozen tissue samples from 11 patients were selected for analysis of immunohistochemistry. Positivity of immunostaining was assessed semi-quantitatively by scoring intensity as absent or positive by three independent investigators without prior knowledge of the clinical follow-up data. Cases were accepted only as positive if reviewers independently defined them thus.

### *ELISA*

Serum was obtained from an independent set of 35 lung-ADC patients who were treated with gefitinib based on the same protocol as this clinical study at Hiroshima University hospital in Japan (5 for PR, 10 for SD, and 20 for PD). The sera of all the patients were obtained with informed consent at the time of diagnosis and every 4 weeks after the beginning of treatment, and stored at -80 °C. The serum TGFA levels were measured by an ELISA using a commercially available enzyme test kits (TGF-alpha ELISA kit: Oncogene Rsearch Products, San Diego, CA, USA).

### *In vitro gefitinib treatment and AREG-autocrine assay*

Human NSCLC (adenocarcinoma) cell lines PC-9, NCI-H358, and NCI-H522 were purchased from the American Type Culture Collection (ATCC; Rockville, MD, USA). To detect expression of *AREG* in these NSCLC cells, total RNA from each line was reverse-transcribed for single-stranded cDNAs using oligo(dT)$_{12-18}$ primer and Superscript II (Invitrogen). Semi-quantitative reverse transcriptase-PCR (RT-PCR) was carried out as described previously.[14] gefitinib (4-(3-chloro-4-fluoroanilino)-7-methoxy-6-(3-morpholinopropoxy) quinazoline: ZD1839, Iressa), an inhibitor of epidermal growth factor receptor tyrosine kinase, was provided by AstraZeneca Pharmaceuticals (Macclesfield, UK). The drug was dissolved in DMSO at a concentration of 10mM and kept at -20°C.

We performed flow-cytometry to determine the sensitivity of lung adenocarcinoma cell lines to gefitinib treatment. Cells were plated at densities of 5 X $10^5$ cells/100-mm dish and treated with 1·0 μM of gefitinib in appropriate serum-free medium. The cells were trypsinized 72 hours after the treatment, collected in PBS, and fixed in 70% cold ethanol for 30 min. After treatment with 100 μg/ml RNase (Sigma-Aldrich Co., St. Louis, MO, USA), the cells were stained with 50 μg/ml propidium iodide (Sigma-Aldrich Co.) in PBS. Flow cytometry was performed on a Becton Dickinson FACScan and analyzed by ModFit software (Verity Software House, Inc., Topsham, ME, USA). The percentages of nuclei in G0/G1, S, and G2/M phases of the cell cycle and sub-G1 population were determined from at least 20,000 ungated cells.

To investigate whether AREG functions as an autocrine anti-apoptotic factor in lung adenocarcinoma cells treated with gefitinib, we carried out the following assay. First, gefitinib-sensitive PC-9 cells, which do not express *AREG*, were cultured in serum-free medium for at least 8 hours prior to gefitinib treatment. These cells were then incubated with 0·5 or 1·0 μM of gefitinib for 72 hours in media that were either serum-free or supplemented with 10% FCS, or in serum-free conditioned medium collected from 72-hour cultures of *AREG*-expressing cells (NCI-H358 or NCI-H522). Each medium was replaced once with the same medium containing gefitinib at the 48-hour time point. To detect the response of each cell line to gefitinib, viability was evaluated by MTT assays using Cell Counting Kits (WAKO, Osaka, Japan).

To confirm the autocrine effect of AREG on the gefitinib-resistance of NSCLC cells, we cultured PC-9 cells for 72 hours in serum-free medium containing 1·0 μM of gefitinib and recombinant AREG protein (Genzyme-Techne, Minneapolis, MN, USA) in final concentrations of 1-100 ng/ml. Cell viability was evaluated by MTT assays. A possible effect of AREG itself on the viability of NSCLC cells was evaluated also, by culturing the PC-9 cells in serum- and gefitinib-free medium containing only recombinant AREG protein. MTT assays were performed as above.

**Results**

***Response to gefitinib treatment***

Of the 53 patients enrolled in this trial, 46 had tumors diagnosed as adenocarcinomas (86·8%); five were squamous-cell carcinomas (9·4%); two were large cell carcinomas (3·8%). Fifteen patients achieved a PR and nobody revealed a CR; 17 patients were classified as SD, and 19 as PD. No clinical-response data were available for two of the patients. The tumor-response rate (CR+PR/CR+PR+SD+PD) for this treatment was 29·4%, and the disease control rate (CR+PR+SD/CR+PR+SD+PD) was 62·8% (table 1).

Tumor samples were collected from 43 patients. Samples from 32 of those 43 contained sufficient numbers of cancer cells for analysis of expression profiles on our cDNA microarray. The numbers of samples that were judged to be suitable for further microarray analysis, were 8 for PR, 7 for SD, and 13 for PD (table 2). 17 of the 28 samples were analyzed as learning cases (7 for PR and 10 for PD), and 11 were as test cases (1 for PR, 3 for PD, and 7 for SD) for establishing a predictive scoring system for the efficacy of gefitinib treatment. For further validation of the prediction system, another blinded set of samples from 5 newly enrolled test-cases (4 for PD and 1 for SD) were obtained and added finally to the initial 11 test cases above.

***Identification ofgenes associated with sensitivity to gefitinib***

We attempted to extract genes that were differentially expressed between tumors from seven patients in the PR group (defined as responders) and those from 10 patients in the PD group (defined as non-responders) by comparing expression levels of 27,648 genes. (tables 2, 3).

We carried out a random-permutation test to distinguish between the two subclasses defined by tumor response, and identified 51 genes whose permutational p-values were less than 0·001 (table 4). Expression levels of 40 genes were higher, and those of the other 11 were lower, in the non-responders.

Establishment of a predictive scoring system for the efficacy of gefitinib treatment Based on the expression profiles of the 51 genes selected above, we tried to establish a predictive scoring system for the efficacy of gefitinib treatment. Prediction scores, termed gefitinib response score (GRS), were calculated according to procedures described previously (see Methods). To determine the number of candidates that provided the best separation of the two groups, we ranked the 51 genes on the basis of the significance of their permutational *p*-values and calculated prediction scores by the leave-one-out test, in decrements of 1 starting from the bottom of the rank-ordered list (51, 50, 49, 48 etc.). We calculated a classification score (CS), a standard we had previously defined for evaluation of the ability to discriminate two classes, for each set of genes.[17]

As shown in Figure 2A, we obtained different prediction scores when the number of discriminating genes was changed. We obtained the best CS, meaning the best separation of responders from non-responders, when we calculated the scores using only the 12 top-ranked genes in our candidate list.

Hierarchical clustering analyses using all 51 genes, or only the top 12, classified all 17 cases into one of two groups according to the response to gefitinib (figure 2, B). The two groups were most clearly separated when we used the top 12 genes for cluster analysis. Finally, we established a numerical drug-response-scoring algorithm that might be clinically applicable for predicting sensitivity of an individual NSCLC to gefitinib, on the basis of expression levels of the 12 selected genes.

To validate this prediction system we investigated 8 additional ("test") NSCLC cases (1 for PR and 7 for PD) that were completely independent of the 17 "learning" cases used for establishing the system. We examined gene-expression profiles in each of those samples and then calculated GRS on the basis of the expression levels of the 12 discriminating genes. As shown in Figure 2C, scores obtained by the GRS system were concordant with the clinical responses to gefitinib in all eight "test" cases.

***GRS values for patients with SD in tumor response***

GRS values for the eight test-SD patients were calculated according to the predictive scoring system established above. Although the values were widely distributed from - 83·0 (predicted as non-responder) to 61·6 (responder), the scores of patients who retained SD status throughout the observation period were likely to be higher than those of patients who had been judged as SD at a certain time-point of the study but showed progression of the disease within three or four months after the start of treatment (figure 2, C). Although the GRS system was established on the basis of gene-expression profiles that distinguished between patients with PR and patients with PD (without SD) in tumor response, these results suggest that the GRS serves in classifying SD patients into groups according to their response to gefitinib.

***Validation of GRS with semi-quantitative RT-PCR analysis***

To confirm differential expression of the top 12 predictive genes between PR and PD cases, expression values derived from microarray data were correlated with values from semi-quantitative RT-PCR of RNAs from the same patients (5 PR and 7 PD) (figure 3, A, table 5, A). Spearman rank correlations were positive for all of the 12 genes and significantly positive for seven of 12 genes.

***Immunohistochemical validation of GRS***

To validate differential expression of the predictive protein markers between PR and PD cases, we carried out immunohistochemical staining with five different antibodies for AREG, TGFA, ADAM9, CD9, and OSMR, all of which were known to be involved in the ligand-EGFRs signaling and whose permutational *p*-values were less than 0·01. We first stained paired tumor tissue sections obtained by TBB and lymph-node biopsy from the same patients using these 5 antibodies. No intra-patient differences on protein expression of these five markers were observed in three different patients (figure 3, B). We also validated the microarray data with the five markers in 11 NSCLC samples (5 for PR and 6 for PD). The results were consistent with the microarray data (figure 3, C, table 5, B).

***Serum levels of TGFA***

To further evaluate the availability of the prediction system in routine clinical situations, we detected TGFA protein using ELISA in serum samples from 5 PR, 10 SD, and 20 PD patients that were independently collected for serological test and were not enrolled in microarray analysis. The serum levels of TGFA were 19·0 $\pm$ 2·8 pg/ml (mean$\pm$SE) in PD patients, 13·9 $\pm$ 1·9 pg/ml in SD patients, and 12·8 $\pm$ 1·4 pg/ml in PR patients (figure 4). Twelve of 20 serum samples from PD patients were positive for TGFA and all samples from PR patients were negative, when 16·0 pg/ml was used as a cutoff.

***In vitro gefitinib treatment and AREG-autocrine assay***

AREG, a ligand for EGFR and other ERBB members was significantly over-expressed in non-responders but not (or hardly) detectable in responders. To investigate whether AREG protein leads to resistance of NSCLCs to gefitinib therapy when it is secreted in an autocrine manner, we performed the following biological analyses. We initially identified expression of AREG mRNA in lung-adenocarcinoma cell lines NCI-H358 and -H522, but not in PC-9, by means of RT-PCR experiments (figure 5, A). Next, we performed flow-cytometric analysis 72 hours after treatment of PC-9 cells with 1·0 $\mu$M of gefitinib, and found that gefitinib increased the percentages of nuclei in sub-G1 (24%) compared with cells with no treatment (6%) (data not shown). This result suggested that gefitinib might induce apoptosis in PC-9 cells.

We then analyzed the viability of PC-9 cells, which are gefitinib-sensitive and do not express *AREG*, after culture in serum-free medium or in serum-free, conditioned medium obtained from NCI-H358 or -H522 cells grown in the presence or absence of 0·5 or 1·0 $\mu$M of gefitinib. As shown in Figure 5B, the viability of PC-9 cells incubated in the serum-free, conditioned medium containing gefitinib was greater than that of PC-9 cells grown in serum-free medium with the same concentrations of gefitinib. As the supplier of gefinitib has reported previously, the anti-tumor effect of gefitinib decreases in the presence of 10% FCS, suggesting that this assay should be suitable for quantitative measurement of gefitinib dosage and activity.

To investigate whether AREG, secreted in an autocrine manner, inhibits apoptosis of NSCLC cells treated with gefitinib, we cultured PC-9 cells in serum-free medium containing recombinant AREG protein at final concentrations of 1-100 ng/ml, in the presence or absence of 1·0 $\mu$M gefitinib. The viability of PC-9 cells incubated with both AREG and 1·0 $\mu$M gefitinib was increased in comparison to cells incubated with 1-0 $\mu$M gefitinib only, in an AREG-dose-dependent manner (figure 5, C). On the other hand, recombinant AREG alone had no effect on the viability of PC-9 cells (figure 5, C). This observation appeared to indicate that AREG inhibits the apoptosis induced by gefitinib, but does not in itself affect cell viability. Immunostaining for AREG is shown in Figure 6.

**Discussion**

A large body of evidence supports the view that molecules in the EGFR autocrine pathway are involved in a number of processes important to cancer formation and progression, including cell proliferation, angiogenesis, and metastatic spread.[5] Therapeutic blockade of specific signaling, therefore, could be a promising strategy for cancer treatment. Gefitinib, a synthetic anilinoquinazoline, inhibits the tyrosine kinase activity of EGFR by competing with adenosine triphosphate for a binding site on the intracellular domain of the receptor.[7] In phase II trials (IDEAL 1 and IDEAL 2), use of gefitinib as a 2nd-, 3rd-, or 4th-line monotherapy for advanced NSCLC achieved tumor-response rates of nearly 20%,[8-10] which were superior to those achieved with conventional cytotoxic agents. Multivariate analysis of patients in the IDEAL 1 study suggested that the response rate in females might be higher than in males, and higher in patients with adenocarcinomas than in patients with squamous-cell carcinomas (odds ratios 2·7 and 3·5 respectively).[9] Recent study suggested that individuals in whom gefitinib is efficacious are more likely to have adenocarcinomas of the bronchioloalveolar subtype and to be never smokers (odds ratios 13·5 and 4·2 respectively).[19] The higher tumor-response rate (29·4%) documented in the clinical trial reported here might reflect a higher proportion of patients with adenocarcinoma (46 adenocarcinomas, five squamous-cell carcinomas and two large-cell carcinomas) than has been the case in other studies. The clinicopathological determinants of gefitinib sensitivity including bronchioloalveolar carcinoma (BAC) features are predictve to a certan extent,[9,10,19,20] however, previous reports and our observations obviously suggest that no factors can perfectly predict the response of NSCLC to gefitinib treatment. Therefore novel methods to discriminate responders from non-responders in advance could allow a more focused use of gefitinib in clinical settings.

By statistical analysis of gene-expression profiles of advanced NSCLCs obtained on cDNA microarrays, we identified dozens of genes associated with sensitivity to gefitinib. We introduced a prediction-scoring system based on expression of the 12 genes that had shown the most significant differences in expression levels between responder (PR) and non-responder (PD) groups. This set of genes was selected from expression profiles of lung adenocarcinomas; however, the GRS system successfully classified all eight of our "test" PR and PD cases in accord with their clinical responses to gefitinib, and one of them was a squamous-cell carcinoma. Moreover, this system was likely to separate intermediate tumor responses (SD) into two groups, one representing patients who succeeded in maintaining the tumor-static effect for a long period and the other representing patients who failed to do so.

In practical terms, we need to predict the chemosensitivity of individual tumors using the minimally invasive techniques available at every hospital, because patients with advanced NSCLCs are rarely candidates for surgical resection of their tumors. Therefore we have tried to establish a prediction system that requires only the amount of cancerous tissue that can be obtained by, for example, flexible bronchofiberscopy. By verifying individual steps of the method, we were able to precisely profile gene expression in biopsy specimens as small as 1 mm. Relevant microarray results were confirmed by semi-quantitative RT-PCR for 12 genes that showed the most significant differences to establish a GRS system. Furthermore, we validated the effectiveness of antibodies for 5 different biomarkers (AREG, TGFA, ADAM9, CD9, and OSMR), all of which were reported to be involved in the ligand-EGFR signaling, for discriminating potential responders from non-responders, in both TBB and lymph-node biopsy samples. Moreover, we were able to detect serum TGFA proteins in lung-ADC patients by ELISA. Further evaluation of these markers for clinical use are necessary, however, the limited number of genes required for prediction should eventually enable laboratories to diagnose in advance the efficacy of gefitinib treatment for an NSCLC patient, using routine procedures such as serological examinations of blood, PCR experiments, or immunohistochemical analysis of biopsy specimens.

To our knowledge, this is the first report about gene-expression profiles of unresectable "advanced" lung cancers, although profiles of surgically resected specimens of "early" lung cancers have been reported.[21,22] However, about 70% of tumors in patients diagnosed with NCSLC are already locally advanced or metastatic, which generally renders them resistant to conventional therapeutic modalities. Therefore the genes listed here should be useful for disclosing molecular mechanisms of lung-cancer progression and may be potential targets for drug development.

Gefitinib was developed as a "selective" inhibitor of EGFR-TK; however, no clear association between the level of EGFR activation and response to gefitinib has been found *in vitro* or *in vivo*.[7,23] In clinical trials, gefitinib has been more effective against adenocarcinomas than against squamous-cell carcinomas,[9,10] although over-expression of EGFR is less frequent in adenocarcinomas.[24] Therefore, it is important to identify which individual tumors are good targets for this treatment. In our analysis using clinical samples, the difference in EGFR protein expression between responders and non-responders were not statistically significant. On the other hand, amphiregulin *(AREG)* and transforming growth factor alpha (*TGFA*), both of which encode the ligand for EGFR and other ERBB members, were significantly over-expressed in non-responders but not (or hardly) detectable in responders ($p$=0·0000000000093 and 0·0095 respectively; table 4).

The significance of the ligands and the EGFR autocrine loop in growth and survival of lung-cancer cells is

indisputable,[24-26] but the role of AREG in formation and progression of cancers is poorly understood. However, several lines of evidence suggest that over-expression of *AREG* is associated with shortened survival of patients with NSCLC.[24] Moreover, anti-apoptotic activity of AREG in human lung-adenocarcinoma cells was reported recently.[25] To investigate whether the anti-apoptotic activity of AREG leads to resistance of NSCLC cells to gefitinib therapy, we performed a biological assay using a gefitinib-sensitive but *AREG*-non-expressing NSCLC cell line, PC-9. We found that the anti-tumor activity of gefitinib on PC-9 cells was dramatically decreased by autocrine secretion of AREG. This evidence strongly suggests that although growth-factor signaling by the EGFR is markedly complicated at every step because of the multiplicity of ligands, dimerization partners, effectors, and downstream pathways,[26] AREG might be a principal activator of the ligands-receptor autocrine growth pathway that leads to cancer progression and resistance to gefitinib.

Several elements associated with the EGFR-TK pathway are present on our list of differentially-expressed genes. For example, genes encoding dual specificity phosphatase 3 (*DUSP3*), *ADAM9, CD9,* and *OSMR* were expressed predominantly in non-responders ($p$=0·00000000094, 0·01, 0·000022, and 0.0000011, respectively). *DUSP3* gene modulates EGFR signaling by dephosphorylating mitogen activated protein kinase (MAPK), a key mediator of signal transduction,[27] and ADAM9 is involved in activation of EGFR signaling by shedding the ectodomain of proHB-EGF (pro Heparin-binding epidermal growth factor-like growth factor).[28] CD9 physically interacts with transmembrane TGFA. CD9 expression strongly decreases the growth factor- and PMA- induced proteolytic conversions of transmembrane to soluble TGFA and strongly enhances the TGFA-induced EGFR activation.[29] OSMR is reported to be constitutively associated with ERBB2 in breast cancer cells. [30] Although other target molecules for gefitinib have been suggested, our results suggest that EGFR signaling is at least one of the important processes involved in response to this drug.

Since gefitinib can induce apoptosis of some cancer cells *in vivo,* other molecules with anti-apoptotic activity, as well as AREG, may contribute to a tumor's resistance to the drug. *AVEN* (apoptosis, caspase-activation inhibitor), which was specifically expressed in our non-responders ($p$=0·00000000042), is known to enhance the anti-apoptotic activity of Bcl-xL and to suppress Apaf-1-mediated caspase activation.[31] On the other hand, mechanisms regulating drug transport should also affect drug resistance. *GCLC* (glutamate-cysteine ligase, catalytic subunit), which plays an important role in cellular detoxification of anticancer drugs such as cisplatin, etoposide and doxorubicin,[32] was over-expressed in our group of non-responders ($p$=0·00000012). As these genes correlated negatively with responses to chemotherapy in our panel of tumors (i.e. the higher the expression of these genes, the greater the resistance to gefitinib), they might be involved in the mechanism(s) leading to that resistance. It should be noted also that the functions of nearly half of our candidate prediction-genes are unknown. Therefore further investigations will be needed to reveal more clearly the biological events underlying responses of NSCLCs to gefitinib.

In summary, we identified 51 genes whose expression differed significantly between responders and non-responders to gefitinib among human lung carcinomas, and established a numerical scoring system, based on expression patterns of 12 of those genes, to predict the response of individual tumors to this drug. Although further validation using a larger set of clinical cases will be necessary, the data presented here may yield valuable insights into the molecular events underlying signal-suppressing strategies and provide important information about gefitinib treatment for individual NSCLC patients by testing a set of genes with high predictive values.

References

1. Fossella, F.V., et al., Randomized phase III trial of docetaxel versus vinorelbine or ifosfamide in patients with advanced non-small-cell lung cancer previously treated with platinum-containing chemotherapy regimens. The TAX 320 Non-Small Cell Lung Cancer Study Group. J Clin Oncol, 2000. 18(12): p. 2354-62.

2. Non-small Cell Lung Cancer Collaborative Group. Chemotherapy in non-small cell lung cancer: a meta-analysis using updated data on individual patients from 52 randomised clinical trials. Bmj, 1995. 311(7010): p. 899-909.

3. Schiller, J.H., et al., Comparison of four chemotherapy regimens for advanced non-small-cell lung cancer. N Engl J Med, 2002. 346(2): p. 92-8.

4. Kelly, K., et al., Randomized phase III trial of paclitaxel plus carboplatin versus vinorelbine plus cisplatin in the treatment of patients with advanced non-small-cell lung cancer: a Southwest Oncology Group trial. J Clin Oncol, 2001. 19(13): p. 3210-8.

5. Baselga, J., Why the epidermal growth factor receptor? The rationale for cancer therapy. Oncologist, 2002. 7 Suppl 4: p. 2-8.

6. Traxler, P., Tyrosine kinases as targets in cancer therapy - successes and failures. Expert Opin Ther Targets, 2003. 7(2): p. 215-34.

7. Wakeling, A.E., et al., ZD1839 (Iressa): an orally active inhibitor of epidermal growth fador signaling with potential for cancer therapy. Cancer Res, 2002. 62(20): p. 5749-54.

8. Herbst, R.S., Dose-comparative monotherapy trials of ZD1839 in previously treated non-small cell lung cancer patients. Semin Oncol, 2003. 30(1 Suppl 1): p. 30-8.

9. Fukuoka, M., et al., Final results from a phase □ trial of ZD1839 ('Iressa') for patients with advanced non-small cell lung cancer (IDEAL 1). Pro Am Soc Clin Oncol 2002. 21;298a(A1188).

10. Kris, MG., et al., A phase II trial of ZD1839 ('Iressa') in advanced non-small cell lung cancer (NSCLC) patients who had failed platinum- and docetaxel-based regimens (IDEAL 2). Pro Am Soc Clin Oncol 2002. 21;292a(A1166).

11. Inoue, A., et al., Severe acute interstitial pneumonia and gefitinib. Lancet, 2003. 361(9352): p. 137-9.

12. Bohm, M., et al., Microbeam MOMeNT: non-contact laser microdissection of membrane-mounted native tissue. Am J Pathol, 1997. 151(1): p. 63-7.

13. Okabe, H., et al., Genome-wide analysis of gene expression in human hepatocellular carcinomas using cDNA microarray: identification of genes involved in viral carcinogenesis and tumor progression. Cancer Res, 2001. 61(5): p. 2129-37.

14. Kitahara, O., et al., Alterations ofgene expression during colorectal carcinogenesis revealed by cDNA microarrays after laser-capture microdissection of tumor tissues and normal epithelia. Cancer Res, 2001. 61(9): p. 3544-9.

15. Golub, T.R., et al., Molecular classification of cancer: class discovery and class prediction by gene expression monitoring. Science, 1999. 286(5439): p. 531-7.

16. MacDonald, T.J., et al., Expression profiling of medulloblastoma: PDGFRA and the RAS/MAPK pathway as therapeutic targets for metastatic disease. Nat Genet, 2001. 29(2): p. 143-52.

17. Kaneta.Y.,et al., Prediction of sensitivity to STI571 among chronic myeloid leukemia patients by genome-wide cDNA microarray analysis. Jpn J Cancer Res 2002. 93, p. 849-856.

18. Pavelic, K., et al., Evidence for a role of EGF receptor in the progression of human lung carcinoma. Anticancer Res, 1993. 13(4): p. 1133-7.

19. Kikuchi, T., et al., Expression profiles of non-small cell lung cancers on cDNA microarrays: Identification of genes for prediction of lymph-node metastasis and sensitivity to anti-cancer drugs. Oncogene, 2003. 22(14): p. 2192-205.

20. Heighway, J., et al., Expression profiling of primary non-small cell lung cancer for target identification. Oncogene, 2002. 21(50): p. 7749-63.

21. Beer, D.G., et al., Gene-expression profiles predict survival ofpatients with lung adenocarcinoma. Nat Med, 2002. 8(8): p. 816-24.

22. Miura, K., et al., Laser capture microdissection and microarray expression analysis of lung adenocarcinoma reveals tobacco smoking- and prognosis-related molecular profiles. Cancer Res, 2002. 62(11): p. 3244-50.

23. Moasser, M.M., et al., The tyrosine kinase inhibitor ZD1839 ("Iressa") inhibits HER2-driven signaling and suppresses the growth of HER2-overexpressing tumor cells. Cancer Res, 2001. 61(19): p. 7184-8.

24. Rusch, V., et al., Overexpression of the epidermal growth factor receptor and its ligand transforming growth factor alpha is frequent in resectable non-small cell lung cancer but does not predict tumor progression. Clin Cancer Res, 1997. 3(4): p. 515-22.

25. Fontanini, G., et al., Evaluation of epidermal growth factor-related growth factors and receptors and of neoangiogenesis in completely resected stage I-IIIA non-small-cell lung cancer: amphiregulin and microvessel count are independent prognostic indicators ofsurvival. Clin Cancer Res, 1998. 4(1): p. 241-9.

26. Brundage, M.D., D. Davies, and W.J. Mackillop, Prognostic factors in non-small cell lung cancer: a decade ofprogress. Chest, 2002.122(3): p. 1037-57.

27. Hurbin, A., et al., Inhibition of apoptosis by amphiregulin via an insulin-like growth factor-1 receptor-dependent pathway in non-small cell lung cancer cell lines. J Biol Chem, 2002. 277(51): p. 49127-33.

28. Yarden, Y. and M.X. Sliwkowski, Untangling the ErbB signalling network Nat Rev Mol Cell Biol, 2001. 2(2): p. 127-37.

29. Prenzel, N., et al., EGF receptor transactivation by G-protein-coupled receptors requires metalloproteinase cleavage of proHB-EGF. Nature 1999. 402(6764):884-8

30. Nelson, Chau, et al., Aven, a novel inhibitor of caspase activation, binds Bcl-xL and Apaf-1. Molec Cell 2000. 6: p. 31-41.

31. Tipnis, SR., et al., Overexpression of the regulatory subunit of r-glutamylcysteine synthetase in Hela cells increases r-glutamylcysteine synthetase activity and confers drug resistance. Biochem J 1999. 337, p. 559-566.

Table 1: Summary of baseline patient characteristics and response

| Characteristics | | Percentage (%) | Number of Patient |
|---|---|---|---|
| Sex | | | |
| | male | 58·5 | (31) |
| | female | 41·5 | (22) |
| Age | | | |
| | median | 59 | |
| | range | 35-80 | |
| Histology | | | |
| | adenocarcinoma | 86-8 | (46) |
| | squamous cell carcinoma | 9·4 | (5) |
| | large cell carcinoma | 3·8 | (2) |
| Stage | | | |
| | IIIA | 1·9 | (1) |
| | IIIB | 7·5 | (4) |
| | IV | 90·6 | (48) |
| Performance Status | | | |
| | 0 | 26·4 | (14) |
| | 1 | 60·4 | (32) |
| | 2 | 13·2 | (7) |
| Number of Prior Regimen | | | |
| | 1 | 24·5 | (13) |
| | 2 | 35·9 | (19) |

(continued)

| | | |
|---|---|---|
| Number of Prior Regimen | | |
| 3 | 28·3 | (15) |
| 4 | 0 | (0) |
| 5 | 7·5 | (4) |
| 6 | 3·8 | (2) |
| Response to Gefitinib Therapy | | |
| CR | 0 | (0) |
| PR | 28·3 | (15) |
| SD | 32·1 | (17) |
| PD | 35·8 | (19) |
| unknown | 3·8 | (2) |
| Tumor Response Rate (% (CR+PR/CR+PR+SD+PD) | 29·4 | (15) |
| Disease Control Rate (%) (CR+PR+SD/CR+PR+SD+PD) | 62·8 | (32) |

Table 2: Number of cases suitable for analysis and their best overall responses

| | Best Overall Response | | | | |
|---|---|---|---|---|---|
| Number of Cases | PR | SD | PD | Unknown | Total |
| All cases enrolled | 15 | 17 | 19 | 2 | 53 |
| Cases that consented to the study | 15 | 14 | 13 | 1 | 43 |
| Cases suitable for analysis | 8 | 10 | 13 | 1 | 32 |
| Learning cases (1) | 7 | 0 | 10 | 0 | 17 |
| Test cases (1,2) | 1 | 7 | 3 | 0 | 11 |

(1) Learning cases were used for developing the GRS, whereas test cases were used for validation of the (2) Another blinded set or samples from 5 newly enrolled cases (4 PD and 1 SD) were also added to these 11 test cases later.

Table 3: Clinicopathological features of patients

| Case No. (*) | Sex | Age | Histology Type(1) | T N M | Stage Classificatio n (2) | Number of Previous Chemotherapy | EGFR Stained Tumour Cell (%) | EGFR mutation (3) | Plasma Gefitinib Concentration (ng/ml) | Response to Gefitinib (4) 1st mont h | 2nd mont h | 3rd mont h | 4th mont h | Best Overall Response(5) PR | use tor Predictio n (6) | GRS (7) 100 |
|---|---|---|---|---|---|---|---|---|---|---|---|---|---|---|---|---|
| LC01 | female | 36 | ADC | 1 0 1 | IV | 1 | | None detected | 258·9 | PR | PR | PR | PR | | learning | |
| LC02 | male | 64 | ADC | 2 3 1 | IV | 3 | 80 | | 140·3 | PR | PR | PR | PR | PR | learning | 100 |
| LC03 | female | 54 | ADC | 2 0 1 | IV | 3 | 80 | | 167·0 | PR | PR | PR | PR. | PR | learning | 100 |
| LC04 | female | 75 | ADC | 2 1 1 | IV | 1 | 20 | None detected | 169·7 | PR | PR | PR | PR | PR | learning | 100 |
| LC05 | female | 73 | ADC | 0 2 | 1 IV | 5 | 30 | 46 A760del (2481 2495c | 300·6 | PR | PR | PR | PR | PR | learning | 100 |
| LC06 | female | 75 | ADC | 4 1 | 1 IV | 3 | | None detected | 874·0 | SD | PR | PR | PR | PR | learning | 100 |
| LC07 | female | 70 | ADC | 2 1 | 1 IV | 3 | 80 | 7_A750del (2485_2496d | 460·8 | SD | PR | PR | PR | PR | learning | 100 |
| LC08 | female | 47 | ADC | 4 3 1 | IV | 2 | 95 | L858R (2819T>G) | 306·5 | PR | PR | PR | PR | PR | test | 54.8 |
| mean (range) 62 (36-76) | | | | | | 2·6(1-5) | 84 (20-95) | | 334·7 (140·3-874·0) | | | | | | | |
| LC09 | female | 63 | ADC | 4 0 1 | IV | 3 | 90 | | 743·4 | SD | SD | SD | SD | SD | test | 61.6 |
| LC10 | male | 56 | ADC | 2 0 | 1 IV | 6 | 70 | | 511·8 | SD | SD | SD | SD | SD | test | -9.8 |
| LC11 | male | 67 | ADC | 4 0 1 | IV | 2 | 0 | | 631·3 | SD | SD | SD | SD | SD | test | -5.3 |
| LC12 | male | 53 | ADC | 4 3 1 | IV | 2 | | None detected | 306·1 | SD | SD | SD | PD | SD | test | -23.8 |
| | | | | | | | | | | | | | | | | -58.5 |
| LC13 | female | 56 | ADC | 4 2 0 | IIIB | 2 | 40 | | 364·8 | SD | SD | PD | | SD | test | -83 |
| LC14 | female | 62 | ADC | 4 2 1 | IV | 3 | 60 | | 322·4 | SD | SD | PD | | SD | test | |
| LC15 | male | 61 | ADC | 0 0 1 | IV | 5 | 60 | | 278·9 | SD | SD | PD | | SD | test | -40.5 |
| mean 60 (53-67) | | | | | | 3·3(2-6) | 53(0-90) | | 451·2(278·9-631·3) | | | | | | | |
| LC16 | male | 42 | ADC | 4 3 1 | IV | 5 | | None detected | 212·6 | SD | PD | | | PD | learning | -63.9 |
| | | | | | | | | | | | | | | | | -86 |
| LC17 | female | 54 | ADC | 2 3 1 | IV | 2 | 50 | None detected | 320·6 | SD | PD | | | PD | learning | -67.8 |
| LC18 | female | 61 | ADC | 1 3 0 | IIIB | 2 | | None detected | 229·3 | SD | PD | | | PD | learning | |
| LC19 | male | 59 | ADC | 0 2 1 | IV | 2 | 30 | W817C (2697G>T) | 150·7 | SD | PD | | | PD | learning | -57.1 |
| | | | | | | | | | | | | | | | | -59.1 |
| LC20 | male | 65 | ADC | 0 3 1 | IV | 3 | | None detected | 167·8 | SD | PD | | | PD | learning | -73.1 |
| LC21 | male | 55 | ADC | 4 3 1 | IV | 3 | 80 | None detected | | PD | | | | PD | learning | |
| LC22 | male | 80 | ADC | 4 3 1 | IV | 2 | 80 | Q787Q (2607G>A) | | PD | | | | PD | learning | -55.5 |

(continued)

| Case No. (*) | Sex | Age | Histology Type(1) | T N M | Stage Classification (2) | Number of Previous Chemotherapy | EGFR Stained Tumour Cell (%) | EGFR mutation (3) | Plasma Gefitinib Concentration (ng/ml) | Response to Gefitinib (4) 1st month | 2nd month | 3rd month | 4th month | Best Overall Response(5) PR | use tor Prediction (6) | GRS (7) 100 |
|---|---|---|---|---|---|---|---|---|---|---|---|---|---|---|---|---|
| LC23 | male | 35 | ADC | 4 0 1 | IV | 5 | | None detected | | PD | | | | PD | learning | -100 |
| | | | | | | | | | | | | | | | | -46.7 |
| LC24 | male | 57 | ADC | 4 3 1 | IV | 1 | 0 | None detected | | PD | | | | PD | learning | -86.1 |
| LC25 | female | 65 | ADC | 2 0 1 | IV | 1 | | None detected | 356·3 | PD | | | | PD | learning | -67.7 |
| LC26 | male | 64 | SCC | 3 3 1 | IV | 2 | | None detected | 405·6 | SD | PD | | | PD | test | -69.4 |
| LC27 | female | 65 | ADC | 4 2 1 | IV | 1 | | L858R (2819T>G) | | PD | | | | PD | test | -64.8 |
| LC28 | male | 74 | ADC | 2 1 1 | IV | 1 | 10 | | | PD | | | | PD | test | |
| mean | | 60(35-80) | | | | 2.3(1-5) | 49(0-90) | | 263·2(150·7-405·6) | | | | | | | |

(1) ADC, adenocarcinoma; SCC, squamous-cell carcinoma.
(2) TNM clinical classification and stage grouping were assessed based on the UICC/WHO classification.
(3) Mutation at codon position 709-870 (from p-loop to activation loop) of EGFR (GenBank Accession No. NM005228).
(4) Objective Tumor Response to Gefitinib was assessed every 4 weeks after the start of treatment using UICC/WHO Criteria, PR, partial response; SD, stable disease; PD, progressive disease
(5) Overall Best Response was evaluated based on the definitions as mentioned in materials and methods.
(6) learning, samples used for developing the GRS; test, samples used for validation of the GRS.
(7) GRS: gefitinib response score determined by prediction system
(*) For further validation of the GRS, another blinded set of samples from 5 newly enrolled cases (4 PD and 1 SD) were also added to these 28 cases later.

Table 4. List of 51 candidate genes for discriminating responder (PR) from non-responder (PD) to gefitinib (*)

| Rank Ort | GenBank | Symbol | Gene Name | Predominantly Expressed Clas | Permutational p-value | Median-fold Difference (log2; |
|---|---|---|---|---|---|---|
| 1 | NM_0240: | *FLJ2266*: | hypothetical protein FLJ22662 | PD | 8.1E-12 | 2.0 |
| 2 | BC009799 | *AREG* | amphiregulin (schwannoma-derived growth factor) | PD | 9.3E-12 | 8.0 |
| 3 | NM_0143: | *CORO1C* | coronin,actin binding protein, 1C | PD | 2.3E-10 | 4.6 |
| 4 | BC01048E | *AVEN* | apoptosis, caspase activation inhibitor | PD | 4.2E-10 | 4.3 |
| 5 | NM_0040! | *DUSP3* | dual specificity phosphatase 3 (vaccinia virus phosphatase VH1-rela | PD | 9.4E-10 | 4.4 |
| 6 | A1026836 | *DJ473B4* | hypothetical protein dJ473B4 | PD | 1.7E-09 | 8.0 |
| 7 | BU500509 | *PHLDA2* | pleckstrin homology-like domain. family A. member 2 | PD | 1.8E-09 | 8.0 |
| 8 | NM_0160! | *RBM7* | RNA binding motif protein 7 | PD | 1.8E-08 | 2.9 |
| 9 | BX092512 | | EST | PD | 7.7E-08 | 3.0 |
| 10 | A1436027 | *OSMR* | oncostatin M receptor | PD | 1.1E-07 | 3.7 |
| 11 | A19T1137 | *GCLC* | glutamale-cysteine ligase, catalytic subunit | PD | 1.2E-07 | 3.9 |
| 12 | B002487 | *COL4A3t* | collagen, type IV. alpha 3 (Goodpasture antigen) binding protein | PD | 2.0E-07 | 3.6 |
| 13 | U52522 | *ARFIP2* | ADP-ribosylation factor interacting protein 2 (arfaptin 2) | PD | 26E-07 | 2.8 |
| 14 | BM99605: | *C10orf9* | chromosome 10 open reading frame 9 | PD | 4.2E-07 | 2.5 |
| 15 | AK025452 | *NIP30* | NEFA-interacting nuclear protein NIP30 | PD | 5.1E-07 | 3.7 |
| 16 | N52048 | *KMA077t* | KIAA0776 protein | PD | 5.4E-07 | 7.2 |
| 17 | AA507009 | *SLC35F2* | solute carrier family 35, member F2 | PD | 6.0E-07 | 5.8 |
| 18 | AA226243 | *GAMLG* | calcium modulating ligan | PD | 6.8E-07 | 5.0 |
| 19 | AF005888 | *NOC4* | neighbor of COX4 | PD | 1.1E-06 | 4.0 |
| 20 | AF012281 | *PDZK1* | PDZ domain containing 1 | PD | 1.3E-06 | 4.5 |
| 21 | AI188190 | *DIS3* | mitotic control protein dis3 homolog | PD | 1.7E-06 | 3.8 |
| 22 | BC001535 | *CGI-48* | CGI-48 protein | PD | 20E-06 | 3.5 |
| 23 | NM_00701 | *CPSF6* | cleavage and polyadenylation specific factor 6.68kDa | PD | 22E-06 | 3.4 |

(continued)

| Rank Ort | GenBank | Symbol | Gene Name | Predominantly Expressed Clas | Permutational p-value | Median-fold Difference (log2; |
|---|---|---|---|---|---|---|
| 24 | NM_D022! | *KIF3C* | kinesin family member 3C | PD | 22E-06 | 3.5 |
| 25 | BQ135232 | *CD9* | CD9 antigen (p24) | PD | 22E-06 | 1.7 |
| 26 | BC051322 | *LRRC8* | leucine rich repeat containing 8 | PD | 25E-06 | 3.4 |
| 27 | BC03850 | *SNF1LK* | SNF1-like kinase | PD | 2.6E-06 | 2.8 |
| 28 | U78556 | *CRA* | cisplatin resistance associated | PD | 27E-06 | 3.7 |
| 29 | BC035625 | *EGR2* | early growth response 2 (Krox-20 homolog, Drosophila) | PD | 3.4E-06 | 3.0 |
| 30 | X52426 | *KRT13* | keratin 13 | PD | 1.9E-05 | 3.4 |
| 31 | NM_0055 | *BCAT1* | branched chain aminotransferase 1, cytosofic | PD | 2.3E-05 | 1.7 |
| 32 | NM_D066 | *SDCCAG* | serologically defined colon cancer antigen 3 | PR | 2.6E-05 | 3.7 |
| 33 | AA464095 | *PIGK* | phosphatidylinositol glycan, class K | PD | 3.2E-05 | 1.1 |
| 34 | AA96118E | *MRPS9* | mitochondrial ribosomal protein S9 | PD | 9.8E-05 | 2.3 |
| 35 | NM_0181: | *ASPM* | asp (abnormal spindle)-like, microcephaly associated (Drosophila) | PR | 23E-04 | 2.8 |
| 36 | NM_0227: | *ACBD3* | acyl-Coenzyme A binding domain containing 3 | PD | 24E-04 | 3.8 |
| 37 | AA160544 | *ZNF325* | zinc finger protein 325 | PR | 27E-04 | 4.5 |
| 38 | AK057653 | *LOC2855* | hypothetical protein LOC285513 | PD | 27E-04 | 3.8 |
| 39 | NM_0033 | *TSSC1* | tumor suppressing subtransferable candidate 1 | PD | 2.9E-04 | 4.7 |
| 40 | BCD07451 | *XAB1* | XPA binding protein 1 | PD | 3.0E-04 | 1.3 |
| 41 | BC035467 | *HNLF* | putative NFkB activating protein HNLF | PR | 3.5E-04 | 1.1 |
| 42 | CK004097 | *EIF4EBP* | eukaryotic translation initiation factor 4E binding protein 2 | PR | 3.6E-04 | 1.4 |
| 43 | NM_1446I | *MGC232*: | hypothetical protein MGC23280 | PR | 4.2E-04 | 2.3 |
| 44 | NM_00461 | *SSA2* | Sjogren syndrome antigen A2 (60kDa, ribonucleoprotein autoantige | PR | 4.2E-04 | 1.2 |
| 45 | NM_0027: | *PRKACA* | protein kinase, cAMP-dependent, catalytic, alpha | PR | 5.0E-04 | 1.2 |

(continued)

| Rank Ort | GenBank | Symbol | Gene Name | Predominantly Expressed Clas | Permutational p-value | Median-fold Difference (log2; |
|---|---|---|---|---|---|---|
| 46 | NM_0051I | *FEE2* | fasciculation and elongation protein zeta 2 (zygin II) | PD | 6.1E-04 | 3.3 |
| 47 | NM_0058: | *SRRM1* | serine/arginine repetitive matrix 1 | PR | 7.0E-04 | 1.4 |
| 48 | NM_0062I | *PDGFRL* | platelet-derived growth factor receptor-like | PD | 7.0E-04 | 2.4 |
| 49 | AI096936 | *SNX13* | sorting nexin 13 | PR | 8.4E-04 | 1.6 |
| 50 | NM_0147I | *KIAA025I* | KIAA0258 gene product | PD | 8.9E-04 | 2.5 |
| 51 | BF973104 | *TOM7* | homolog of Tom7 (S. cerevisiae) | PR | 1.0E-03 | 1.5 |
| (*) The 12 and 51 gene sets were listed as the rank-order of permutational ρ-values that were less than 0.001. | | | | | | |

| Table 4A List of 12 Candidate Genes for Discriminating Responder (PR) from Non-responder (PD) to Gefitinib | | | | |
|---|---|---|---|---|
| | | | | |
| Rank Order | Gene Bank ID | Gene Symbol | Gene Name | Nucleotides |
| | 1 NM_024829 | *FLJ22662* | hypothetical protein FLJ22662 | ATACGGCATCCATGAAATATAT CATGCGATACAACAATTATAAG AAGGATCCTTACAGTAGAGGTG ACCCCTGTAATACCATCTGCTG CCGTGAGGACCTGAACTCACCT AACCCAAGTCCTGGAGGTTGTT ATGACACAAAGGTGGCAGATAT CTACCTAGCATCTCAGTACACA TCCTATGCCATAAGTGGTCCCA CAGTACAAGGTGGCCTCCCTGT TTTTCGCTGGGACCGTTTCAAC AAAACTCTACATCAGGGCATGC CAGAGGTCTACAACTTTGATTT TATTACCATGAAACCAATTTTG AAACTTGATATAAAATGAAGGA GGGAGATGACGGACTAGAAGAC |

(continued)

| Rank Order | Gene Bank ID | Gene Symbol | Gene Name | Nucleotides |
|---|---|---|---|---|
| | 2 BC009799 | *AREG* | amphiregulin (schwannoma-derived growth factor) | CTCCACTCGCTCTTCCAACACC<br>CGCTCGTTTTGCGGCAGCTCGT<br>GTCCCAGAGACCGAGTTGCCCC<br>AGAGACCGAGACGCCGCCGCTG<br>CGAAGGACCAATGAGAGCCCCG<br>CTGCTACCGCCGGCGCCGGTGG<br>TGCTGTCGCTCTTGATACTCGG<br>CTCAGGCCATTATGCTGCTGGA<br>TTGGACCTCAATGACACCTACT<br>CTGGGAAGCGTGAACCATTTTC<br>TGGGGACCACAGTGCTGATGGA<br>TTTGAGGTTACCTCAAGAAGTG<br>AGATGTCTTCAGGGAGTGAGAT<br>TTCCCCTGTGAGTGAAATGCCT<br>TCTAGTAGTGAACCGTCCTCGG<br>GAGCCGACTATGACTACTCAGA<br>AGAGTATGATAACGAACCACAA<br>ATACCTGGCTATATTGTCGATG<br>ATTCAGTCAGAGTTGAACAGGT<br>AGTTAAGCCCCCCCAAAACAAG<br>ACGGAAAGTGAAAATACTTCAG<br>ATAAACCCAAAAGAAAGAAAAA<br>GGGAGGCAAAAATGGAAAAAAT<br>AGAAGAAACAGAAAGAAGAAAA<br>ATCCATGTAATGCAGAATTTCA<br>AAATTTCTGCATTCACGGAGAA<br>TGCAAATATATAGAGCACCTGG<br>AAGCAGTAACATGCAAATGTCA<br>GCAAGAATATTTCGGTGAACGG<br>TGTGGGGAAAAGTCCATGAAAA<br>CTCACAGCATGATTGACAGTAG<br>TTTATCAAAAATTGCATTAGCA<br>GCCATAGCTGCCTTTATGTCTG<br>CTGTGATCCTCACAGCTGTTGC |
| | 3 NM_014325 | *CORO1C* | coronin, actin binding protein 1C | GATAGGCCACATTCCAGTAAGA<br>ACTCAATTTGTCTCCCAAATTT<br>GCAGAAACAAAACGTGATTTAA<br>AAGCTGAGCTTTTTATCAGAAA<br>GCTTTTTTGATGTTTTAAGTGT<br>TATGTGACTTGTTGAACTTTTT<br>AAAAAGTGCTACTTTTAAAATC<br>CCAGATACTCTGAATTTTAGAA<br>AACAAACTAATTCTGATTGTGT<br>CGTGCCCAAGTACCCTTTTTTT<br>TTTAATGAGTAGGGACCAATGC<br>CACATTGCTTTTTATATTTCTT<br>TCTTTTTTAATGTTGCCAAAAC<br>CAAAAGTAGCTTTGTTTTCCTT<br>TGTATTTTGCTACTTTGCAGTA<br>TTTGTGTGTGTGGTTTTTTTTC<br>CTTAATTTGAAAGGGACAGCAC<br>TGTGTATGTTTA |

(continued)

| Rank Order | Gene Bank ID | Gene Symbol | Gene Name | Nucleotides |
|---|---|---|---|---|
| | 4 BC010488 | *A VEN* | apoptosis, caspase activation inhibitor | AGGAGACCATTTGGAAGAAGAA<br>CTAGATCTGTTGCTTAATTTAG<br>ATGCACCTATAAAAGAGGGAGA<br>TAACATCTTACCAGATCAGACG<br>TCTCAGGACCTGAAATCCAAGG<br>AAGATGGGGAGGTGGTCCAAGA<br>GGAAGAAGTTTGTGCAAAACCA<br>TCTGTGACTGAAGAAAAAAACA<br>TGGAACCTGAGCAACCAAGTAC<br>CTCCAAAAATGTTACCGAGGAA<br>GAGCTGGAAGACTGGTTGGACA<br>GCATGATTTCCTAAAAAGGGGA<br>AAAAAAGTGCCTGAAGCAAATC<br>TTGGTTGCCTTCTAACGGCAGG<br>TGGGCATAAGGCTGTCCTTCAG<br>GACCAGCCAGTTTACAAGCATG<br>TCTCAAGCTAGTGTGTTCCATT<br>ATGCTCACAGCAGTAAATGCCT<br>ACCTCTGTGTTTGACATCTGAA<br>AGAATACATTGAAGCAGCTTGT<br>TGCATTTGTTTTTCTGGCTTAG<br>TAATCTAATAGATTTCCTTAAG<br>GGCAGGAGATAGACTCTGGCCC<br>TTGTTTCTAGCCTCCTTCCTTG<br>CAGTGTTTACAACATAGCCAGT<br>GTTTACAGCATAGCA |
| | 5 NM_004090 | *DUSP3* | dual specificity phosphatase 3 (vaccinia virus phosphatase VH1-related) | GGATCCTTTATTGGTGGTAGAG<br>CAAAAAAACCCAAACACGATAA<br>ACCTTTCAAAAGACTTTCTAAG<br>GATGATATTGGAATGCACCAGC<br>CCTCACATGTGTATGCACATTT<br>GCCAGAATATAAGAGTTTTGTT<br>TTAAATACAGTCTTGTTAGGAT<br>TTTACGTTATTGTTATTATGGA<br>AAGTGATTGTGATGCTATTTAT<br>CTTCAGGGTCACTCTGG |
| | 6 AI026836 | *DJ473B4* | hypothetical protein dJ473B4 | GCAGTCGTTTCAACCAGGTAGT<br>TTTGGGTTGTTTTTAAAGCCCT<br>TTTGAGGTCTTACACATTATTA<br>ACTTTAAAATAATCAGGCAGCT<br>AAGAATAATTACTAGAAAAATC<br>ATCTACCACTTCAAACATGGTC<br>AACTACTTCAAAACTGCACCTA<br>GAGAATCAGGTACCTGAAGTAG<br>AACAAGAAGCCTGGAGGTGGAC<br>TTTGAGAGGAGGGAATACCC |

(continued)

| Rank Order | Gene Bank ID | Gene Symbol | Gene Name | Nucleotides |
|---|---|---|---|---|
| | 7 BU500509 | *PHLDA2* | pleckstrin homology-like domain, family A, member 2 | TACGTGTACTTCACCATCGTCA<br>CCACCGACCACAAGGAGATCGA<br>CTTCCGCTGCGCGGGCGAGAGC<br>TGCTGGAACGCGGCCATCGCGC<br>TGGCGCTCATCGATTTCCAGAA<br>CCGCCGCGCCCTGCAGGACTTT<br>CGCAGCCGCCAGGAACGCACCG<br>CACCCGCCGCACCCGCCGAGGA<br>CGCCGTGGCTGCCGCGGCCGCC<br>GCACCCTCCGAGCCCTCGGAGC<br>CCTCCAGGCCATCCCCGCAGCC<br>CAAACCCCGCACGCCATGAGCC<br>CGCCGCGGGCCATACGCTGGAC<br>GAGTCGGACCGAGGCTAGGACG<br>TGGCCGGCGCTCTCCAGCCCTG<br>CAGCAGAAGAACTTCCCGTGCG<br>CGCGGATCCTCGCTCCGTTGCA<br>CGGGCGCCTTAAGTTATTGGAC<br>TATCTAATATCTATGTATTTAT<br>TTCGCTGGTTCTTTGTAGTCAC<br>ATATTTTATAGTCTTAATATCT<br>TGTTTTTGCATCACTGTGCCCA<br>TTGCAAATAAATCACTTGGCCA<br>GTTTGCTTTTCTACCATCC |
| | 8 NM_16090 | *RBM7* | RNA binding motif protein 7 | CTGTGACATGCTCTTGAGCTTT<br>ACCCTAGTTGAACATACATGTG<br>TAGATTTACACATACTGTTTCA<br>TTNNNNAATTTAGAAATTGTTC<br>ATTAAATCCCATTTGAGGTATA<br>AGTCACTCAGGAAGTTAAAATA<br>TCTCTACACGTATATTTTTACA<br>TTAAAAATACAGTGTTAGCATA<br>ANNNNCCCTTTNNNNNGAAGAA<br>CAAAAATGTCAGTGCATAGTTA<br>GATAAAATGGTAAAATGTTTTA<br>CTGAAAGCATACTTTTTTGGAA<br>AATAGATTCATGAAGCCTTTAA<br>GTGCTGCTTCTGTCAGTCAAAC<br>GTTAAAAACTTTAACATTTTCA<br>AAGTGCCCAGACTGTGTACAAA<br>GACACATGTAATGGAGATTGTA<br>CAGGTTGTTTTTTTGTTTGAAC<br>CTTTGAAAGAGTTTAATCTTAA<br>CGTTTTCTAATTTTAAAATTTT<br>AAAATCTTGTTTAACAAAAGCT<br>TGTATTAAGATACTGTTTTCAT<br>TTCATTACAGAATTGTTTATAA<br>AAGTTCATTTGTTGAAAANNNA<br>GGATCCTTTTTAATACCACAGC<br>ATTTGTACTGTTCCT |

(continued)

| Rank Order | Gene Bank ID | Gene Symbol | Gene Name | Nucleotides |
|---|---|---|---|---|
| | 9 BX092512 | | EST | ATATGTGCACACACACACTCACACCCACACCCATAAAGATTTTGCACTCCTTGAAGGTACACTAACTCACCATTTTTATCATACTTATCCCAGTGTGCCACAGTTACTGGCTTATATGCCTGTCTCTGCTATCTTATTTTATCTGTCTCCACAACACAGCAAACTACCTGGCCTTCAATAAAGGGCTTATGAATTATTCATGAATCCATTTTGCCAGGTGCCTAGCCCTGTGTCTGGCTTGAAGCAGGTGTTCCCAAGGTGTGGCATGGCTGAGTGAATACAAAT |
| | 10 A1436027 | *OSMR* | oncostatin M receptor | CACCAATGAGCTTACTACCCAACTTCAAAACTAGGACTCTAACAATAACTTCTGTCATATCTCATCCTGTAACGCCCCCACCTTCGCTCCTTCCGCCAAGATAATTATCACTTTAAATTGTGTGCGTGTGTATTCTCATTTCTTATGTGATGGTAAAAATGCCTTTATTTTGTTTGGTTTTAATGCATAGAAAGGACATCAAGCTGT |
| | 11 AI971137 | *GCLC* | glutamate-cysteine ligase, catalytic subunit | CTCTAAAAGCCATTCACTCCAGATTTTACCTGGGGAATATTCTACATACTGCTTACTTTCTCTATAAAACTCATCAATAAATCATGAAAGGCACTGAGTTTTGTAAATCAGGACCCTAAATGTTTAATTGTAAATAAGTTTCAGATAATTATTATAGCTTTGCGTTGAAGTTNNNNNNNNNTTTTTCTCAACTAGTTAAGTCAACTGCTTCTGAAATAACTCTGTATTGTAGATTATGCAGATCTTTACAGGCATAAATATTTAAACTGTAATATGCTAACTTGAAGAGATTGCAATAAAGCTGCTTCAGCTAAC |

(continued)

| Rank Order | Gene Bank ID | Gene Symbol | Gene Name | Nucleotides |
|---|---|---|---|---|
| | 12 BQ024877 | *COL4A3BP* | collagen, type IV, alpha 3 (Goodpasture antigen) binding protein | CTCACTGAAGTTGAAATGACTG CCCACTTCAAAATCTTCATTGT GTTTACACACCAGTGTATTTAT ACAAATCAGAGGCATTTTGTAG ATGCTTTGCTGACTTGTTCAGC TCTGTAAAAACACAGAAATCAG ACCCATTTTGTAAAGCGGAAAA TCATGTTACATGGAACATGTCC TGTATATATCACATACATGGTA ATGGAGTCTTAATGATAAGTGC AAGATAATAATTTAATGATGGG ATTAGTCTGATCGCTTAATATG CACAATCCTGGAAGTGAATTAC TTGCATCAGATATAGTGATATT TATTATTCTGTACAGAGAGAAA AATACATATAAAACATATGCTT ACATTACATGCACGCGGATTTC ATGCTCCATAATCTTTTCTATT TTTTAATTTACCTTTCTGTAAA TGATGTGCATGGAATATGCCTT ATAGAAAAAATGCTGTTCATAAT TTGACTACGTGGAAAAGTGCCT ATATGGTGGTAATGCTAGTAAG GCA |

Table 5A: Correlation of cDNA microarray data with semi-quantatative RT-F

| | | Spearman rank correlation | |
|---|---|---|---|
| Rank Order | Gene Symbol | p | p-value |
| 1 | *FLJ22662* | 0.69 | 0.02 |
| 2 | *AREG* | 0.53 | 0.08 |
| 3 | *CORO1C* | 0.35 | 0.24 |
| 4 | *AVEN* | 0.63 | 0.04 |
| 5 | *DUSP3* | 0.63 | 0.04 |
| 6 | *DJ473B4* | 0.45 | 0.14 |
| 7 | *PHLDA2* | 0.84 | 0.01 |
| 8 | *RBM7* | 0.83 | 0.01 |
| 9 | *EST(BX092512)* | 0.63 | 0.04 |
| 10 | *OSMR* | 0.67 | 0.03 |
| 11 | *GCLC* | 0.46 | 0.13 |
| 12 | *COL4A3BP* | 0.27 | 0.24 |
| Correlations positive for all 12 genes and significantly positive for 7 of 12 ge | | | |

Table 5B: Result of immunohistochemical staining

| | PR | PD |
|---|---|---|
| AREG | 1/5 | 5/6 |
| TGFA | 2/5 | 6/6 |
| ADAM9 | 1/5 | 4/6 |

(continued)

| | PR | PD |
|---|---|---|
| CD9 | 2/5 | 5/6 |
| OSMR | 2/5 | 6/6 |

**SUMMARY PARAGRAPHS** - The present invention is defined in the claims and the accompanying description. For convenience other aspects of the present description are presented herein by way of numbered paragraphs (para.s).

1. A set of isolated marker genes comprising at least one gene identified as having differential expression as between patients who are responders and non responders to an erbB receptor tyrosine kinase inhibitor; said gene set comprising one or more genes selected from at least the group consisting of the 51 genes listed in Table 4 herein including gene-specific oligonucleotides derived from said genes.
2. A set according to paragraph I comprising at least one or more of the first 40 genes listed in Table 4 herein.
3. A set according to paragraph 1 comprising at least one or more of the first 20 genes listed in Table 4 herein.
4. A set according to paragraph 1 comprising at least one or more of the first 12 genes listed in Table 4 herein.
5. A set according to paragraph 1 comprising at least one or more of the first 5 genes listed in Table 4 herein.
6. A set according to para. 1 which is first 12 genes listed in Table 4 herein, namely the genes FLJ22622 (e.g. GenBank NM_024829), AREG (e.g. GenBank BC009799), C0R01C (e.g. GenBank NM_014325), AVEN (e.g. GenBank BC010488), DUSP3 (e.g. GenBank NM_004090, DJ473B4 (e.g. GenBank AI026836), PHLDA2 (e.g. GenBank BU500509), RBM7 (e.g. GenBank NM_0106090), EST (GenBank BX0952512), OSMR (e.g. GenBank AI436027), GCLC (e.g. GenBank AI971137), COL4A3BP (e.g. GenBank BQ024877).
7. A set according to para. 6, wherein the genes somprise the sequences set forth in Table 4a.
8. A set according to paragraph 6, wherein the set comprises gene-specific oligonucleotides, said oligonucleotides comprising 5 to 50 nucleotides of the sequences set forth in Table 4a.
9. A set according to any preceding paragraph wherein the inhibitor is selected from gefitinib, OSI-774, PKI-166, EKB-569, GW2016 and CI-1033.
10. A set according to paragraph 9 wherein the agent is gefitinib.
11. A set according to any of paragraphs 1 to 8 wherein the inhibitor is an anti-erbB antibody.
12. A set according to paragraph 11 wherein the antibody is trastuzumab or cetuximab.
13. A method of predicting the responsiveness of a patient or patient population with cancer to treatment with an erbB receptor kinase inhibitor, or for selecting patients or patient populations that will respond to an erbB receptor kinase inhibitor comprising comparing the differential expression of one or more marker genes, said marker genes selected from the gene sets as defined in any one of paragraphs 1 to 6.
14. A method according to paragraph 13 wherein the responsiveness of the patients is represented by the generation of a Drug Response Score.
15. A method according to paragraphs 13 or 14 wherein the comparison is performed by microarray assay.
16. A method according to paragraph 13 or paragraph 14, wherin the comparison is performed by immunohistochemistry.
17. A method according to paragraph 16, said method comprising detecting the differential expression of amphiregulin.
18. A method according to any of paragraphs 13 to 17 wherein the inhibitor is as defined in any of paragraphs 9 to 12.
19. A diagnostic kit for use in the method of paragraph 13 to 18 comprising a marker gene set selected from the group as defined in any one of paragraphs 1 to 8 on a suitable support medium.
20. A kit according to paragraph 19 which comprises a microarray.
21. A method of treating a patient with cancer comprising administering an inhibitor according to any of paragraphs 9 to 12 and testing the differential expression of a set of marker genes, said set selected from the group as defined in any one of paragraphs 1 to 8.
22. The use of an isolated gene sequence, selected from the group consisting of the 51 genes listed in Table 4 herein including gene-specific oligonucleotides derived from said genes to measure the expression level of said gene in a tissue sample from a patient having NSCLC.
23. A diagnostics kit comprising means for determining the level of expression, of one or more genes selected from selected from the group consisting of the 51 genes listed in Table 4 herein including gene-specific oligonucleotides derived from said genes in a tissue sample from a NSCLC patient, comprising a support material comprising a set of isolated marker genes, as defined in any of paragraphs 1 to 8, at least one gene thereof attached thereto.
24. The use of an inhibitor as defined in any of paragraphs 9 to 12 in the treatment of NSCLC patents identified according to the method of paragraphs 13.

25. A method of treating patents or patient populations having NSCLC identified according to the method of any one of paragraphs 13 to 17 comprising administering to said patients an erbB receptor tyrosine kinase inhibitor.
26. Use of an erbB receptor tyrosine kinase inhibitor in the manufacture of a medicament for the treatment of patients or patient populations having NSCLC identified according to the method of any one of paragraphs 13 to 17.
27. A method of testing, or testing for, an erbB tyrosine kinase receptor inhibitor comprising treating the patient and assessing if the compound modulates gene expression of at least one of the gene from the marker gene set according to any of paragraphs 1 to 8 relative to a relevant control.
28. A method of carrying out a clinical trial to measure the effect or effectiveness of erbB receptor tyrosine kinase inhibition or inhibitors comprising measuring the relative levels of expression of a gene set as defined in any of paragraphs 1- 8 in a patient or patient population.

## Claims

1. An *in vitro* method of predicting the responsiveness of a patient with cancer to the treatment with an erbB receptor kinase inhibitor, or for selecting a patient that will respond to an erbB receptor kinase inhibitor, comprising comparing the differential expression of the AREG gene.

2. The method according to claim 1, wherein the comparison is performed by microarray assay.

3. The method according to claim 1, wherein the comparison is performed by immunohistochemistry.

4. The method according to any one of claims 1 to 3, wherein the inhibitor is selected from the group consisting of gefitinib, OSI-774, PKI-166, EKB-569, GW2016, CI-1033, an anti-erbB antibody, trastuzumab and cetuximab.

## Patentansprüche

1. In-vitro-Verfahren zur Vorhersage der Ansprechbarkeit eines an Krebs leidenden Patienten auf die Behandlung mit einem erbB-Rezeptorkinase-Inhibitor oder zur Auswahl eines Patienten, der auf einen erbB-Rezeptorkinase-Inhibitor ansprechen wird, wobei man die unterschiedliche Expression des AREG-Gens vergleicht.

2. Verfahren nach Anspruch 1, wobei der Vergleich mittels Mikroarray-Test erfolgt.

3. Verfahren nach Anspruch 1, wobei der Vergleich mittels Immunhistochemie erfolgt.

4. Verfahren nach einem der Ansprüche 1 bis 3, wobei der Inhibitor aus der aus Gefitinib, OSI-774, PKI-166, EKB-569, GW2016, CI-1033, einem Anti-erbB-Antikörper, Trastuzumab und Cetuximab bestehenden Gruppe ausgewählt ist.

## Revendications

1. Procédé *in vitro* de prédiction de la réactivité d'un patient présentant un cancer au traitement avec un inhibiteur de la kinase du récepteur erbB, ou pour la sélection d'un patient qui va réagir à un inhibiteur de la kinase du récepteur erbB, comprenant la comparaison de l'expression différentielle du gène AREG.

**2.** Procédé selon la revendication 1, dans lequel la comparaison est réalisée par un essai sur micro-réseau.

**3.** Procédé selon la revendication 1, dans lequel la comparaison est réalisée par immunohistochimie.

**4.** Procédé selon l'une quelconque des revendications 1 à 3, dans lequel l'inhibiteur est choisi dans le groupe consistant en le géfitinib, l'OSI-774, le PKI-166, l'EKB-569, le GW2016, le CI-1033, un anticorps anti-erbB, le trastuzumab et le cétuximab.

# Figure 1

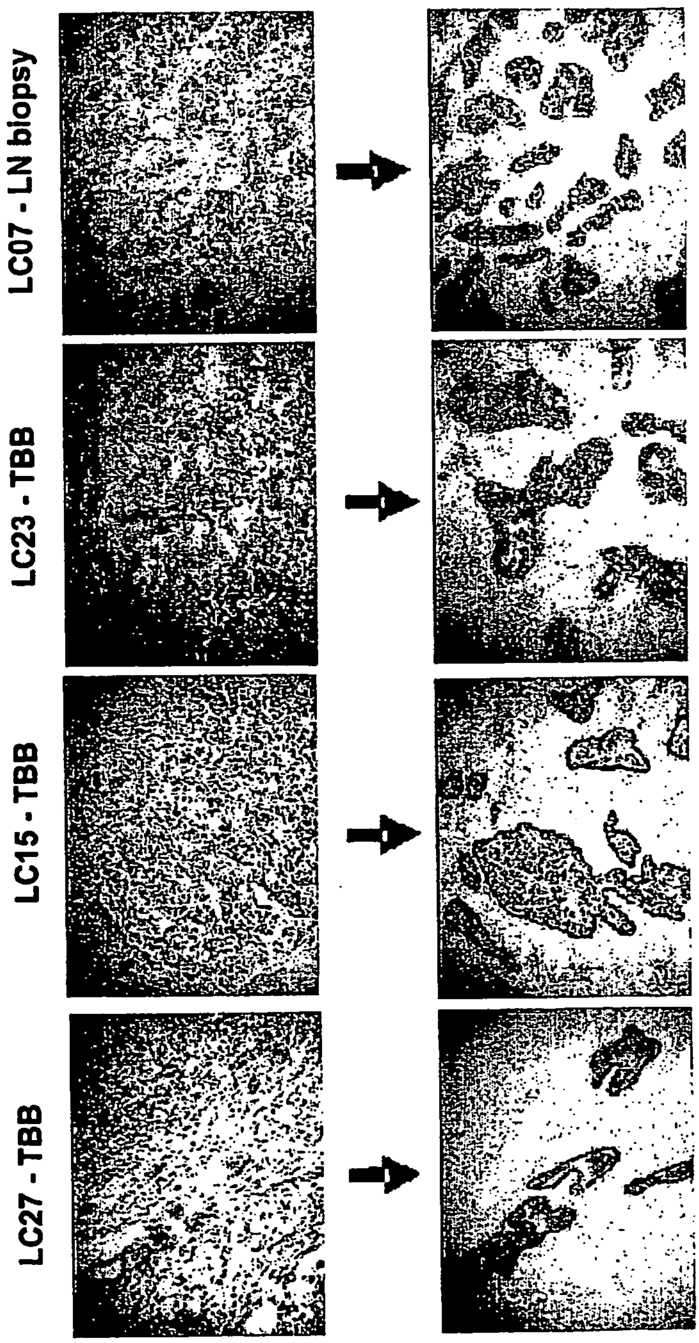

# Figure 2

A

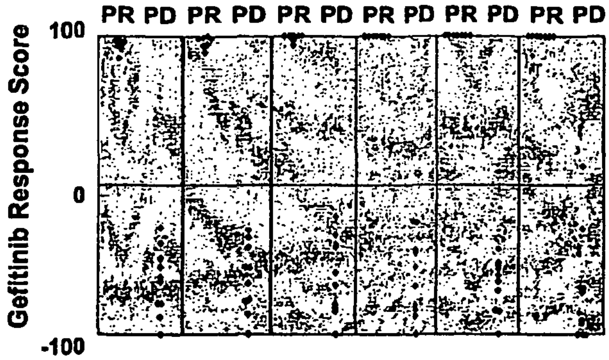


| | | | | | | |
|---|---|---|---|---|---|---|
| Number of Discriminating Gene | 51 | 40 | 30 | 20 | 12 | 5 |
| Classification Score | 5.94 | 5.93 | 5.64 | 6.08 | 9.48 | 4.67 |

B

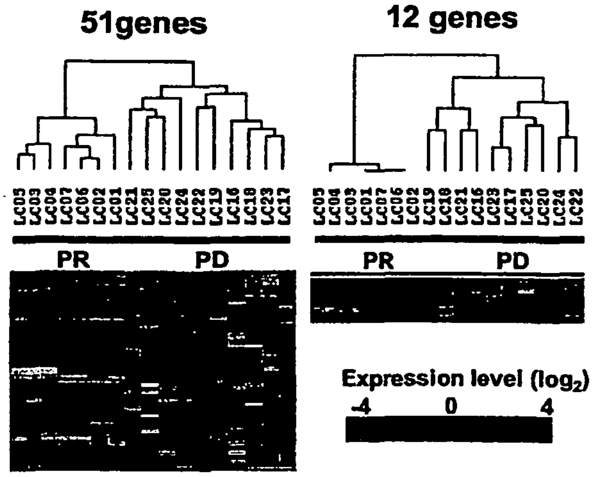

# Figure 2 cont.

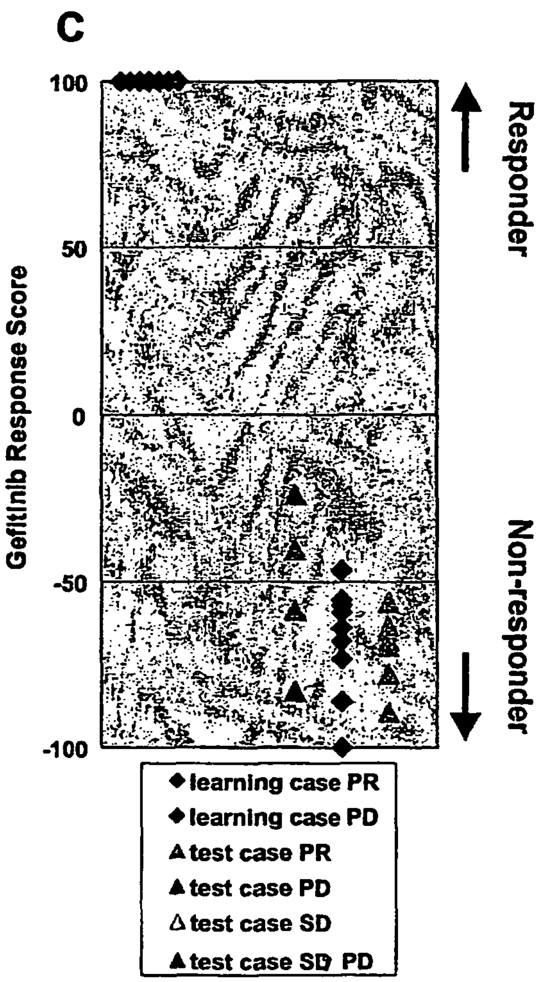

# Figure 3

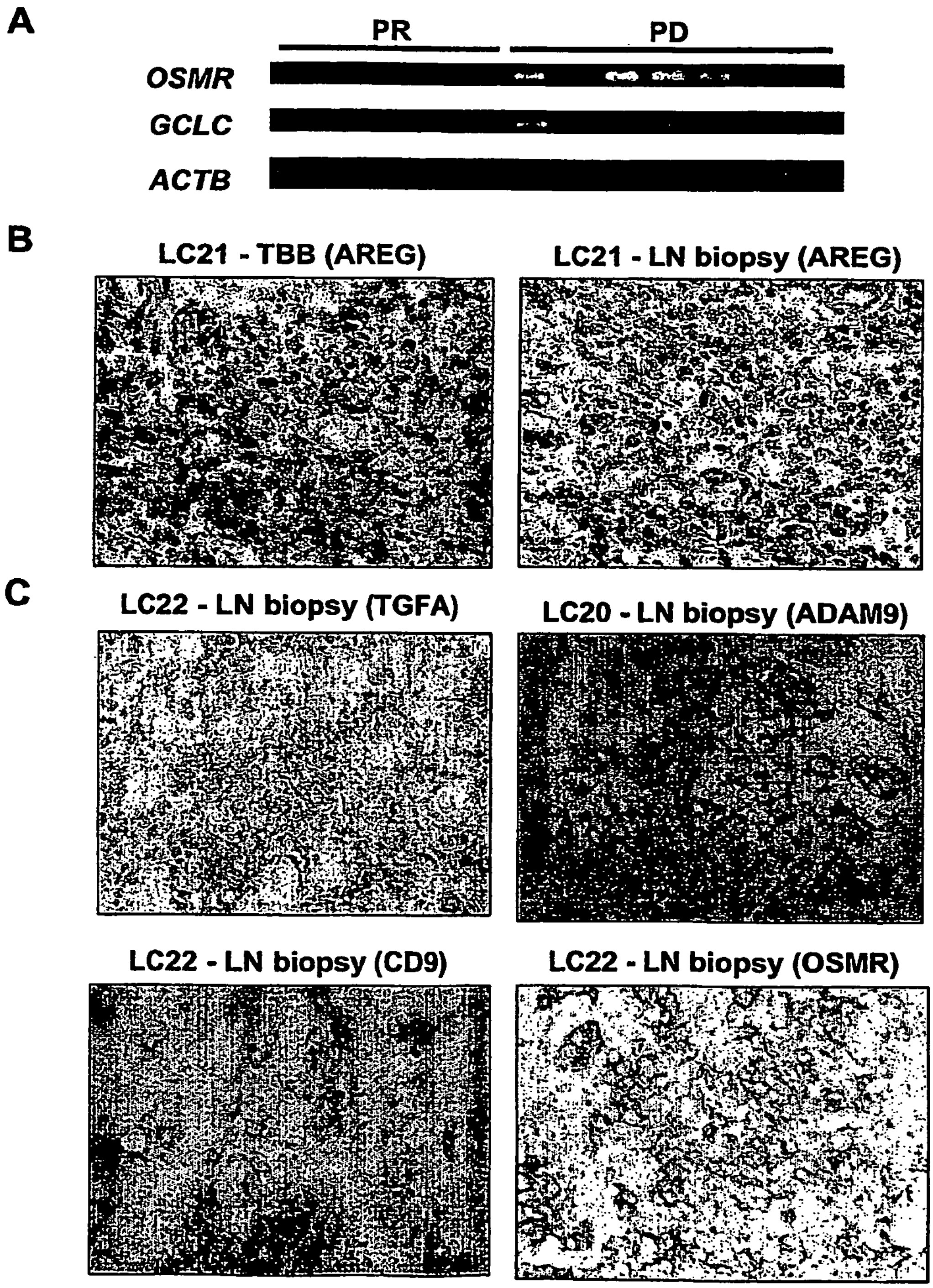
A
PR
PD
OSMR
GCLC
ACTB
B
LC21 - TBB (AREG)
LC21 - LN biopsy (AREG)
C
LC22 - LN biopsy (TGFA)
LC20 - LN biopsy (ADAM9)
LC22 - LN biopsy (CD9)
LC22 - LN biopsy (OSMR)

# Figure 4

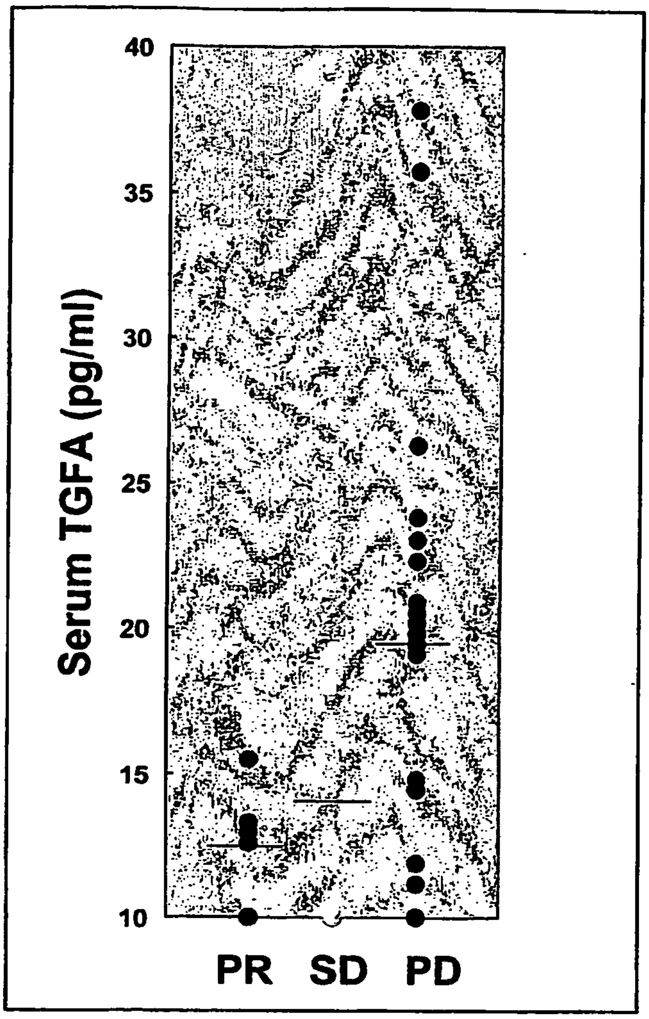

# Figure 5

A

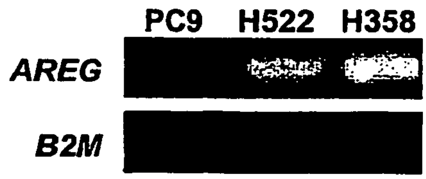
PC9 H522 H358
AREG
B2M

B

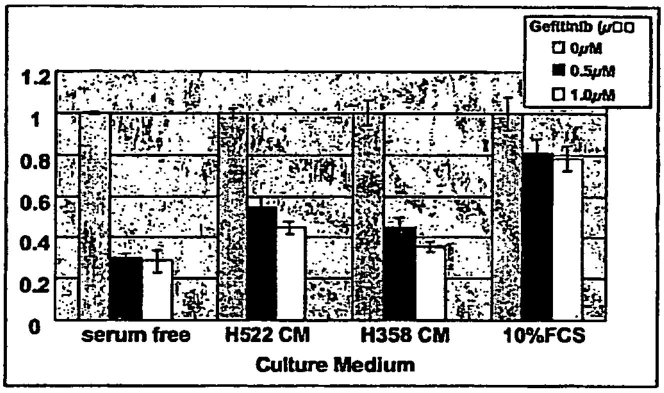
Gefitinib
0μM
0.5μM
1.0μM
1.2
1
0.8
0.6
0.4
0.2
0
serum free
H522 CM
H358 CM
10%FCS
Culture Medium

C

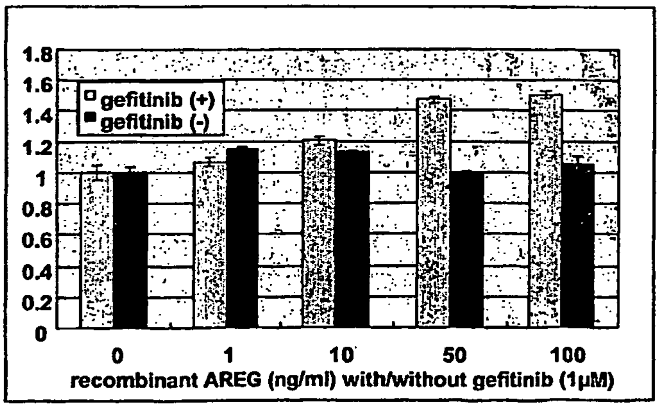
1.8
1.6
1.4
1.2
1
0.8
0.6
0.4
0.2
0
gefitinib (+)
gefitinib (-)
0
1
10
50
100
recombinant AREG (ng/ml) with/without gefitinib (1μM)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0566226 A
- WO 9633980 A
- WO 9730034 A
- WO 9630347 A
- WO 9955683 A
- WO 9738983 A
- WO 0031048 A
- WO 9702266 A
- EP 0787722 A
- WO 9850038 A
- WO 9909016 A
- WO 9924037 A
- US 6002008 A
- WO 9935146 A
- WO 0104111 A
- US 0110063 W
- US 2002090979 A
- US 5837832 A
- GB 0312451 A
- GB 0322636 A
- GB 0327132 A


### Non-patent literature cited in the description


- **WILKS.** *Advances in Cancer Research,* 1993, vol. 60, 43-73
- **OLAYIOYE et al.** *EMBO J.,* 2000, vol. 19, 3159
- **KLAPPER et al.** *Adv. Cancer Res.,* 2000, vol. 77, 25
- **CERNY et al.** *Brit. J. Cancer,* 1986, vol. 54, 265
- **REUBI et al.** *Int. J. Cancer,* 1990, vol. 45, 269
- **RUSCH et al.** *Cancer Research,* 1993, vol. 53, 2379
- **BRABENDER et al.** *Clin. Cancer Res.,* 2001, vol. 7, 1850
- **HENDLER et al.** *Cancer Cells,* 1989, vol. 7, 347
- **BRABENDER et al.** *Clin. Cancer Res.,* 2007, vol. 7, 1850
- **ROSS et al.** *Cancer Investigation,* 2001, vol. 19, 554
- **YU et al.** *Bioessays,* 2000, vol. 22.7, 673
- **MENDELSOHN et al.** *Oncogene,* 2000, vol. 19, 6550
- **YAISH et al.** *Science,* 1988, vol. 242, 933
- **KOLIBABA et al.** *Biochimica et Biophysica Acta,* 1997, vol. 133, F217-F248
- **AL-OBEIDI et al.** *Oncogene,* 2000, vol. 19, 5690-5701
- **MENDELSOHN et al.** *Oncogene,* 2000, vol. 19, 6550-6565
- **J R WOODBUM et al.** *Proc. Amer. Assoc. Cancer Research,* 1997, vol. 38, 633
- *Pharmacol. Ther.,* 1999, vol. 82, 241-250
- *CHEMICAL ABSTRACTS,* 184475-35-2
- *J.Med. Chem.,* 1999, vol. 42, 1803-1815
- *Nature Medicine,* 2000, vol. 6, 1024-1028
- **VELCULESCU et al.** *Science,* vol. 270 (5235), 484-487
- **SAMBROOK et al.** Molecular Cloning: A Laboratory Manual. 1989
- **LEMIEUX et al.** *Molecular Breeding,* 1998, vol. 4, 277-289
- Parallel Analysis with Biological Chips. **SCHENA ; DAVIS.** PCR Methods Manual
- Genes, Genomes and Chips. **SCHENA ; DAVIS.** DNA Microarrays: A Practical Approach. Oxford University Press, 1999
- The Chipping Forecast (Nature Genetics special issue; January 1999 Supplement. Microarray Biochip Technology. Eaton Publishing Company, January 1999
- **CORTES.** *The Scientist,* 2000, vol. 14 (17), 25
- **GWYNNE ; PAGE.** Microarray analysis: the next revolution in molecular biology. *Science,* 06 August 1999
- **EAKINS ; CHU.** *Trends in Biotechnology,* 1999, vol. 17, 217-218
- **CELIS et al.** *FEBS Lett,* 2000, vol. 480 (1), 2-16
- **LOCKHART ; WINZELER.** *Nature,* 2000, vol. 405 (6788), 827-836
- **MARX.** *Science,* 2000, vol. 289, 1670-1672
- **SCHERF et al.** *Nat Genet,* 2000, vol. 24 (3), 236-44
- **ROSS et al.** *Nat Genet.,* March 2000, vol. 24 (3), 227-35
- **WANG et al.** *Science,* 1998, vol. 280 (5366), 1077-82

- *Chemical & Engineering News,* 22 February 1999, vol. 77 (8), 27-36
- **ROCKETT ; DIX.** *Xenobiotica,* 2000, vol. 30 (2), 155-77
- **AFSHARI et al.** *Cancer Res1,* 1999, vol. 59 (19), 4759-60
- **NUWAYSIR et al.** *Molecular Carcinonucleotide sequencesis,* 1999, vol. 24, 153-159
- **CORTESE.** *The Scientist,* 2000, vol. 14 (11), 26
- **SHALON et al.** *Genome Res,* 1996, vol. 6 (7), 639-45
- **J. SAMBROOK ; E. F. FRITSCH ; T. MANIATIS.** Molecular Cloning: A Laboratory Manual. Cold Spring Harbor Laboratory Press, 1989
- **AUSUBEL, F. M. et al.** Current Protocols in Molecular Biology. John Wiley & Sons, 1995
- **B. ROE ; J. CRABTREE ; A. KAHN.** DNA Isolation and Sequencing: Essential Techniques. John Wiley & Sons, 1996
- Oligonucleotide Synthesis: A Practical Approach. Irl Press, 1984
- **D. M. J. LILLEY ; J. E. DAHLBERG.** Methods of Enzymology: DNA Structure Part A: Synthesis and Physical Analysis of DNA Methods in Enzymology. Academic Press, 1992
- **FOSSELLA, F.V. et al.** Randomized phase III trial of docetaxel versus vinorelbine or ifosfamide in patients with advanced non-small-cell lung cancer previously treated with platinum-containing chemotherapy regimens. The TAX 320 Non-Small Cell Lung Cancer Study Group. *J Clin Oncol,* 2000, vol. 18 (12), 2354-62
- Non-small Cell Lung Cancer Collaborative Group. Chemotherapy in non-small cell lung cancer: a meta-analysis using updated data on individual patients from 52 randomised clinical trials. *Bmj,* 1995, vol. 311 (7010), 899-909
- **SCHILLER, J.H. et al.** Comparison of four chemotherapy regimens for advanced non-small-cell lung cancer. *N Engl J Med,* 2002, vol. 346 (2), 92-8
- **KELLY, K. et al.** Randomized phase III trial of paclitaxel plus carboplatin versus vinorelbine plus cisplatin in the treatment of patients with advanced non-small-cell lung cancer: a Southwest Oncology Group trial. *J Clin Oncol,* 2001, vol. 19 (13), 3210-8
- **BASELGA, J.** Why the epidermal growth factor receptor? The rationale for cancer therapy. *Oncologist,* 2002, vol. 7 (4), 2-8
- **TRAXLER, P.** Tyrosine kinases as targets in cancer therapy - successes and failures. *Expert Opin Ther Targets,* 2003, vol. 7 (2), 215-34
- **WAKELING, A.E. et al.** ZD1839 (Iressa): an orally active inhibitor of epidermal growth fador signaling with potential for cancer therapy. *Cancer Res,* 2002, vol. 62 (20), 5749-54
- **HERBST, R.S.** Dose-comparative monotherapy trials of ZD1839 in previously treated non-small cell lung cancer patients. *Semin Oncol,* 2003, vol. 30 (1), 30-8
- **FUKUOKA, M. et al.** Final results from a phase □ trial of ZD1839 ('Iressa') for patients with advanced non-small cell lung cancer (IDEAL 1). *Pro Am Soc Clin Oncol,* 2002, vol. 21, 298
- **KRIS, MG. et al.** A phase II trial of ZD1839 ('Iressa') in advanced non-small cell lung cancer (NSCLC) patients who had failed platinum- and docetaxel-based regimens (IDEAL 2). *Pro Am Soc Clin Oncol,* 2002, vol. 21, 292
- **INOUE, A. et al.** Severe acute interstitial pneumonia and gefitinib. *Lancet,* 2003, vol. 361 (9352), 137-9
- **BOHM, M. et al.** Microbeam MOMeNT: non-contact laser microdissection of membrane-mounted native tissue. *Am J Pathol,* 1997, vol. 151 (1), 63-7
- **OKABE, H. et al.** Genome-wide analysis of gene expression in human hepatocellular carcinomas using cDNA microarray: identification of genes involved in viral carcinogenesis and tumor progression. *Cancer Res,* 2001, vol. 61 (5), 2129-37
- **KITAHARA, O. et al.** Alterations ofgene expression during colorectal carcinogenesis revealed by cDNA microarrays after laser-capture microdissection of tumor tissues and normal epithelia. *Cancer Res,* 2001, vol. 61 (9), 3544-9
- **GOLUB, T.R. et al.** Molecular classification of cancer: class discovery and class prediction by gene expression monitoring. *Science,* 1999, vol. 286 (5439), 531-7
- **MACDONALD, T.J. et al.** Expression profiling of medulloblastoma: PDGFRA and the RAS/MAPK pathway as therapeutic targets for metastatic disease. *Nat Genet,* 2001, vol. 29 (2), 143-52
- **KANETA.Y.** Prediction of sensitivity to STI571 among chronic myeloid leukemia patients by genome-wide cDNA microarray analysis. *Jpn J Cancer Res,* 2002, vol. 93, 849-856
- **PAVELIC, K. et al.** Evidence for a role of EGF receptor in the progression of human lung carcinoma. *Anticancer Res,* 1993, vol. 13 (4), 1133-7
- **KIKUCHI, T. et al.** Expression profiles of non-small cell lung cancers on cDNA microarrays: Identification of genes for prediction of lymph-node metastasis and sensitivity to anti-cancer drugs. *Oncogene,* 2003, vol. 22 (14), 2192-205
- **HEIGHWAY, J. et al.** Expression profiling of primary non-small cell lung cancer for target identification. *Oncogene,* 2002, vol. 21 (50), 7749-63
- **BEER, D.G. et al.** Gene-expression profiles predict survival ofpatients with lung adenocarcinoma. *Nat Med,* 2002, vol. 8 (8), 816-24

- **MIURA, K. et al.** Laser capture microdissection and microarray expression analysis of lung adenocarcinoma reveals tobacco smoking- and prognosis-related molecular profiles. *Cancer Res,* 2002, vol. 62 (11), 3244-50
- **MOASSER, M.M. et al.** The tyrosine kinase inhibitor ZD1839 (''Iressa'') inhibits HER2-driven signaling and suppresses the growth of HER2-overexpressing tumor cells. *Cancer Res,* 2001, vol. 61 (19), 7184-8
- **RUSCH, V. et al.** Overexpression of the epidermal growth factor receptor and its ligand transforming growth factor alpha is frequent in resectable non-small cell lung cancer but does not predict tumor progression. *Clin Cancer Res,* 1997, vol. 3 (4), 515-22
- **FONTANINI, G. et al.** Evaluation of epidermal growth factor-related growth factors and receptors and of neoangiogenesis in completely resected stage I-IIIA non-small-cell lung cancer: amphiregulin and microvessel count are independent prognostic indicators ofsurvival. *Clin Cancer Res,* 1998, vol. 4 (1), 241-9
- **BRUNDAGE, M.D. ; D. DAVIES ; W.J. MACKILLOP.** Prognostic factors in non-small cell lung cancer: a decade ofprogress. *Chest,* 2002, vol. 122 (3), 1037-57
- **HURBIN, A. et al.** Inhibition of apoptosis by amphiregulin via an insulin-like growth factor-1 receptor-dependent pathway in non-small cell lung cancer cell lines. *J Biol Chem,* 2002, vol. 277 (51), 49127-33
- **YARDEN, Y. ; M.X. SLIWKOWSKI.** Untangling the ErbB signalling network. *Nat Rev Mol Cell Biol,* 2001, vol. 2 (2), 127-37
- **PRENZEL, N. et al.** EGF receptor transactivation by G-protein-coupled receptors requires metalloproteinase cleavage of proHB-EGF. *Nature,* 1999, vol. 402 (6764), 884-8
- **NELSON, CHAU et al.** Aven, a novel inhibitor of caspase activation, binds Bcl-xL and Apaf-1. *Molec Cell,* 2000, vol. 6, 31-41
- **TIPNIS, SR. et al.** Overexpression of the regulatory subunit of r-glutamylcysteine synthetase in Hela cells increases r-glutamylcysteine synthetase activity and confers drug resistance. *Biochem J,* 1999, vol. 337, 559-566